# EUROPEAN PATENT APPLICATION

(11) **EP 3 702 458 A1**
(43) Date of publication of application: **02.09.2020**
(21) Application number: 18870256.7
(22) Date of filing: 26.10.2018
(51) Int. Cl.: C12N 15/113, C12N 5/22, C12N 9/22, A61K 35/18, A61P 7/06

(54) **METHOD FOR INCREASING FETAL HEMOGLOBIN EXPRESSION LEVEL**

(30) Priority: 27.10.2017 CN 201711027708
(71) Applicant: Edigene Inc., Beijing 102206 (CN)
(72) Inventor: YUAN, Pengfei, Beijing 102206 (CN); FANG, Riguo, Beijing 102206 (CN); YU, Lingling, Beijing 102206 (CN); XIA, Penghui, Beijing 102206 (CN); WANG, Jia, Beijing 102206 (CN); LIANG, Fucai, Beijing 102206 (CN)
(74) Representative: Lavoix
(86) International application number: PCT/CN2018/112068
(87) International publication number: WO 2019/080920

(57) **Abstract**

Provided is a method for gene editing of an enhancer site of the BCL11A in hematopoietic stem cells. The genetically modified hematopoietic stem cells have the functions of normal cells, and can significantly increase the expression of fetal hemoglobin so as to be used in the treatment of β thalassemia and sickle cell anemia.

## Description

### CROSS REFERENCE

This application claims priority of the Chinese patent application No. 2017110277086 filed on October 27, 2017, and the disclosure of the application is incorporated herein by reference in its entirety.

### FIELD OF THE INVENTION

The invention relates to a cell treatment regimen for treating anemia diseases such as thalassemia, sickle cell anemia and the like, and it comprises efficiently and safely performing genetic modification of a enhancer locus of BCL11A in human hematopoietic stem cells by gene editing technology, and up-regulating the expression of γ globin and fetal hemoglobin, thereby achieving the purpose of treating the diseases.

### BACKGROUND OF THE INVENTION

Thalassemia, also known as *globin aplastic anemia* or Mediterranean anemia, is a group of hemolytic anemias caused by genetic factors such as genetic defects. Hereditary genetic defects result in the loss or lack of synthesis of one or more globin peptide chains in hemoglobin, leading to a pathological state of anemia or hemolysis. The reduction or loss of globin chains for synthesizing hemoglobin leads to abnormal structure of hemoglobin. The deformability of an erythrocyte containing abnormal hemoglobin is reduced and its life span is shortened. *In situ* hemolysis may occur in bone marrow, and when entering peripheral blood circulation, the erythrocytes will be destroyed in advance by organs such as spleen, thereby resulting in anemia, iron deposition in the body and even abnormal development. Due to the diversity and complexity of gene mutations for different globin chains, the types and quantity of the deficient globin and the clinical symptoms show apparent variation.

Thalassemia is named and classified according to the type of the deficient globin chain and the deficient degree thereof. According to different types of globin peptide chain formation disorders, thalassemia may be divided into α type, β type, δ type, and δ β type. At present, thalassemia is one of the most common genetic diseases with gene defects in the world. Statistically speaking, 4.83% of the global population carries globin mutant genes, including 1.67% of α, β thalassemia heterozygotes, and 1.92% of them carrying hemoglobin with sickle cell mutation, 0.95% carrying hemoglobin E, 0.29% carrying hemoglobin C, etc. It is a common genetic disorder, since the resulting global birth rate of the population with abnormal hemoglobin and disease symptoms is no less than 0.024%.

β thalassemia is a type of thalassemia, and its pathogenesis is due to the gene mutation of β globin chain, for most patients it's a point mutation, while for a few patients it's a large segment deletion of the gene. Gene deletions and certain point mutations result in complete inhibition of synthesis of a part of the β globin peptide chain, and this kind of condition is called β⁰ thalassemia. While a few point mutations partly inhibit the synthesis of the β chain, but still retain the synthesis of some part of the peptide chain, and this kind of condition is called β⁺ thalassemia. Different combinations of mutations may cause different clinical symptoms. There are many gene mutation types of β thalassemia. According to statistics, there are more than 300 genetic abnormalities around the world, more than 100 mutation sites have been found, and currently 28 mutation sites have been clinically reported in China. Among them, there are 6 common mutations, incuding about 45% of β41-42 (-TCTT deletion); about 24% of IVS-II654 (C to T point mutation); about 14% of β17 (A to T point mutation); about 9% of TATA box-28 (A to T point mutation); about 2% of B71-72 (+A insertion mutation),, and about 2% of β26 (G to A point mutation), etc.

There are two mutant gene types for severe β thalassemia, one is the Phomozygote, and the other is the double heterozygote of β⁰ and β⁺ thalassemia. Since the formation of β globin peptide chain is nearly completely inhibited, the β globin chain cannot be produced *in vivo,* and the normal synthesis of hemoglobin A consisting of α chain and β chain is reduced or eliminated. Although the excess α chain proteins can bind to the γ chains in an erythrocyte to form hemoglobin F, as the synthesis of γ chain is gradually inhibited (also called hemoglobin chain synthesis switching) after birth, the excess α chains is deposited in erythrocytes to form inclusion bodies adhering to the surface of erythrocyte membrane. Such that the characteristics of erythrocyte membrane are changed, the cells become stiff to cause a decrease in deformability, and they are destroyed in the bone marrow to result in "ineffective hematopoiesis". Although some erythrocytes of a thalassemia patient can develop and mature in bone marrow, and eventually be released into the peripheral blood circulation, when they pass through the peripheral local microcirculation (such as spleen and other organs), mechanical breakage will be easily occurred due to the decrease of their deformability. For the above reasons, a baby patient is clinically asymptomatic at birth. After birth, due to the expression of HbF (fetal hemoglobin) and up to 120 days life span of erythrocytes, the chronic hemolytic anemia is often shown 6 months later when the cell damage is increased by its pathological changes, i.e. γ chain synthesis is physiologically inhibited; synthesis hemoglobin chain is switched to β chain, and meanwhile β chain synthesis fails due to gene defects, and these further lead to changes in bone marrow composition. Repeated blood transfusions are required in the treatment process, resulting in hemosiderosis and thereby affecting the functions of important organs.

Similar to β thalassemia, sickle-shaped erythrocyte anemia is an autosomal recessive hereditary disease, except that this anemia has a single mutation site. It is caused by the single base mutation of β globin, i.e., the codon 6 for the normal β gene is mutated from GAG (encoding glutamic acid) to GTG (encoding valine). In the case of a mutant homozygote, normal α globin and abnormal β globin form a tetrameric complex called HbS, its capacity of carrying oxygen is half of a normal hemoglobin, and it aggregates into multimers in the deoxygenated state. Since the resulting multimers are aligned parallel to the membrane, and closely contact with the cell membrane. When the number of multimers reaches a certain level, the cell membrane changes from a normal concave shape to a sickle shape. With poor deformability, sickle-shaped erythrocytes break easily, hemolysis is occurred, resulting in blood vessel blockage, injury, necrosis and the like.

The treatments of β thalassemia and sickle cell anemia comprise general supportive care, high-dose blood transfusion & regular iron chelation therapy, hematopoietic stem cell transplantation, drug therapy for inducing fetal hemoglobin, and exploratory gene therapy. At present, allogeneic hematopoietic stem cell transplantation, among all the treatments, is the only hope for cure. Since the first hematopoietic stem cell transplantation in a thalassemia patient was successfully carried out in 1981 by Thomas, the hematopoietic stem cell transplantation technology has been used in a number of thalassemia research centers around the world, and it successfully replaced the traditional blood transfusion & iron chelation treatment regimen. However, the widespread use of hematopoietic stem cell transplantation in the treatment of thalassemia remains limited, due to the severe lack of HLA-matched donors and the death caused by GVHD (graft-versus-host reaction) after transplantation. At the same time, researchers have been continuously exploring drugs for treating thalassemia. At present, the only oral drug approved by FDA for clinical treatment is hydroxyurea, which alleviates the clinical symptoms of the disease mainly by inducing fetal hemoglobin expression. However, the clinical efficacy of this drug is inconsistent and there are significant side effects, as well as dose-related myelosuppression. It is urgent to develop new therapeutic methods for the treatment of anemia associated diseases such as β thalassemia, and sickle cell disease.

Several documents are cited throughout this specification. Each document herein (including any journal article or abstract, published or unpublished patent application, issued patent, manufacturer's instructions, instructions for use, etc.) is incorporated herein by reference in its entirety. However, it is not an admission that the documents cited herein are in fact prior art to the present invention.

### CONTENTS OF THE INVENTION

According to the invention, a new generation of hematopoietic stem cells are developed by gene editing technology, such as CRISPR/Cas9 gene editing technology. Compared with hematopoietic stem cells in the prior art, the knockout efficiency of BCL11A enhancer gene in the hematopoietic stem cells is significantly increased, thereby greatly improving the expression of fetal hemoglobin in erythrocytes after differentiation and maturation, and the problem of low editing efficiency of BCL11A enhancer and the insufficient fetal hemoglobin expression for the clinical application in the prior art is solved in a certain extent. In addition, the present invention further improves the strategy for culturing and differentiating the hematopoietic stem cells subjected to gene editing, not only greatly shortening the differentiation process from the hematopoietic stem cells to mature erythrocytes, but also greatly increasing the quantity of the harvested mature erythrocytes, and thereby partially satisfying the requirements for clinical application.

Specifically, the invention relates to the following items:
1. A method for increasing fetal hemoglobin (HbF) expression in human hematopoietic stem cells, comprising:
   disrupting a BCL11A genomic region from positions 60495219-60495336 in the chromosome 2 of the hematopoietic stem cells by gene editing technology.
2. The method of item 1, wherein the gene editing technology is a zinc finger nuclease-based gene editing technology, a TALEN gene editing technology, or a CRISPR/Cas gene editing technology.
3. The method of item 2, wherein the gene editing technology is CRISPR/Cas9 gene editing technology.
4. The method of any one of items 1-3, wherein the target nucleotide sequence of BCL11A gene is complementary to a sequence selected from any one of SEQ ID NOs: 3-25.
5. The method of item 3 or 4, an sgRNA comprising a sequence selected from any one of SEQ ID NOs: 3-25 is introduced into the hematopoietic stem cell for the gene editing of the BCL11A genome.
6. The method of item 5, wherein the sgRNA is a 2'-O-methyl sgRNA and/or an internucleotide 3'-thio-sgRNA.
7. The method of item 6, wherein the chemical modification is 2'-O-methyl modification of the first one, two and/or three bases at the 5' end and/or the last base at the 3' end of the sgRNA.
8. The method of any one of items 3-7, wherein the sgRNA and the Cas9-encoding nucleotides are co-introduced into the hematopoietic stem cells.
9. The method of item 8, wherein the sgRNA and the Cas9-encoding nucleotides are co-introduced into the hematopoietic stem cells by electroporation.
10. The method of item 9, wherein the electroporation conditions are 200-600 V, 0.5-2 ms.
11. A method for efficiently editing hematopoietic stem cells in vitro by a CRISPR/Cas9 system, comprising: introducing an sgRNA comprising a sequence selected from any one of SEQ ID NOs: 3-25 into the hematopoietic stem cells, wherein the sgRNA is a 2'-O-methyl sgRNA and/or an internucleotide 3'-thio sgRNA.
12. The method of item 11, wherein the sgRNA and the Cas9-encoding nucleotides are co-introduced into the hematopoietic stem cells.
13. The method of item 12, wherein the sgRNA and the Cas9-encoding nucleotides are co-introduced into the hematopoietic stem cells by electroporation.
14. The method of item 13, wherein the electroporation conditions are 200-600 V, 0.5-2 ms.
15. A hematopoietic stem cell obtained by the method of any one of items 1-14.
16. A human hematopoietic stem cell increasing the fetal hemoglobin (HbF) expression by genetic modification, wherein one or more sites in the target sequence of the BCL11A genome from positions 60495219-60495336 in the chromosome 2 of the hematopoietic stem cell are disrupted by a gene editing technology.
17. Precursor cells at different stages of differentiation before mature erythrocytes, obtained by differentiation culture of the hematopoietic stem cells of item 15 or 16.
18. Mature erythrocytes obtained by differentiation culture of the hematopoietic stem cells of item 15 or 16.
19. A method for preparing mature erythrocytes or precursor cells thereof being genetically modified to increase the fetal hemoglobin (HbF) expression, comprising:
   (a) obtaining genetically modified hematopoietic stem cells using the method of any one of items 1-14; and
   (b) performing hematopoietic stem cell erythroid expansion and differentiation of the genetically modified hematopoietic stem cells by using a HSPCs erythroid expansion and differentiation medium;
   wherein the HSPCs erythroid expansion and differentiation medium comprises a basal medium and a composition of growth factors, and wherein the composition of growth factors comprises a stem cell growth factor (SCF); interleukin-3 (IL-3) and erythropoietin (EPO).
20. The method of item 19, further comprising:
   performing erythroid differentiation and enucleation on hematopoietic stem cells by using an erythroid differentiation and enucleation medium;
   wherein the erythroid differentiation and enucleation medium comprises a basal medium, growth factors, and antagonists and/or inhibitors of a progesterone receptor and a glucocorticoid receptor.
21. The method of item 20, wherein the growth factors in the erythroid differentiation and enucleation medium comprise erythropoietin (EPO), wherein the antagonists and/or inhibitors of a progesterone receptor and a glucocorticoid receptor are any one or two or more selected from the following compounds (I)-(IV): 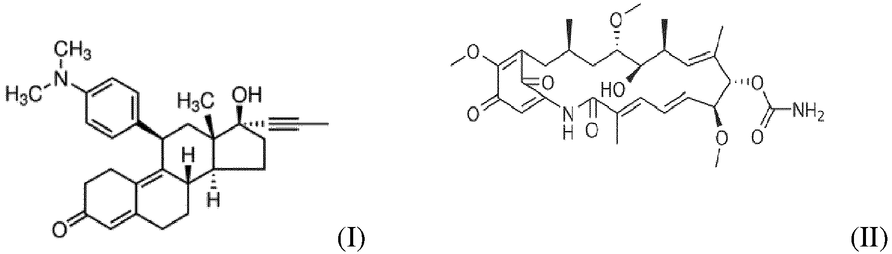 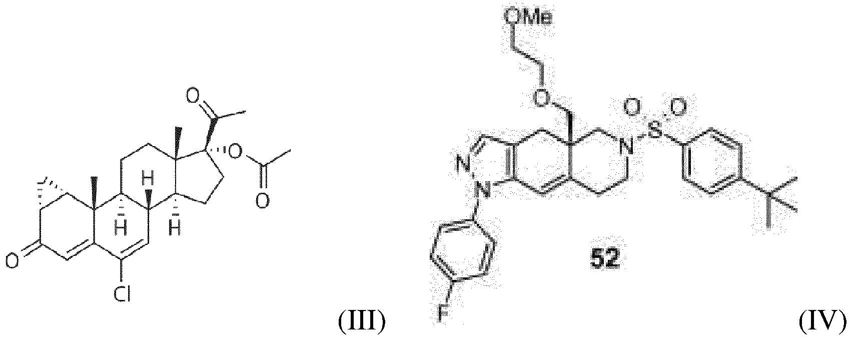
22. A mature erythrocyte or precursor cell thereof obtained by any one of items 19-21.
23. A composition comprising the hematopoietic stem cells of item 15 or 16, or the precursor cells of item 17 or 22, or the mature erythrocytes of item 18 or 22.
24. A medical preparation comprising the hematopoietic stem cells of item 15 or 16, or the precursor cells of item 17 or 22, or the mature erythrocytes of item 18 or 22.
25. Use of the hematopoietic stem cells of item 15 or 16, or the precursor cells of item 17 or 22, or the mature erythrocytes of item 18 or 22 in the prevention or treatment of a disease in a subject in need thereof.
26. The use of item 25, wherein the disease is an anemia disease, a hemorrhagic disease, a tumor, or other diseases requiring massive blood transfusion for prevention or treatment.
27. The use of item 26, wherein the disease is β thalassemia or sickle cell anemia.
28. The use of any one of items 25-27, wherein the subject is a human.
29. Use of the hematopoietic stem cells of item 15 or 16, or the precursor cells of item 17 or 22, or the mature erythrocytes of item 18 or 22 in the manufacture of a medicament or medical preparation for preventing or treating a disease in a subject.
30. The use of item 29, wherein the disease is an anemia disease, a hemorrhagic disease, a tumor or other diseases requiring massive blood transfusion for prevention or treatment.
31. The use of item 30, wherein the disease is β thalassemia or sickle cell anemia.
32. The use of any one of items 29-31, wherein the subject is a human.
33. An sgRNA construct comprising a nucleotide sequence selected from any one of SEQ ID NOs: 3-25.
34. The construct of item 33, comprising a 2'-O-methyl nucleotide modification and/or an internucleotide 3'-thio modification.
35. Construct of item 34, wherein the chemical modification is a 2'-O-methyl modification of the first one, two and/or three bases at the 5' end and/or the last base at the 3' end of a nucleotide sequence selected from any one of SEQ ID NOs: 3-25.
36. A vector, a host cell, or a formulation comprising the construct of any one of items 33-35.
37. Use of the construct of any one of items 33-35 in gene editing of hematopoietic stem cells.
38. A method for treating or preventing an anemia disease, a hemorrhagic disease, a tumor, or other diseases requiring massive blood transfusion for prevention or treatment in a subject, comprising administering the hematopoietic stem cells of item 15 or 16, the precursor cells of item 17 or 22, or the mature erythrocytes of item 18 or 22 to the subject.
39. The method of item 38, wherein the disease is β thalassemia or sickle cell anemia.
40. The method of item 39, wherein the subject is a human.
41. A kit for treating or preventing an anemia disease, a hemorrhagic disease, a tumor, or other diseases requiring massive blood transfusion in a subject, comprising the construct of the sgRNA of any one of items 33-35, or the vector of item 36.
42. The kit of item 41, further comprising Cas9 mRNA.

### ADVANTAGES OF THE INVENTION

According to the invention, the enhancer locus of BCL11A in hematopoietic stem cells of healthy donors and anemia patients are edited with the optimized electroporation transfection system, thereby meeting the requirements of clinical treatment. In addition, the erythroid differentiation of the edited hematopoietic stem cells can significantly increase the expression of γ globin and fetal hemoglobin (HbF), and the hematopoietic system of an animal model may be reconstituted. The off-target analysis shows that the safety degree of the method is high, and the side effects caused by gene editing are hardly detected.

### BRIEF DESCRIPTION OF THE DRAWINGS

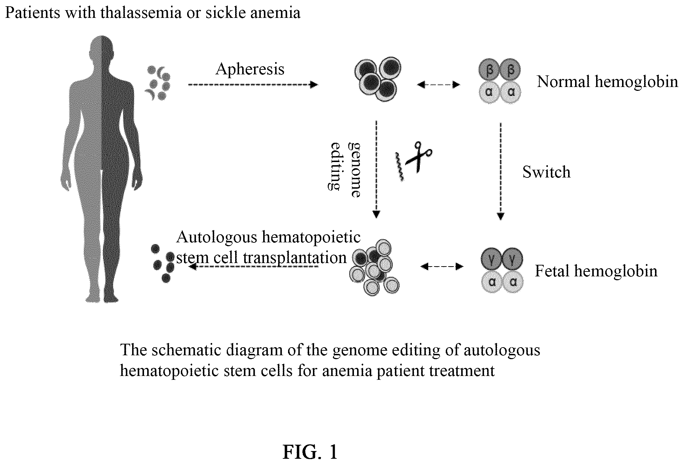

**FIG. 1****:** The therapeutic schedule for the treatment of β thalassemia and sickle cell anemia with genetically modified autologous hematopoietic stem cells. Peripheral blood is mobilized from a patient, CD34-positive hematopoietic stem cells are isolated *in vitro.* Fetal hemoglobin expression is improved through *in vitro* gene editing. The genetically modified autologous hematopoietic stem cells are infused back to the patient.

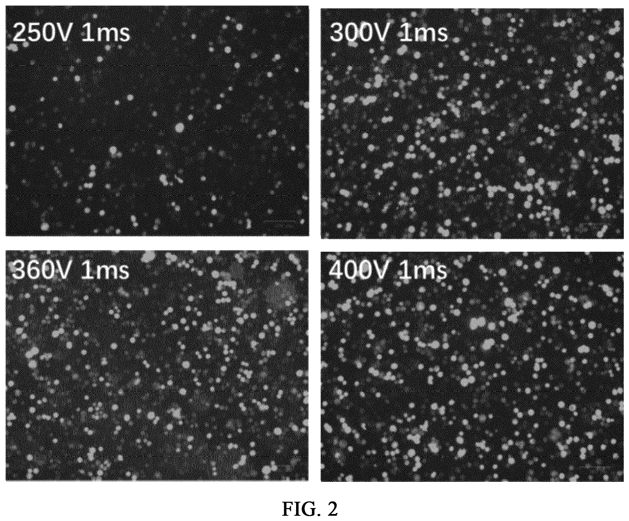

**FIG. 2****:** Fluorescence photomicrographs, obtained 4 days after transfecting with GFP (green fluorescent protein) by electroporation in multi-batch experiments on cancer cell line K562 for determining the optimal electroporation conditions. "V" refers to the pulse voltage and "ms" refers to the pulse time.

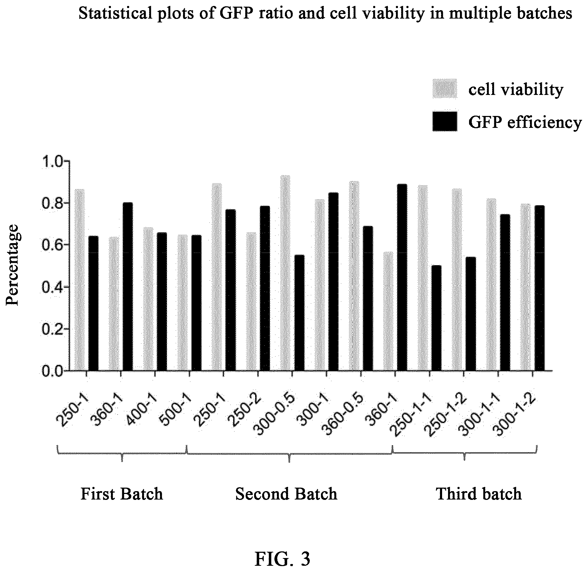

**FIG. 3****:** A graph showing flow cytometry analysis of 7-AAD and statistical analysis of GFP expression, obtained 4 days after transfecting with GFP by electroporation in multi-batch experiments on cancer cell line K562 for determining the optimal electroporation conditions. 7-AAD shows the viability of cells. 7-AAD (7-amino-actinomycin D) is a nucleic acid dye which cannot pass through the normal plasma membrane. During the process of apoptosis and cell death, the permeability of the plasma membrane to 7-AAD is gradually increased; being excited by excitation light with proper wavelength, 7-AAD emits bright red fluorescence. 7-AAD negative cells are normal live cells. Electrorotation efficiency is indicated by GFP. "250-1" means a voltage of 250 V and a pulse time of 1 ms, "250-1-1" and "250-1-2" respectively indicate the two repetitions of "250V, 1ms"; "300-1-1" and "300-1-2" respectively indicate the two repetitions of "300V, 1ms".

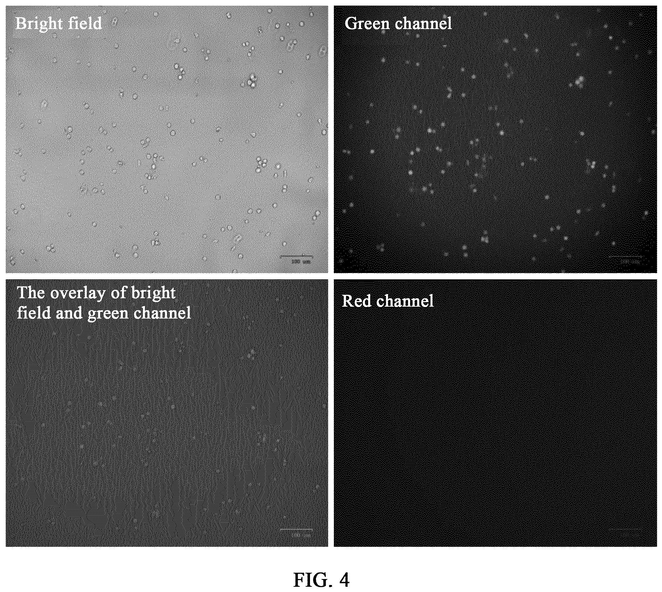

**FIG. 4****:** Fluorescence photomicrographs, obtained 4 days after transfecting the hematopoietic stem cells with GFP mRNA by electroporation under the electroporation conditions of 300 V, 1 ms. The four fields included are the bright field, green channel, red channel, and overlay of the bright field and green channel.

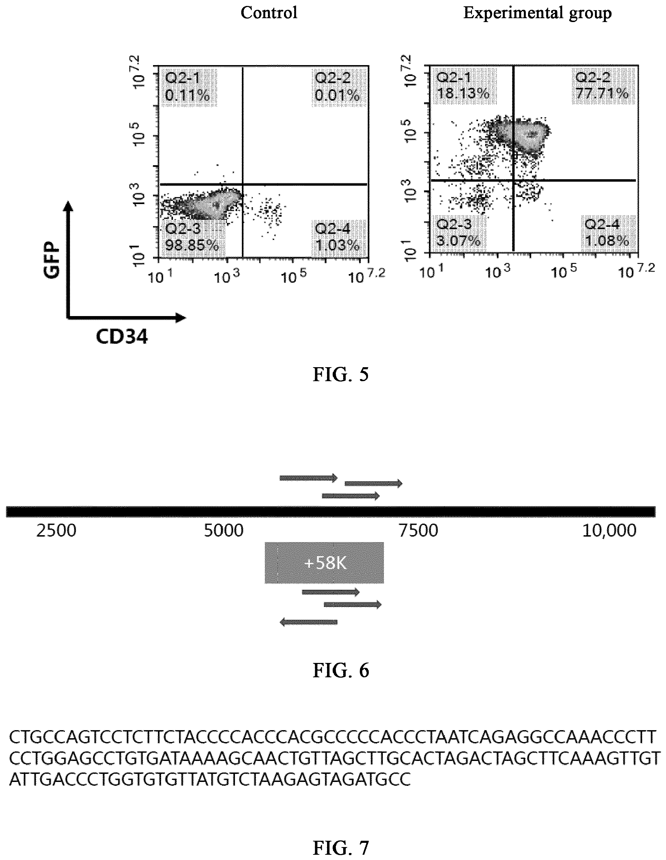

**FIG. 5****:** Flow cytometry analysis of GPF and expression of CD34 protein, 4 days after transfecting the hematopoietic stem cells with GFP mRNA by electroporation under the electroporation conditions of 300V, 1ms.
**FIG. 6****:** A schematic diagram of multiple sgRNAs designed for the locus 58K of human BCL11A enhancer.
**FIG. 7****:** Information of the 150bp sequence at the locus 58K of human BCL11A enhancer.

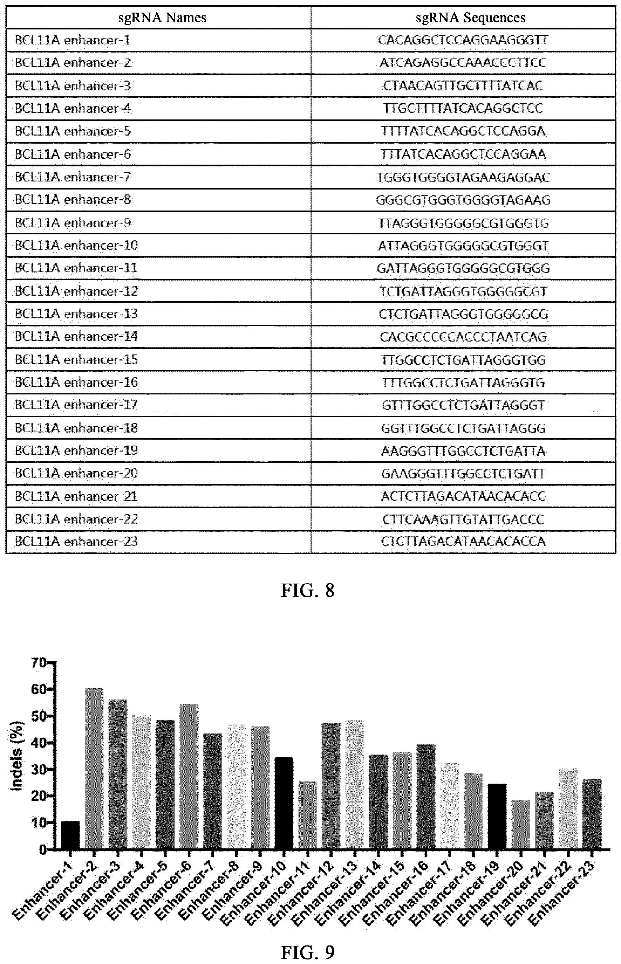

**FIG. 8****:** Specific DNA sequence information of 23 sgRNAs designed for the locus 58K (150 bp) of human BCL11A enhancer.
**FIG. 9****:** The statistical analysis of Indels efficiency, which is obtained by transfecting CD34-positive hematopoietic stem cells with Cas9 mRNA and multiple sgRNAs by electroporation, then extracting the genomes 4 days later, amplifying the fragments and performing Sanger sequencing, and finally making said statistical analysis by using TIDE software.

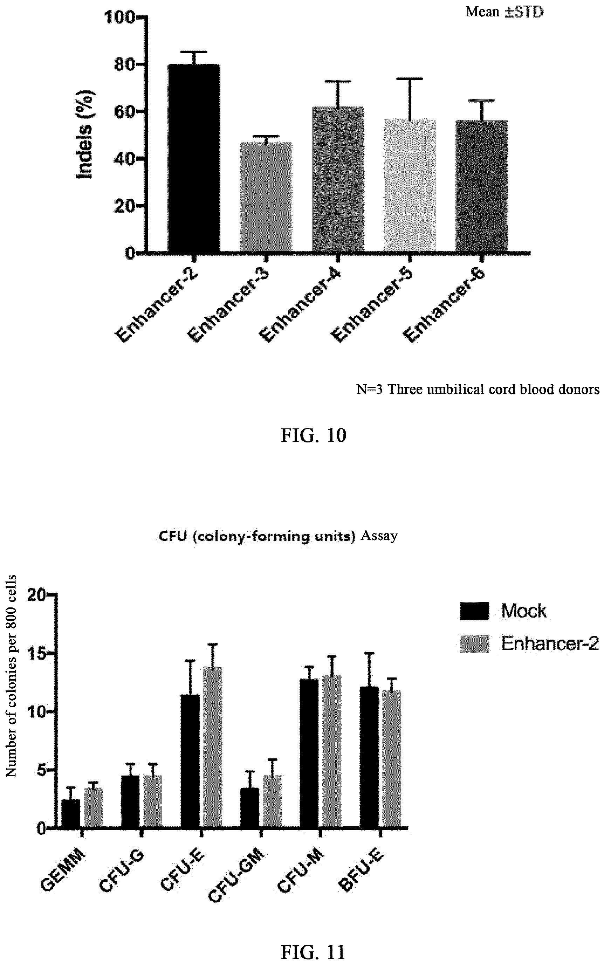

**FIG. 10****:** The statistical analysis of Indels efficiency, which is obtained by transfecting CD34-positive hematopoietic stem cells derived from 3 different cord blood souces with Cas9 mRNA and BCL11A enhancer-2, 3, 4, 5, and 6 (i.e., enhancer-2, 3, 4, 5, and 6 in the Figure) sgRNAs by electroporation., then performing said statistical analysis by TIDE software after 4 days.
**FIG. 11****:** A graph showing the number of colonies for different blood systems, which is obtained by transfecting CD34-positive hematopoietic stem cells derived from cord blood with Cas9 mRNA and BCL11A enhancer-2 sgRNA by electroporation, performing *in vitro* colony-forming units assay (CFU assay) 2 days later, and then counting the number of colonies for different blood systems 14 days later; wherein BFU-E, CFU-M, CFU-GM, CFU-E, CFU-G, and CFU-MM represent the cell colonies of different lineages, such as the erythroid, myeloid, lymphoid lineage, etc. in blood system, and wherein Mock represents cells not being genetically modified.

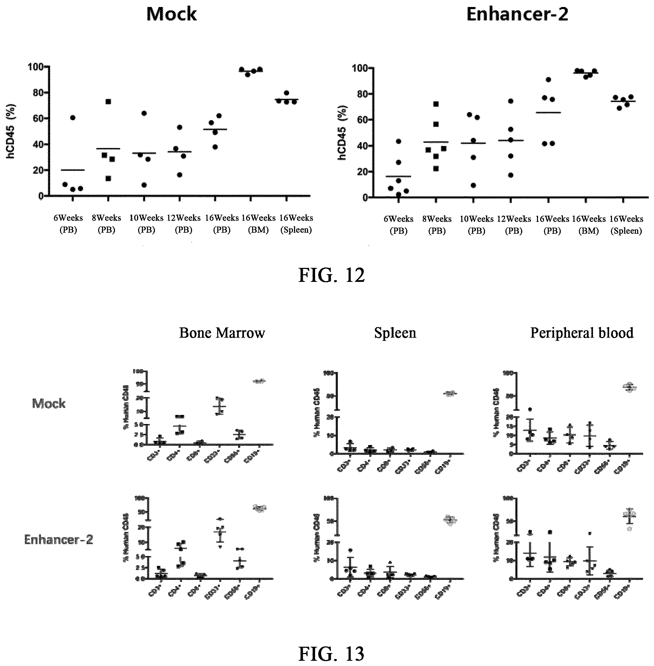

**FIG. 12****:** Graphs showing the proportion of human CD45-positive cells, which are obtained by transfecting the hematopoietic stem cells with Cas9 mRNA and BCL11A enhancer-2 sgRNA by electroporation, and transplanting the genetically modified and unmodified cells into NPG immunodeficient mouse model irradiated by irradiator respectively, after 6 weeks, 8 weeks, 10 weeks, 12 weeks, and 16 weeks, the proportion of human CD45-positive cells is detected in mouse peripheral blood; and 16 weeks after transplantation, the proportion of human CD45-positive cells is also detected in mouse bone marrow and spleen; wherein the proportion of CD45-positive cells is calculated through dividing % human CD45-positive cells by (% human CD45-positive cells +% mouse CD45-positive cells); % human CD45-positive cells, and % mouse CD45-positive cells are determined by flow cytometry assay respectively; and wherein Mock represents cells not being genetically modified, and Enhancer-2 represents genetically modified cells.
**FIG. 13****:** Graphs showing the proportion of human CD45-positive cells, which are obtained by transfecting the hematopoietic stem cells derived from cord blood with Cas9 mRNA and BCL11A enhancer-2 sgRNA by electroporation, and transplanting the genetically modified and unmodified cells into NPG immunodeficient mouse model irradiated by irradiator respectively, 16 weeks later the proportion of human cell membrane proteins such as CD3, CD4, CD8, CD33, CD56 and CD19 versus human CD45 protein are respectively detected in mouse bone marrow, spleen and peripheral blood; wherein Mock represents cells not being genetically modified, and Enhancer-2 represents genetically modified cells.

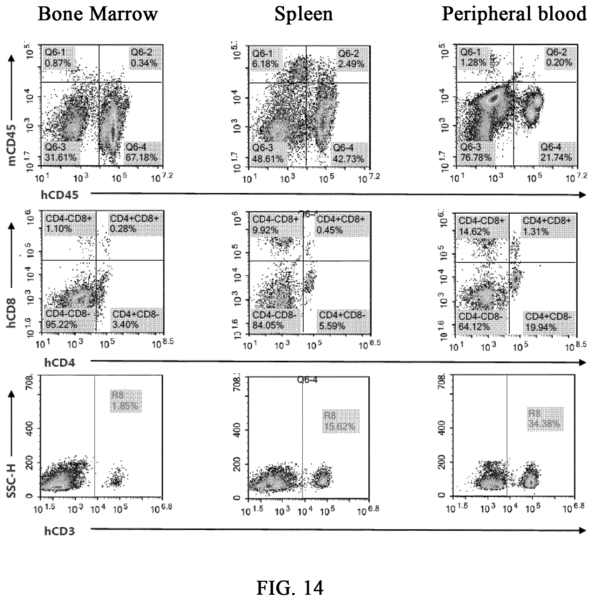

**FIG. 14****:** Graphs showing the expression of mouse CD45, human CD45, CD3, CD4 and CD8, which are obtained by transfecting the hematopoietic stem cells derived from cord blood with Cas9 mRNA and BCL11A enhancer-2 sgRNA by electroporation, and transplanting the genetically modified cells into NPG immunodeficient mouse model irradiated by irradiator, 16 weeks later the expression of mouse CD45, human CD45, CD3, CD4 and CD8 are respectively detected in mouse bone marrow, spleen and peripheral blood by flow cytometry assay; wherein the SSC-H channel refers to lateral angle scattering, which represents the granularity of a cell, and the larger value indicates larger granularity of a cell; the granularity represents the degree of buckling on the cell surface, the number of subcellular organelles, and particles in a cell, etc.

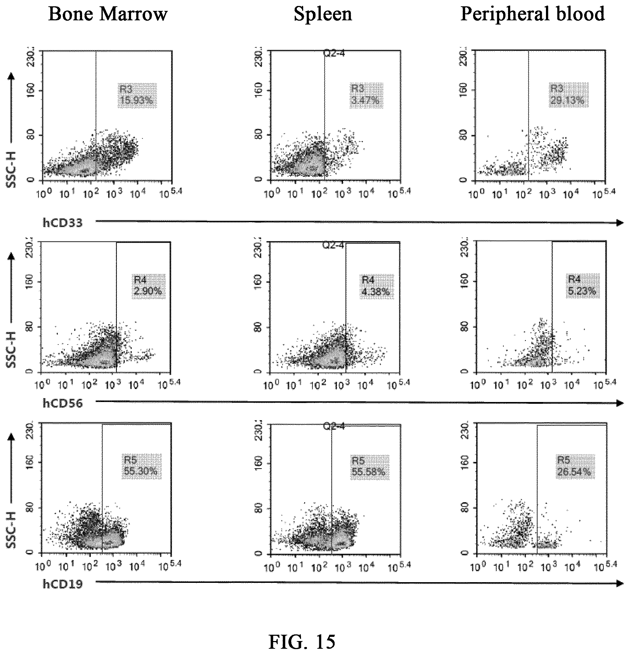

**FIG. 15****:** Graphs showing the expression of human CD33, CD56, and CD19 in one mouse, which are obtained by transfecting the hematopoietic stem cells derived from cord blood with Cas9 mRNA and BCL11A enhancer-2 sgRNA by electroporation, and transplanting the genetically modified cells into NPG immunodeficient mouse model irradiated by irradiator, 16 weeks later the expressions of human CD33, CD56, CD19 are respectively detected in mouse bone marrow, spleen, peripheral blood by flow cytometry assay; wherein the SSC-H channel refers to lateral angle scattering, which represents the granularity of a cell, and the larger value indicates larger granularity of a cell; the granularity represents the degree of buckling on the cell surface, the number of subcellular organelles, and particles in a cell, etc.

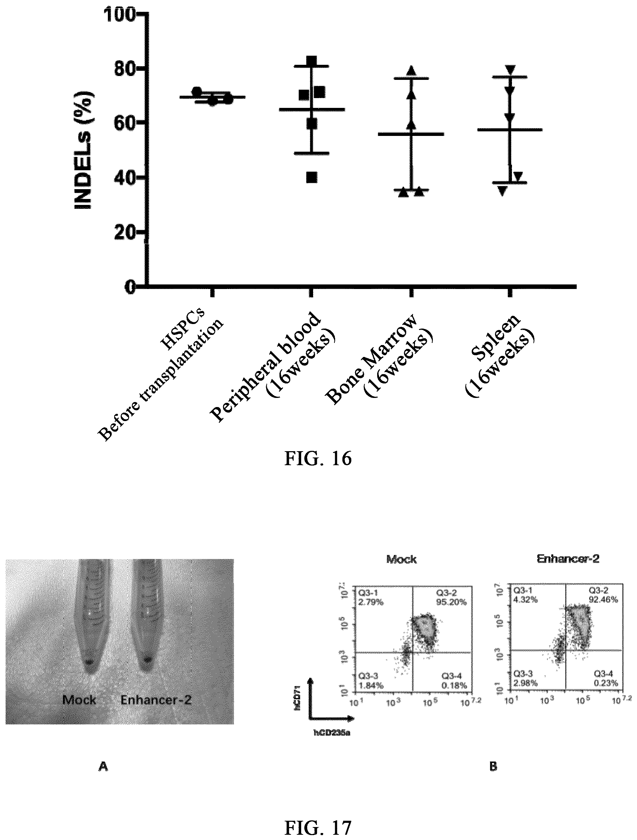

**FIG. 16****:** A graph showing statistical analysis of Indels efficiency, which is obtained by transfecting the hematopoietic stem cells derived from cord blood with Cas9 mRNA and BCL11A enhancer-2 sgRNA by electroporation, then extracting the genomes of the cells before transplantation, and genomes of peripheral blood, bone marrow, spleen 16 weeks after transplantation, amplifying the target fragments and performing Sanger sequencing, and finally analyzing Indels efficiency of gene editing by TIDE software.
**FIG. 17****:** Graphs showing erythroid differentiation, which are obtained by transfecting the hematopoietic stem cells derived from cord blood with Cas9 mRNA and BCL11A enhancer-2 sgRNA, then performing erythroid differentiation, and detecting the cells after 12 days. Fig.17-A is a photograph of erythrocytes after differentiation. Fig.17-B represents the detection results of the expression of human CD71 and human 235a membrane proteins, indicating erythroid differentiation efficiency. Mock represents cells not being genetically edited, and Enhancer-2 represents genetically modified cells.

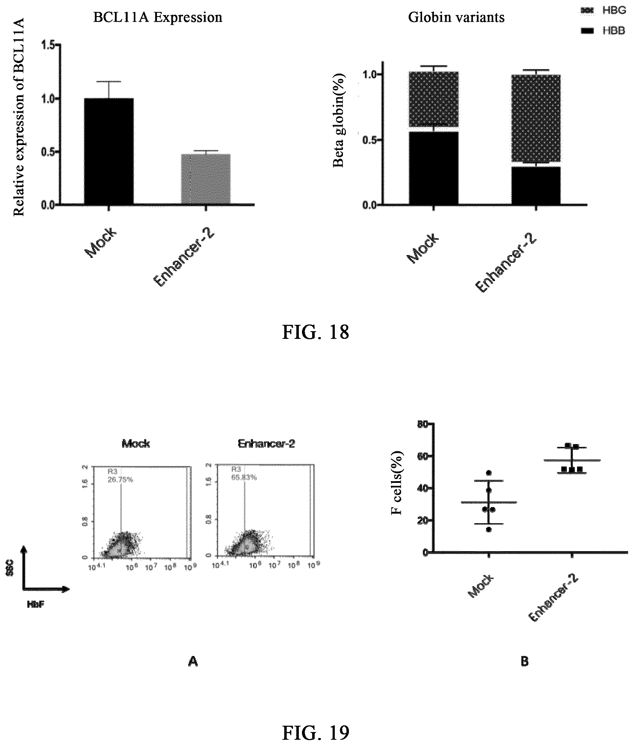

**FIG. 18****:** Graphs showing the expression of mRNAs, which are obtained by transfecting the hematopoietic stem cells derived from cord blood with Cas9 mRNA and BCL11A enhancer-2 sgRNA by electroporation then performing erythroid differentiation, and detecting mRNA expression of BCL11A, HBB, HBG and other genes by quantitative fluorescence PCR 12 days later. Mock represents cells not being genetically modified, and Enhancer-2 represents genetically modified cells.
**FIG. 19****:** Graphs showing the expression of fetal hemoglobin (HbF), which are obtained by transfecting the hematopoietic stem cells derived from cord blood with 6 µg of Cas9 mRNA and 4 µg of BCL11A enhancer-2 sgRNA by electroporation, then performing erythroid differentiation, and detecting the expression of fetal hemoglobin (HbF) in the cells after 12 days. The left panel shows the graphs about flow cytometry analysis, and the right panel is the statistical analysis graph about fetal hemoglobin expression. Mock represents cells not being genetically modified, and Enhancer-2 represents genetically modified cells.

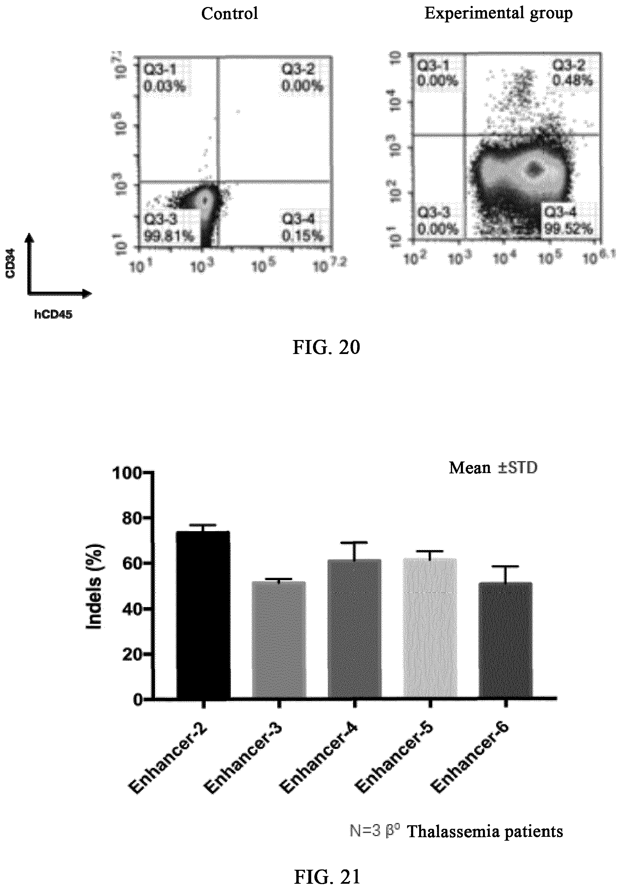

**FIG. 20****:** Graphs showing the detection results of the expression of CD45 and CD34 in freshly isolated peripheral blood of patients with β thalassemia. The left panel is the control group, and the right panel is the experimental group. The experimental samples are from 3 patients with β thalassemia respectively.
**FIG. 21****:** A graph showing the statistical analysis of Indels efficiency, which is obtained by transfecting CD34-positive hematopoietic stem cells isolated from peripheral blood of 3 different patients suffering from β thalassemia with Cas9 mRNA and BCL11A enhancer-2, 3, 4, 5 and 6 (i.e., enhancer-2, 3, 4, 5 and 6 in Fig. 21) sgRNAs by electroporation, after 4 days, performing statistical analysis of Indels efficiency by TIDE software.

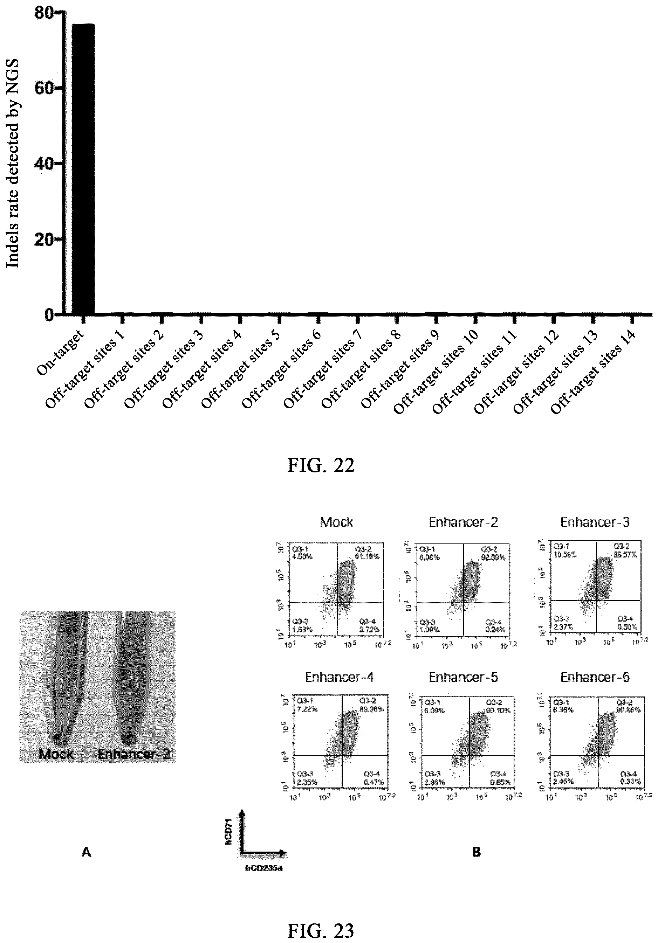

**FIG. 22****:** A graph showing the statistical analysis of potential off-target sites, which is obtained by transfecting CD34-positive hematopoietic stem cells isolated from peripheral blood of patients suffering from β thalassemia with Cas9 mRNA and BCL11A enhancer-2 sgRNA by electroporation, extracting the genomes, and performing NGS deep sequencing analysis about the statistical graph of the 14 potential off-target sites.
**FIG. 23****:** Graphs showing the erythroid differentiation, which are obtained by transfecting the hematopoietic stem cells derived from patients suffering from β thalassemia with Cas9 mRNA and BCL11A enhancer-2, 3, 4, 5, and 6 (i.e., Enhancer-2, 3, 4, 5, and 6 in Fig. 23) sgRNAs by electroporation, then performing erythroid differentiation and detecting the cells after 12 days. Fig.23-A shows a photograph of the erythrocytes after differentiation. Fig.23-B represents the detection results of the expression of human CD71 and human 235a membrane proteins, indicating the efficiency of erythroid differentiation. Mock represents cells not being genetically modified, and Enhancer-2, 3, 4, 5 and 6 represent genetically modified cells.

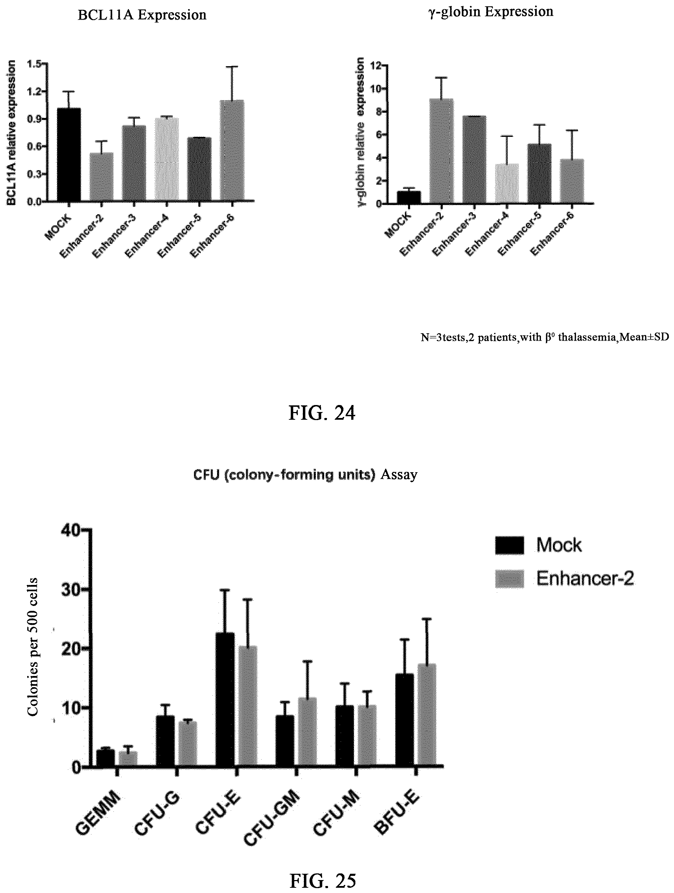

**FIG. 24****:** Graphs showing the expression of genes, which are obtained by transfecting the CD34-positive hematopoietic stem cells isolated from peripheral blood of patients suffering from β thalassemia with Cas9 mRNA and BCL11A enhancer-2, 3, 4, 5, and 6 (i.e., enhancer-2, 3, 4, 5, and 6 in Fig. 24) sgRNAs by electroporation, 12 days after the erythroid differentiation, detecting gene expression of BCL11A and γ-globin gene. Mock represents cells not being genetically edited, and Enhancer-2, 3, 4, 5 and 6 represent genetically modified cells.
**FIG. 25****:** A graph showing the number of colonies for different blood systems, which is obtained by transfecting CD34-positive hematopoietic stem cells derived from patients suffering from β thalassemia with Cas9 mRNA and BCL11A enhancer-2 sgRNA by electroporation, performing *in vitro* colony-forming units assay (CFU assay) 2 days later, and then counting the number of colonies for different blood systems 14 days later. BFU-E, CFU-M, CFU-GM, CFU-E, CFU-G, and CFU-MM represent the colonies of different lineages such as erythroid, myeloid, and lymphoid lineage, etc. in blood system.

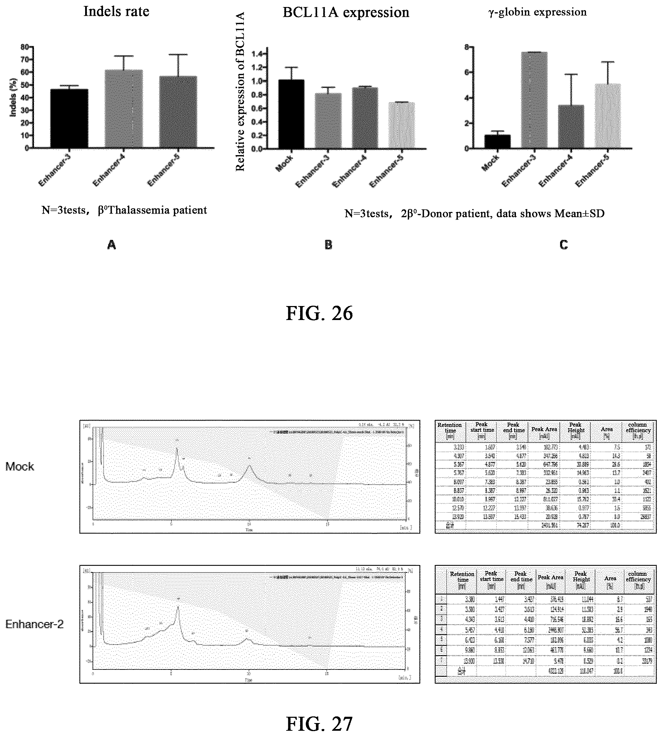

**FIG. 26****:** Graphs showing the Indels efficiency and expression of genes, which are obtained by transfecting CD34-positive hematopoietic stem cells derived from patients suffering from β thalassemia with Cas9 mRNA and BCL11A enhancer-3, -4, or -5 sgRNA by electroporation, evaluating gene editing efficiency, the gene expression of BCL11A and γ-globin gene. A) 4 days after electroporation, performing statistical analysis of the Indels efficiency produced by different sgRNAs by TIDE software; B) performing erythroid differentiation after transfecting the hematopoietic stem cells by electroporation, and detecting the gene expression of BCL11A 12 days later; C) performing erythroid differentiation after transfecting the hematopoietic stem cells by electroporation, and detecting the gene expression of γ-globin gene 12 days later. Mock represents cells not being genetically edited, and Enhancer-3, -4, and -5 represent the genetically modified cells transfected with the Enhancer-3, -4, -5 sgRNA respectively.
**FIG. 27****:** Graphs showing the results of chromatography about cells not being genetically modified (Mock) and genetically modified cells (edited with enhancer-2 sgRNA), and the expression levels of HbF and HbA.

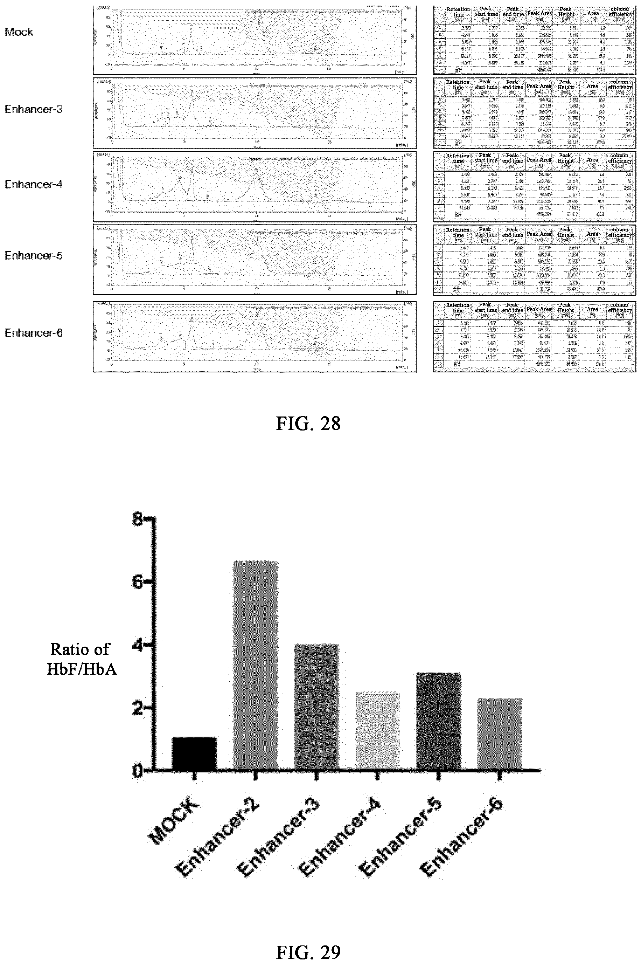

**FIG. 28****:** Graphs showing the results of chromatography about cells not being genetically modified (Mock) and genetically modified cells (edited with enhancer-3, -4, -5, and -6 sgRNA), and the expression levels of HbF and HbA.
**FIG. 29****:** A graph showing the ratio of the expression level of HbF to that of HbA calculated on the basis of the results of chromatography in Figs. 27 and 28.

### DETAILED DESCRIPTION OF SPECIFIC EMBODIMENTS

The application relates to gene editing of a BCL11A enhancer locus of CD34-positive hematopoietic stem cells, and obtaining hematopoietic stem cells with efficiently edited BCL11A enhancer by optimizing various conditions of a CRISPR/Cas system, such as sgRNA, transfection conditions and the like. Experimental results prove that the resulting hematopoietic stem cells have normal functions for erythroid differentiation *in vitro* and the reconstituted hematopoietic system in animal models, and the off-target analysis proves that the cells are safe and reach the standards for clinical treatment.

The present invention provides genetically modified hematopoietic stem cells, erythrocyte precursors, or mature erythrocytes of clinical therapeutic levels for the treatment or prevention of anemias, hemorrhagic diseases, tumors or other diseases requiring massive blood transfusion, such as β thalassemia and sickle cell anemia.

The present invention provides an sgRNA (e.g., a chemically modified sgRNA) comprising a nucleotide sequence selected from any one of SEQ ID NOs: 3-25, for gene editing of the hematopoietic stem cells.

The present invention also provides a method for gene editing of hematopoietic stem cells of patients with β thalassemia and sickle cell anemia.

One purpose of the present invention is to efficiently modify the gene of CD34⁺ hematopoietic stem cells by CRISPR/Cas gene editing technology. For example, CD34⁺ hematopoietic stem cells may be obtained from umbilical cord blood or bone marrow, then gene editing is performed by introducing Cas9 and a given sgRNA into the hematopoietic stem cells under the optimized electroporation conditions, and the genetically modified hematopoietic stem cells are transplanted into experimental animals for the evaluation of the ability of the hematopoietic stem cells to repopulate the hematopoietic system. The sgRNA may be designed for targeting BCL11A enhancer such as targeting BCL11A (+58) locus, by a variety of design software such as "CRISPR RGEN TOOLS" software. In some embodiments, the designed sgRNA ischemically modified, such as a 2'-O-methyl modification and an internucleotide 3'-thio modification at the three bases of its 5'-end and 3'-end. Efficient sgRNAs are obtained through the test of combinations of Cas9 mRNA and different sgRNAs. In certain embodiments, the hematopoietic stem cells are obtained by clinical grade magnetic column sorting, and Cas9 protein encoding nucleotides and chemically modified sgRNAs for gene editing are obtained by *in vitro* transcription assays. The genetically modified hematopoietic stem cells are differentiated into erythroid lineage, and the erythropoiesis rate is detected. For example, the genetically modified hematopoietic stem cells are transplanted into NPG mice irradiated by irradiator for the evaluation of their ability to repopulate the hematopoietic system; or the CD34-positive hematopoietic stem cells of patients may be isolated for the evaluation of gene editing efficiency, erythroid differentiation ability, γ globin and fetal hemoglobin expression.

Firstly, the invention provides a method for improving the expression of fetal hemoglobin (HbF), which comprises performing gene editing of an enhancer locus of BCL11A in the hematopoietic stem cells by CRISPR/Cas9 editing technology. An sgRNA targeting sequence of BCL11A enhancer locus in hematopoietic stem cell is designed in the editing process. The enhancer loci of BCL11A are named as loci +62, +58 and +55, according to their distances (numbers of kilobase) from the transcription initiation site. It is reported that the erythroid enhancer of BCL11A gene negatively regulates fetal hemoglobin (HbF) expression, wherein the region containing the positions with a distance of 55 kb (kb represents 1000 bases), 58 kb and 62 kb from the transcription initiation site is a key regulatory region. Although researchers have studied the loci +55, +58, and +62, the length of gene sequences before and after the above three loci are all above 1000 bp, with a total of about 6000 bp, and thus those skilled in the art do not know which specific region may be edited to achieve the desired editing effect, and even for locus +58, the gene editing efficiency differs greatly (see: Bauer, D. E. et al. An erythroid enhancer of BCL11A subject to genetic variation determines fetal hemoglobin level. Science. 342 253-257 (2013), and Canver MC, et al. Enhancer section by Cas9-mediated in situ saturating mutagenesis. Nature. 2015Nov 12 of BCL11A; 527(7577): 192-7.). Therefore, the first problem for those skilled in the art is to find a specific region which is crucial for gene editing efficiency.

The inventors of the present application have found through intensive studies that a base sequence of 150 bp at locus +58 (e.g., as shown in SEQ ID NO: 2) has a great influence on the efficiency of gene editing. The region targeted by the sgRNAs of the present invention is an editable region and may be used efficiently for clinical application. The 150 bp genomic sequence is located in the region from base 60495197 to base 60495346 (abbreviated herein as chr2: 60495197-60495346) on human chromosome 2.

All the sgRNAs provided in the invention may be used to efficiently realize gene editing of the hematopoietic stem cells from both healthy donors and anemia patients, wherein the candidate sgRNAs with relatively higher efficiency may obviously improve the expression of fetal hemoglobin. It was reported in the literatures that, by designing sgRNAs libraries for loci +55, +58, and +62, and screening with cell models by using CRISPR/Cas9, a sequence targeting "GATAA" at locus +58 is found to be a key regulatory sequence, i.e., an sgRNA containing the "GATAA" sequence is most effective in increasing fetal hemoglobin. However, it is noteworthy that the the region around +58k anchored by the sgRNA being discovered in the present invention is distinct from the critical base site of "GATAA" disclosed in the prior art. The gene editing of introducing the sgRNAs provided in this invention significantly increases fetal hemoglobin expression, and it is adequate for the clinical treatment.

Those skilled in the art can understand that after acquiring a high efficient gene editing region, one skilled in the art can edit the known high efficient gene editing region and optimize the gene editing conditions to achieve high editing efficiency by using any gene editing methods, such as zinc finger nuclease-based gene editing technology, TALEN gene editing technology, CRISPR/Cas (e.g., CRISPR/Cas9) gene editing technology, as well as other gene editing methods found in the future. Accordingly, the present invention comprises any of the technical schemes for performing gene editing with a BCL11A gene targeting sequence selected from SEQ ID NOs: 3-25 and identified by the present invention through any available gene editing method. In some preferred embodiments, the present invention relates to a solution for achieving efficient gene editing by CRISPR/Cas9 gene editing technology.

As used herein, "CRISPR/Cas" is a gene editing technology including, but not limited to, various naturally occurring or artificially designed CRISPR/Cas systems, such as the CRISPR/Cas9 system. Naturally occurring CRISPR/Cas system is an adaptive immune defense formed in bacteria and archaea during long-term evolution, and is used to combat invading viruses and foreign DNAs. For example, CRISPR/Cas9 works on the principle that crRNA (CRISPR-derived RNA) binds to tracrRNA (trans-activating RNA) through base pairing to form a tracrRNA/crRNA complex, which directs the nuclease Cas9 protein to cleave the double-stranded DNA at a target site in a sequence matching with the crRNA. However, a single guide RNA (sgRNA) with the guiding ability may be formed by transformation with artificially designed tracrRNA and the crRNA, the sgRNA is able to guide a Cas9 to perform site-specific cleavage of DNA. As an RNA-guided dsDNA-binding protein, the Cas9 effector nuclease is able to co-localize RNA, DNA and proteins, thereby providing tremendous potential to be engineered. Cas proteins of type I, II or III may be used in CRISPR/Cas system. In some embodiments of the invention, Cas9 is taken in the method. Other suitable CRISPR/Cas systems include, but are not limited to, the systems and methods described in WO2013176772, WO2014065596, WO2014018423, and US8,697,359.

In another aspect, the invention relates to a series of sgRNA molecules of the invention that enable efficient gene editing.

In the present invention, an "sgRNA", a "gRNA", a "single guide RNA", a "synthetic guide RNA", or a "guide RNA" are used interchangeably. The sgRNA of present invention comprises a guide sequence targeting a target sequence. In a preferred embodiment, the sgRNA of the present invention further comprises a tracr sequence and a tracr chaperone sequence.

A "guide sequence" in the present invention may be a sequence of about 17-20 bp with specified targeting site, and may be used interchangeablely with a "leader sequence" or a "spacer". In the context of formation of a CRISPR complex, a "target sequence" is, for example, a sequence which a guide sequence is designed to be complementary to it, wherein hybridization between a target sequence and the guide sequence promotes the formation of a CRISPR complex, and the hybridization requires that the complementarity between the "target sequence" and the "guide sequence" or the "leader sequence" is sufficient for inducing hybridization and promoting the formation of the CRISPR complex, while a complete complementarity is not a must.

"Complementary" means that a "guide sequence" or "leader sequence" may hybridize to a target nucleotide sequence (in the context of the present invention, a target nucleotide sequence of BCL11A gene in the hematopoietic stem cells) by the nucleotide pairing principle found by Watson and Crick. It will be appreciated by those skilled in the art that a "guide sequence" may hybridize to a target nucleotide sequence so long as it is sufficiently complementary to the target nucleotide sequence, while 100% and complete complementarity between them is not a must. In some embodiments, the degree of complementarity between the guide sequence and its corresponding target sequence may be about or higher than about 75%, 80%, 85%, 90%, 91%, 92%, 93%, 94%, 95%, 96%, 97%, 98%, 99% or higher, when they are optimally aligned by using a suitable alignment algorithm. The optimal alignment may be determined by using any suitable algorithm for aligning sequences, including the Smith-Waterman algorithm, the Needleman-Wimsch algorithm, the Burrows-Wheeler Transform based algorithm, etc.

In general, in the context of an endogenous CRISPR system, the formation of a CRISPR complex (including the hybridization of a guide sequence to a target sequence, and then complexing with one or more Cas proteins) results in the cleavage of one or both strands in or near the target sequence (e.g., within the range of 1, 2, 3, 4, 5, 6, 7, 8, 9, 10, 20, 50 or more base pairs from the target sequence). Without wishing to be bound by theory, the tracr sequence may comprise all or a part of the wild-type tracr sequence, (e.g., about or more than about 20, 23, 26, 29, 32, 35, 38, 41, 44, 47, 50, 53, 56, 59, 62, 65, 70, 75, 80, 85 or more nucleotides of the wild-type tracr sequence), or a tracr sequence comprising the foregoing may also form a part of a CRISPR complex, for example by hybridizing along at least a part of the tracr sequence to all or a part of the tracr chaperone sequence which is operably linked to the guide sequence.

In some embodiments, the tracr sequence is sufficiently complementary to the tracr chaperone sequence to hybridize and participate in the formation of a CRISPR complex. Like the case of hybridizing a"target sequence" to a "guide sequence" or a "leader sequence ", complete complementarity is not necessary so long as it is enough to perform its function. In some embodiments, in the case of optimal alignment, the tracr sequence exhibits at least 50%, 60%, 70%, 80%, 90%, 95%, or 99% complementarity along the length of the tracr chaperone sequence.

The BCL11A genomic region from positions 60495219-60495336 in the chromosome 2 in the hematopoietic stem cells mentioned in the present application is defined according to the positions in the standard human gene sequence of GRCh38. Those skilled in the art will appreciate that the positions may vary with reference to different standard human genomic sequences, but those skilled in the art can understand the corresponding positions of the mentioned region while referring to different standard human gene sequences. In some embodiments, the disruption of the BCL11A genomic region from positions 60495219-60495336 in the chromosome 2 in hematopoietic stem cells comprises the introduction of "insertions and/or deletions (Indel)" of nucleotide sequences into this region, e.g., indels of any type (e.g., selected from A, T, C, G) and/or any number (e.g., 1-20, 1-15, 1-10, 1-9, 1-8, 1-7, 1-6, 1-5, 1-4, 1-3, 1-2) of nucleotides. In some embodiments, the disruption comprises replacing the original nucleotide sequence with a new nucleotide sequence. In some embodiments, the disruption comprises the knock-in or knock-out of a nucleotide sequence in the region.

In the present application, the targeting sequence of BCL11A locus in the hematopoietic stem cells is shown as SEQ ID NO: 2. The sgRNAs of the present application are designed for the 150 bp base region shown in SEQ ID NO: 2, and the sgRNA sequences are required to be complementary to a sequence of at least 17, preferably 18, preferably 19, or preferably 20 contiguous nucleotides in the sequence of SEQ ID NO: 2.

In some embodiments, the present invention provides a method for increasing the fetal hemoglobin (HbF) expression in human hematopoietic stem cells, comprising: disrupting a BCL11A genomic region from positions 60495203-60495222, particularly position 60495219, in the chromosome 2 in the hematopoietic stem cells by gene editing technology. The gene editing technology is a zinc finger nuclease-based gene editing technology, a TALEN gene editing technology or a CRISPR/Cas gene editing technology, preferably a CRISPR/Cas9 gene editing technology, and preferably the target nucleotide sequence in the BCL11A genome is complementary to the guide sequence of the sgRNA comprising SEQ ID NO: 9. The method of the present invention involves introducing an sgRNA comprising the sequence of SEQ ID NO: 9 into hematopoietic stem cells to effectively edit said BCL11A genome, preferably co-introducing the sgRNA with Cas9-encoding nucleotides (e.g., mRNA) into the hematopoietic stem cells, preferably co-introducing the sgRNA with Cas9-encoding nucleotides into the hematopoietic stem cells by electroporation under the electroporation conditions of 200-600 V, 0.5-2 ms. In some embodiments, the sgRNA is 2'-O-methyl modified and/or internucleotide 3'-thio modified, e.g., the chemical modification is 2'-O-methyl modification of the first one, two and/or three bases at the 5' end and/or the last base at the 3' end of the sgRNA. In some embodiments, the invention also relates to a hematopoietic stem cell, wherein one or more sites in the target sequence of the BCL11A genome from positions 60495203-60495222, particularly position 60495219 in the chromosome 2 in the hematopoietic stem cell are disrupted by gene editing technology. Further, the invention relates to erythrocytes obtained from *in vitro* differentiation culture of the hematopoietic stem cells, and medical preparations containing the erythrocytes.

In some embodiments, the present invention provides a method for increasing the fetal hemoglobin (HbF) expression in human hematopoietic stem cells, comprising: disrupting a BCL11A genomic region from positions 60495208-60495227, particularly position 60495224, in the chromosome 2 in the hematopoietic stem cells by gene editing technology. The gene editing technology is a zinc finger nuclease-based gene editing technology, a TALEN gene editing technology or a CRISPR/Cas gene editing technology, preferably a CRISPR/Cas9 gene editing technology, preferably the target nucleotide sequence in the BCL11A genome is complementary to a guide sequence comprising the sgRNA of SEQ ID NO: 10. The method of the present invention involves introducing an sgRNA comprising the sequence of SEQ ID NO: 10 into hematopoietic stem cells to effectively edit said BCL11A genome, preferably co-introducing the sgRNA with Cas9-encoding nucleotides (e.g., mRNA) into the hematopoietic stem cells, preferably co-introducing the sgRNA with Cas9-encoding nucleotides into the hematopoietic stem cells by electroporation under the electroporation conditions of 200-600 V, 0.5-2 ms. In some embodiments, the sgRNA is 2'-O-methyl modified and/or internucleotide 3'-thio modified, e.g., the chemical modification is 2'-O-methyl modification of the first one, two and/or three bases at the 5' end and/or the last base at the 3' end of the sgRNA. In some embodiments, the invention also relates to a hematopoietic stem cell, wherein one or more sites in the target sequence of the BCL11A genome from positions 60495208-60495227, particularly position 60495224 in the chromosome 2 in the hematopoietic stem cell are disrupted by gene editing technology. Further, the invention relates to erythrocytes obtained from *in vitro* differentiation culture of the hematopoietic stem cells, and medical preparations containing the erythrocytes.

In some embodiments, the present invention provides a method for increasing the fetal hemoglobin (HbF) expression in human hematopoietic stem cells, comprising: disrupting a BCL11A genomic region from positions 60495217-60495236, particularly position 60495233 in the chromosome 2 in the hematopoietic stem cells by gene editing technology. The gene editing technology is a zinc finger nuclease-based gene editing technology, a TALEN gene editing technology or a CRISPR/Cas gene editing technology, preferably a CRISPR/Cas9 gene editing technology, and preferably the target nucleotide sequence in the BCL11A genome is complementary to a guide sequence of the sgRNA comprising SEQ ID NO: 11. The method of the present invention involves introducing an sgRNA comprising the sequence of SEQ ID NO: 11 into hematopoietic stem cells to effectively edit said BCL11A genome, preferably co-introducing the sgRNA with Cas9-encoding nucleotides (e.g., mRNA) into the hematopoietic stem cells, preferably co-introducing the sgRNA with Cas9-encoding nucleotides into the hematopoietic stem cells by electroporation under the electroporation conditions of 200-600 V, 0.5-2 ms. In some embodiments, the sgRNA is 2'-O-methyl modified and/or internucleotide 3'-thio modified, e.g., the chemical modification is 2'-O-methyl modification of the first one, two and/or three bases at the 5' end and/or the last base at the 3' end of the sgRNA. In some embodiments, the invention also relates to a hematopoietic stem cell, wherein one or more sites on the target sequence of the BCL11A genome from positions 60495217-60495236, particularly position 60495233 in the chromosome 2 in the hematopoietic stem cell are disrupted by gene editing technology. Further, the invention relates to erythrocytes obtained from *in vitro* differentiation culture of the hematopoietic stem cells, and medical preparations containing the erythrocytes.

In some embodiments, the present invention provides a method for increasing the fetal hemoglobin (HbF) expression in human hematopoietic stem cells, comprising: disrupting a BCL11A genomic region from positions 60495218-60495237, particularly position 60495234 in the chromosome 2 in the hematopoietic stem cells by gene editing technology. The gene editing technology is a zinc finger nuclease-based gene editing technology, a TALEN gene editing technology or a CRISPR/Cas gene editing technology, preferably a CRISPR/Cas9 gene editing technology, and preferably the target nucleotide sequence in the BCL11A genome is complementary to a guide sequence of the sgRNA comprising SEQ ID NO: 12. The method of the present invention involves introducing an sgRNA comprising the sequence of SEQ ID NO: 12 into hematopoietic stem cells to effectively edit said BCL11A genome, preferably co-introducing the sgRNA with Cas9-encoding nucleotides (e.g., mRNA) into the hematopoietic stem cells, preferably co-introducing the sgRNA with Cas9-encoding nucleotides into the hematopoietic stem cells by electroporation under the electroporation conditions of 200-600 V, 0.5-2 ms. In some embodiments, the sgRNA is 2'-O-methyl modified and/or internucleotide 3'-thio modified, e.g., the chemical modification is 2'-O-methyl modification of the first one, two and/or three bases at the 5' end and/or the last base at the 3' end of the sgRNA. In some embodiments, the invention also relates to a hematopoietic stem cell, wherein one or more sites in the target sequence of the BCL11A genome from positions 60495218-60495237, particularly position 60495234 in the chromosome 2 in the hematopoietic stem cell are disrupted by gene editing technology. Further, the invention relates to erythrocytes obtained from *in vitro* differentiation culture of the hematopoietic stem cells, and medical preparations containing the erythrocytes.

In some embodiments, the present invention provides a method for increasing the fetal hemoglobin (HbF) expression in human hematopoietic stem cells, comprising: disrupting a BCL11A genomic region from positions 60495219-60495238, particularly position 60495235 in the chromosome 2 in the hematopoietic stem cells by gene editing technology. The gene editing technology is a zinc finger nuclease-based gene editing technology, a TALEN gene editing technology or a CRISPR/Cas gene editing technology, preferably a CRISPR/Cas9 gene editing technology, preferably the target nucleotide sequence in the BCL11A genome is complementary to a guide sequence of the sgRNA comprising SEQ ID NO: 13. The method of the present invention involves introducing an sgRNA comprising the sequence of SEQ ID NO: 13 into hematopoietic stem cells to effectively edit said BCL11A genome, preferably co-introducing the sgRNA with Cas9-encoding nucleotides (e.g., mRNA) into the hematopoietic stem cells, preferably co-introducing the sgRNA with Cas9-encoding nucleotides into the hematopoietic stem cells by electroporation under the electroporation conditions of 200-600 V, 0.5-2 ms. In some embodiments, the sgRNA is 2'-O-methyl modified and/or internucleotide 3'-thio modified, e.g., the chemical modification is 2'-O-methyl modification of the first one, two and/or three bases at the 5' end and/or the last base at the 3' end of the sgRNA. In some embodiments, the invention also relates to a hematopoietic stem cell, wherein one or more sites in the target sequence of the BCL11A genome from positions 60495219-60495238, particularly position 60495235 in the chromosome 2 in the hematopoietic stem cell are disrupted by gene editing technology. Further, the invention relates to erythrocytes obtained from *in vitro* differentiation culture of the hematopoietic stem cells, and medical preparations containing the erythrocytes.

In some embodiments, the present invention provides a method for increasing the fetal hemoglobin (HbF) expression in human hematopoietic stem cells, comprising: disrupting a BCL11A genomic region from positions 60495221-60495240, particularly position 60495223 in the chromosome 2 in the hematopoietic stem cells by gene editing technology. The gene editing technology is a zinc finger nuclease-based gene editing technology, a TALEN gene editing technology or a CRISPR/Cas gene editing technology, preferably a CRISPR/Cas9 gene editing technology, and preferably the target nucleotide sequence in the BCL11A genome is complementary to a guide sequence of the sgRNA comprising SEQ ID NO: 16. The method of the present invention involves introducing an sgRNA comprising the sequence of SEQ ID NO: 16 into hematopoietic stem cells to effectively edit said BCL11A genome, preferably co-introducing the sgRNA with Cas9-encoding nucleotides (e.g., mRNA) into the hematopoietic stem cells, preferably co-introducing the sgRNA with Cas9-encoding nucleotides into the hematopoietic stem cells by electroporation under the electroporation conditions of 200-600 V, 0.5-2 ms. In some embodiments, the sgRNA is 2'-O-methyl modified and/or internucleotide 3'-thio modified, e.g., the chemical modification is 2'-O-methyl modification of the first one, two and/or three bases at the 5' end and/or the last base at the 3' end of the sgRNA. In some embodiments, the invention also relates to a hematopoietic stem cell, wherein one or more sites in the target sequence of the BCL11A genome from positions 60495221-60495240, particularly position 60495223 in the chromosome 2 in the hematopoietic stem cell are disrupted by gene editing technology. Further, the invention relates to erythrocytes obtained from *in vitro* differentiation culture of the hematopoietic stem cells, and medical preparations containing the erythrocytes.

In some embodiments, the present invention provides a method for increasing the fetal hemoglobin (HbF) expression in human hematopoietic stem cells, comprising: disrupting a BCL11A genomic region from positions 60495222-60495241, particularly position 60495238 in the chromosome 2 in the hematopoietic stem cells by gene editing technology. The gene editing technology is a zinc finger nuclease-based gene editing technology, a TALEN gene editing technology or a CRISPR/Cas gene editing technology, preferably a CRISPR/Cas9 gene editing technology, preferably the target nucleotide sequence in the BCL11A genome is complementary to a guide sequence of the sgRNA comprising SEQ ID NO: 14. The method of the present invention involves introducing an sgRNA comprising the sequence of SEQ ID NO: 14 into hematopoietic stem cells to effectively edit said BCL11A genome, preferably co-introducing the sgRNA with Cas9-encoding nucleotides (e.g., mRNA) into the hematopoietic stem cells, preferably co-introducing the sgRNA with Cas9-encoding nucleotides into the hematopoietic stem cells by electroporation under the electroporation conditions of 200-600 V for 0.5-2 ms. In some embodiments, the sgRNA is 2'-O-methyl modified and/or internucleotide 3'-thio modified, e.g., the chemical modification is 2'-O-methyl modification of the first one, two and/or three bases at the 5' end and/or the last base at the 3' end of the sgRNA. In some embodiments, the invention also relates to a hematopoietic stem cell, wherein one or more sites the target sequence of the BCL11A genome from positions 60495222-60495241, particularly position 60495238 in the chromosome 2 in the hematopoietic stem cell are disrupted by gene editing technology. Further, the invention relates to erythrocytes obtained from *in vitro* differentiation culture of the hematopoietic stem cells, and medical preparations containing the erythrocytes.

In some embodiments, the present invention provides a method for increasing the fetal hemoglobin (HbF) expression in human hematopoietic stem cells, comprising: disrupting a BCL11A genomic region from positions 60495223-60495242, particularly position 60495239 in the chromosome 2 in the hematopoietic stem cells by gene editing technology. The gene editing technology is a zinc finger nuclease-based gene editing technology, a TALEN gene editing technology or a CRISPR/Cas gene editing technology, preferably a CRISPR/Cas9 gene editing technology, and preferably the target nucleotide sequence in the BCL11A genome is complementary to a guide sequence of the sgRNA comprising SEQ ID NO: 15. The method of the present invention involves introducing an sgRNA comprising the sequence of SEQ ID NO: 15 into hematopoietic stem cells to effectively edit said BCL11A genome, preferably co-introducing the sgRNA with Cas9-encoding nucleotides (e.g., mRNA) into the hematopoietic stem cells, preferably co-introducing the sgRNA with Cas9-encoding nucleotides into the hematopoietic stem cells by electroporation under the electroporation conditions of 200-600 V, 0.5-2 ms. In some embodiments, the sgRNA is 2'-O-methyl modified and/or internucleotide 3'-thio modified, e.g., the chemical modification is 2'-O-methyl modification of the first one, two and/or three bases at the 5' end and/or the last base at the 3' end of the sgRNA. In some embodiments, the invention also relates to a hematopoietic stem cell, wherein one or more sites in the target sequence of the BCL11A genome from positions 60495223-60495242, particularly position 60495239 in the chromosome 2 in the hematopoietic stem cell are disrupted by gene editing technology. Further, the invention relates to erythrocytes obtained from *in vitro* differentiation culture of the hematopoietic stem cells, and medical preparations containing the erythrocytes.

In some embodiments, the present invention provides a method for increasing the fetal hemoglobin (HbF) expression in human hematopoietic stem cells, comprising: disrupting a BCL11A genomic region from positions 60495228-60495247, particularly position 60495244 in the chromosome 2 in the hematopoietic stem cells by gene editing technology. The gene editing technology is a zinc finger nuclease-based gene editing technology, a TALEN gene editing technology or a CRISPR/Cas gene editing technology, preferably a CRISPR/Cas9 gene editing technology, preferably the target nucleotide sequence in the BCL11A genome is complementary to a guide sequence of the sgRNA comprising SEQ ID NO: 17. The method of the present invention involves introducing an sgRNA comprising the sequence of SEQ ID NO: 17 into hematopoietic stem cells to effectively edit said BCL11A genome, preferably co-introducing the sgRNA with Cas9-encoding nucleotides (e.g., mRNA) into the hematopoietic stem cells, preferably co-introducing the sgRNA with Cas9-encoding nucleotides into the hematopoietic stem cells by electroporation under the electroporation conditions of 200-600 V, 0.5-2 ms. In some embodiments, the sgRNA is 2'-O-methyl modified and/or internucleotide 3'-thio modified, e.g., the chemical modification is 2'-O-methyl modification of the first one, two and/or three bases at the 5' end and/or the last base at the 3' end of the sgRNA. In some embodiments, the invention also relates to a hematopoietic stem cell, wherein one or more sites in the target sequence of the BCL11A genome from positions 60495228-60495247, particularly position 60495244 in the chromosome 2 in the hematopoietic stem cell are disrupted by gene editing technology. Further, the invention relates to erythrocytes obtained from *in vitro* differentiation culture of the hematopoietic stem cells, and medical preparations containing the erythrocytes.

In some embodiments, the present invention provides a method for increasing the fetal hemoglobin (HbF) expression in human hematopoietic stem cells, comprising: disrupting a BCL11A genomic region from positions 60495229-60495248, particularly position 60495245 in the chromosome 2 in the hematopoietic stem cells by gene editing technology. The gene editing technology is a zinc finger nuclease-based gene editing technology, a TALEN gene editing technology or a CRISPR/Cas gene editing technology, preferably a CRISPR/Cas9 gene editing technology, preferably the target nucleotide sequence in the BCL11A genome is complementary to a guide sequence of the sgRNA comprising SEQ ID NO: 18. The method of the present invention involves introducing an sgRNA comprising the sequence of SEQ ID NO: 18 into hematopoietic stem cells to effectively edit said BCL11A genome, preferably co-introducing the sgRNA with Cas9-encoding nucleotides (e.g., mRNA) into the hematopoietic stem cells, preferably co-introducing the sgRNA with Cas9-encoding nucleotides into the hematopoietic stem cells by electroporation under the electroporation conditions of 200-600 V, 0.5-2 ms. In some embodiments, the sgRNA is 2'-O-methyl modified and/or internucleotide 3'-thio modified, e.g., the chemical modification is 2'-O-methyl modification of the first one, two and/or three bases at the 5' end and/or the last base at the 3' end of the sgRNA. In some embodiments, the invention also relates to a hematopoietic stem cell, wherein one or more sites in the target sequence of the BCL11A genome from positions 60495229-60495248, particularly position 60495245 in the chromosome 2 in the hematopoietic stem cell are disrupted by gene editing technology. Further, the invention relates to erythrocytes obtained from *in vitro* differentiation culture of the hematopoietic stem cells, and medical preparations containing the erythrocytes.

In some embodiments, the present invention provides a method for increasing the fetal hemoglobin (HbF) expression in human hematopoietic stem cells, comprising: disrupting a BCL11A genomic region from positions 60495230-60495249, particularly position 60495246 in the chromosome 2 in the hematopoietic stem cells by gene editing technology. The gene editing technology is a zinc finger nuclease-based gene editing technology, a TALEN gene editing technology or a CRISPR/Cas gene editing technology, preferably a CRISPR/Cas9 gene editing technology, and preferably the target nucleotide sequence in the BCL11A genome is complementary to a guide sequence of the sgRNA comprising SEQ ID NO: 19. The method of the present invention involves introducing an sgRNA comprising the sequence of SEQ ID NO: 19 into hematopoietic stem cells to effectively edit said BCL11A genome, preferably co-introducing the sgRNA with Cas9-encoding nucleotides (e.g., mRNA) into the hematopoietic stem cells, preferably co-introducing the sgRNA with Cas9-encoding nucleotides into the hematopoietic stem cells by electroporation under the electroporation conditions of 200-600 V, 0.5-2 ms. In some embodiments, the sgRNA is 2'-O-methyl modified and/or internucleotide 3'-thio modified, e.g., the chemical modification is 2'-O-methyl modification of the first one, two and/or three bases at the 5' end and/or the last base at the 3' end of the sgRNA. In some embodiments, the invention also relates to a hematopoietic stem cell, wherein one or more sites on the BCL11A genomic target sequence from positions 60495230-60495249, particularly position 60495246 in the chromosome 2 in the hematopoietic stem cell are disrupted by gene editing technology. Further, the invention relates to erythrocytes obtained from *in vitro* differentiation culture of the hematopoietic stem cells, and medical preparations containing the erythrocytes.

In some embodiments, the present invention provides a method for increasing the fetal hemoglobin (HbF) expression in human hematopoietic stem cells, comprising: disrupting a BCL11A genomic region from positions 60495231-60495250, particularly position 60495247 in the chromosome 2 in the hematopoietic stem cells by gene editing technology. The gene editing technology is a zinc finger nuclease-based gene editing technology, a TALEN gene editing technology or a CRISPR/Cas gene editing technology, preferably a CRISPR/Cas9 gene editing technology, and preferably the target nucleotide sequence in the BCL11A genome is complementary to a guide sequence of the sgRNA comprising SEQ ID NO: 20. The method of the present invention involves introducing an sgRNA comprising the sequence of SEQ ID NO: 20 into hematopoietic stem cells to effectively edit said BCL11A genome, preferably co-introducing the sgRNA with Cas9-encoding nucleotides (e.g., mRNA) into the hematopoietic stem cells, preferably co-introducing the sgRNA with Cas9-encoding nucleotides into the hematopoietic stem cells by electroporation under the electroporation conditions of 200-600 V, 0.5-2 ms. In some embodiments, the sgRNA is 2'-O-methyl modified and/or internucleotide 3'-thio modified, e.g., the chemical modification is 2'-O-methyl modification of the first one, two and/or three bases at the 5' end and/or the last base at the 3' end of the sgRNA. In some embodiments, the invention also relates to a hematopoietic stem cell, wherein one or more sites on the BCL11A genomic target sequence from positions 60495231-60495250, particularly position 60495247 in the chromosome 2 in the hematopoietic stem cell are disrupted by gene editing technology. Further, the invention relates to erythrocytes obtained from *in vitro* differentiation culture of the hematopoietic stem cells, and medical preparations containing the erythrocytes.

In some embodiments, the present invention provides a method for increasing the fetal hemoglobin (HbF) expression in human hematopoietic stem cells, comprising: disrupting a BCL11A genomic region from positions 60495234-60495253, particularly position 60495250 in the chromosome 2 in the hematopoietic stem cells by gene editing technology. The gene editing technology is a zinc finger nuclease-based gene editing technology, a TALEN gene editing technology or a CRISPR/Cas gene editing technology, preferably a CRISPR/Cas9 gene editing technology, preferably the target nucleotide sequence in the BCL11A genome is complementary to a guide sequence of the sgRNA comprising SEQ ID NO: 21. The method of the present invention involves introducing an sgRNA comprising the sequence of SEQ ID NO: 21 into hematopoietic stem cells to effectively edit said BCL11A genome, preferably co-introducing the sgRNA with Cas9-encoding nucleotides (e.g., mRNA) into the hematopoietic stem cells, preferably co-introducing the sgRNA with Cas9-encoding nucleotides into the hematopoietic stem cells by electroporation under the electroporation conditions of 200-600 V, 0.5-2 ms. In some embodiments, the sgRNA is 2'-O-methyl modified and/or internucleotide 3'-thio modified, e.g., the chemical modification is 2'-O-methyl modification of the first one, two and/or three bases at the 5' end and/or the last base at the 3' end of the sgRNA. In some embodiments, the invention also relates to a hematopoietic stem cell, wherein one or more sites in the target sequence of the BCL11A genome from positions 60495234-60495253, particularly position 60495250 in the chromosome 2 in the hematopoietic stem cell are disrupted by gene editing technology. Further, the invention relates to erythrocytes obtained from *in vitro* differentiation culture of the hematopoietic stem cells, and medical preparations containing the erythrocytes.

In some embodiments, the present invention provides a method for increasing the fetal hemoglobin (HbF) expression in human hematopoietic stem cells, comprising: disrupting a BCL11A genomic region from positions 60495235-60495254, particularly position 60495251 in the chromosome 2 in the hematopoietic stem cells by gene editing technology. The gene editing technology is a zinc finger nuclease-based gene editing technology, a TALEN gene editing technology or a CRISPR/Cas gene editing technology, preferably a CRISPR/Cas9 gene editing technology, preferably the target nucleotide sequence in the BCL11A genome is complementary to a guide sequence of the sgRNA comprising SEQ ID NO: 22. The method of the present invention involves introducing an sgRNA comprising the sequence of SEQ ID NO: 22 into hematopoietic stem cells to effectively edit said BCL11A genome, preferably co-introducing the sgRNA with Cas9-encoding nucleotides (e.g., mRNA) into the hematopoietic stem cells, preferably co-introducing the sgRNA with Cas9-encoding nucleotides into the hematopoietic stem cells by electroporation under the electroporation conditions of 200-600 V, 0.5-2 ms. In some embodiments, the sgRNA is 2'-O-methyl modified and/or internucleotide 3'-thio modified, e.g., the chemical modification is 2'-O-methyl modification of the first one, two and/or three bases at the 5' end and/or the last base at the 3' end of the sgRNA. In some embodiments, the invention also relates to a hematopoietic stem cell, wherein one or more sites in the target sequence of the BCL11A genome from positions 60495235-60495254, particularly position 60495251 in the chromosome 2 in the hematopoietic stem cell are disrupted by gene editing technology. Further, the invention relates to erythrocytes obtained from *in vitro* differentiation culture of the hematopoietic stem cells, and medical preparations containing the erythrocytes.

In some embodiments, the present invention provides a method for increasing the fetal hemoglobin (HbF) expression in human hematopoietic stem cells, comprising: disrupting a BCL11A genomic region from positions 60495236-60495255, particularly position 60495238 in the chromosome 2 in the hematopoietic stem cells by gene editing technology. The gene editing technology is a zinc finger nuclease-based gene editing technology, a TALEN gene editing technology or a CRISPR/Cas gene editing technology, preferably a CRISPR/Cas9 gene editing technology, preferably the target nucleotide sequence in the BCL11A genome is complementary to a guide sequence of the sgRNA comprising SEQ ID NO: 4. The method of the present invention involves introducing an sgRNA comprising the sequence of SEQ ID NO: 4 into hematopoietic stem cells to effectively edit said BCL11A genome, preferably co-introducing the sgRNA with Cas9-encoding nucleotides (e.g., mRNA) into the hematopoietic stem cells, preferably co-introducing the sgRNA with Cas9-encoding nucleotides into the hematopoietic stem cells by electroporation under the electroporation conditions of 200-600 V, 0.5-2 ms. In some embodiments, the sgRNA is 2'-O-methyl modified and/or internucleotide 3'-thio modified, e.g., the chemical modification is 2'-O-methyl modification of the first one, two and/or three bases at the 5' end and/or the last base at the 3' end of the sgRNA. In some embodiments, the invention also relates to a hematopoietic stem cell, wherein one or more sites in the target sequence of the BCL11A genome from positions 60495236-60495255, particularly position 60495238 in the chromosome 2 in the hematopoietic stem cell are disrupted by gene editing technology. Further, the invention relates to erythrocytes obtained from *in vitro* differentiation culture of the hematopoietic stem cells, and medical preparations containing the erythrocytes.

In some embodiments, the present invention provides a method for increasing the fetal hemoglobin (HbF) expression in human hematopoietic stem cells, comprising: disrupting a BCL11A genomic region from positions 60495247-60495266, particularly position 60495263 in the chromosome 2 in the hematopoietic stem cells by gene editing technology. The gene editing technology is a zinc finger nuclease-based gene editing technology, a TALEN gene editing technology or a CRISPR/Cas gene editing technology, preferably a CRISPR/Cas9 gene editing technology, and preferably the target nucleotide sequence in the BCL11A genome is complementary to a guide sequence of the sgRNA comprising SEQ ID NO: 3. The method of the present invention involves introducing an sgRNA comprising the sequence of SEQ ID NO: 3 into hematopoietic stem cells to effectively edit said BCL11A genome, preferably co-introducing the sgRNA with Cas9-encoding nucleotides (e.g., mRNA) into the hematopoietic stem cells, preferably co-introducing the sgRNA with Cas9-encoding nucleotides into the hematopoietic stem cells by electroporation under the electroporation conditions of 200-600 V, 0.5-2 ms. In some embodiments, the sgRNA is 2'-O-methyl modified and/or internucleotide 3'-thio modified, e.g., the chemical modification is 2'-O-methyl modification of the first one, two and/or three bases at the 5' end and/or the last base at the 3' end of the sgRNA. In some embodiments, the invention also relates to a hematopoietic stem cell, wherein one or more sites in the target sequence of the BCL11A genome from positions 60495247-60495266, particularly position 60495263 in the chromosome 2 in the hematopoietic stem cell are disrupted by gene editing technology. Further, the invention relates to erythrocytes obtained from *in vitro* differentiation culture of the hematopoietic stem cells, and medical preparations containing the erythrocytes.

In some embodiments, the present invention provides a method for increasing the fetal hemoglobin (HbF) expression in human hematopoietic stem cells, comprising: disrupting a BCL11A genomic region from positions 60495252-60495271, particularly position 60495268 in the chromosome 2 in the hematopoietic stem cells by gene editing technology. The gene editing technology is a zinc finger nuclease-based gene editing technology, a TALEN gene editing technology or a CRISPR/Cas gene editing technology, preferably a CRISPR/Cas9 gene editing technology, and preferably the target nucleotide sequence in the BCL11A genome is complementary to a guide sequence of the sgRNA comprising SEQ ID NO: 8. The method of the present invention involves introducing an sgRNA comprising the sequence of SEQ ID NO: 8 into hematopoietic stem cells to effectively edit said BCL11A genome, preferably co-introducing the sgRNA with Cas9-encoding nucleotides (e.g., mRNA) into the hematopoietic stem cells, preferably co-introducing the sgRNA with Cas9-encoding nucleotides into the hematopoietic stem cells by electroporation under the electroporation conditions of 200-600 V, 0.5-2 ms. In some embodiments, the sgRNA is 2'-O-methyl modified and/or internucleotide 3'-thio modified, e.g., the chemical modification is 2'-O-methyl modification of the first one, two and/or three bases at the 5' end and/or the last base at the 3' end of the sgRNA. In some embodiments, the invention also relates to a hematopoietic stem cell, wherein one or more sites in the target sequence of the BCL11A genome from positions 60495252-60495271, particularly position 60495268 in the chromosome 2 in the hematopoietic stem cell are disrupted by gene editing technology. Further, the invention relates to erythrocytes obtained from *in vitro* differentiation culture of the hematopoietic stem cells, and medical preparations containing the erythrocytes.

In some embodiments, the present invention provides a method for increasing the fetal hemoglobin (HbF) expression in human hematopoietic stem cells, comprising: disrupting a BCL11A genomic region from positions 60495253-60495272, particularly position 60495269 in the chromosome 2 in the hematopoietic stem cells by gene editing technology. The gene editing technology is a zinc finger nuclease-based gene editing technology, a TALEN gene editing technology or a CRISPR/Cas gene editing technology, preferably a CRISPR/Cas9 gene editing technology, and preferably the target nucleotide sequence in the BCL11A genome is complementary to a guide sequence of the sgRNA comprising SEQ ID NO: 7. The method of the present invention involves introducing an sgRNA comprising the sequence of SEQ ID NO: 7 into hematopoietic stem cells to effectively edit said BCL11A genome, preferably co-introducing the sgRNA with Cas9-encoding nucleotides (e.g., mRNA) into the hematopoietic stem cells, preferably co-introducing the sgRNA with Cas9-encoding nucleotides into the hematopoietic stem cells by electroporation under the electroporation conditions of 200-600 V, 0.5-2 ms. In some embodiments, the sgRNA is 2'-O-methyl modified and/or internucleotide 3'-thio modified, e.g., the chemical modification is 2'-O-methyl modification of the first one, two and/or three bases at the 5' end and/or the last base at the 3' end of the sgRNA. In some embodiments, the invention also relates to a hematopoietic stem cell, wherein one or more sites in the target sequence of the BCL11A genome from positions 60495253-60495272, particularly position 60495269 in the chromosome 2 in the hematopoietic stem cell are disrupted by gene editing technology. Further, the invention relates to erythrocytes obtained from *in vitro* differentiation culture of the hematopoietic stem cells, and medical preparations containing the erythrocytes.

In some embodiments, the present invention provides a method for increasing the fetal hemoglobin (HbF) expression in human hematopoietic stem cells, comprising: disrupting a BCL11A genomic region from positions 60495257-60495276, particularly position 60495273 in the chromosome 2 in the hematopoietic stem cells by gene editing technology. The gene editing technology is a zinc finger nuclease-based gene editing technology, a TALEN gene editing technology or a CRISPR/Cas gene editing technology, preferably a CRISPR/Cas9 gene editing technology, and preferably the target nucleotide sequence in the BCL11A genome is complementary to a guide sequence of the sgRNA comprising SEQ ID NO: 6. The method of the present invention involves introducing an sgRNA comprising the sequence of SEQ ID NO: 6 into hematopoietic stem cells to effectively edit said BCL11A genome, preferably co-introducing the sgRNA with Cas9-encoding nucleotides (e.g., mRNA) into the hematopoietic stem cells, preferably co-introducing the sgRNA with Cas9-encoding nucleotides into the hematopoietic stem cells by electroporation under the electroporation conditions of 200-600 V, 0.5-2 ms. In some embodiments, the sgRNA is 2'-O-methyl modified and/or internucleotide 3'-thio modified, e.g., the chemical modification is 2'-O-methyl modification of the first one, two and/or three bases at the 5' end and/or the last base at the 3' end of the sgRNA. In some embodiments, the invention also relates to a hematopoietic stem cell, wherein one or more sites in the target sequence of the BCL11A genome from positions 60495257-60495276, particularly position 60495273 in the chromosome 2 in the hematopoietic stem cell are disrupted by gene editing technology. Further, the invention relates to erythrocytes obtained from *in vitro* differentiation culture of the hematopoietic stem cells, and medical preparations containing the erythrocytes.

In some embodiments, the present invention provides a method for increasing the fetal hemoglobin (HbF) expression in human hematopoietic stem cells, comprising: disrupting a BCL11A genomic region from positions 60495264-60495283, particularly position 60495280 in the chromosome 2 in the hematopoietic stem cells by gene editing technology. The gene editing technology is a zinc finger nuclease-based gene editing technology, a TALEN gene editing technology or a CRISPR/Cas gene editing technology, preferably a CRISPR/Cas9 gene editing technology, and preferably the target nucleotide sequence in the BCL11A genome is complementary to a guide sequence of the sgRNA comprising SEQ ID NO: 5. The method of the present invention involves introducing an sgRNA comprising the sequence of SEQ ID NO: 5 into hematopoietic stem cells to effectively edit said BCL11A genome, preferably co-introducing the sgRNA with Cas9-encoding nucleotides (e.g., mRNA) into the hematopoietic stem cells, preferably co-introducing the sgRNA with Cas9-encoding nucleotides into the hematopoietic stem cells by electroporation under the electroporation conditions of 200-600 V, 0.5-2 ms. In some embodiments, the sgRNA is 2'-O-methyl modified and/or internucleotide 3'-thio modified, e.g., the chemical modification is 2'-O-methyl modification of the first one, two and/or three bases at the 5' end and/or the last base at the 3' end of the sgRNA. In some embodiments, the invention also relates to a hematopoietic stem cell, wherein one or more sites in the target sequence of the BCL11A genome from positions 60495264-60495283, particularly position 60495280 in the chromosome 2 in the hematopoietic stem cell are disrupted by gene editing technology. Further, the invention relates to erythrocytes obtained from *in vitro* differentiation culture of the hematopoietic stem cells, and medical preparations containing the erythrocytes.

In some embodiments, the present invention provides a method for increasing the fetal hemoglobin (HbF) expression in human hematopoietic stem cells, comprising: disrupting a BCL11A genomic region from positions 60495299-60495318, particularly position 60495301 in the chromosome 2 in the hematopoietic stem cells by gene editing technology. The gene editing technology is a zinc finger nuclease-based gene editing technology, a TALEN gene editing technology or a CRISPR/Cas gene editing technology, preferably a CRISPR/Cas9 gene editing technology, preferably the target nucleotide sequence in the BCL11A genome is complementary to a guide sequence of the sgRNA comprising SEQ ID NO: 24. The method of the present invention involves introducing an sgRNA comprising the sequence of SEQ ID NO: 24 into hematopoietic stem cells to effectively edit said BCL11A genome, preferably co-introducing the sgRNA with Cas9-encoding nucleotides (e.g., mRNA) into the hematopoietic stem cells, preferably co-introducing the sgRNA with Cas9-encoding nucleotides into the hematopoietic stem cells by electroporation under the electroporation conditions of 200-600 V, 0.5-2 ms. In some embodiments, the sgRNA is 2'-O-methyl modified and/or internucleotide 3'-thio modified, e.g., the chemical modification is 2'-O-methyl modification of the first one, two and/or three bases at the 5' end and/or the last base at the 3' end of the sgRNA. In some embodiments, the invention also relates to a hematopoietic stem cell, wherein one or more sites in the target sequence of the BCL11A genome from positions 60495299-60495318, particularly position 60495301 in the chromosome 2 in the hematopoietic stem cell are disrupted by gene editing technology. Further, the invention relates to erythrocytes obtained from *in vitro* differentiation culture of the hematopoietic stem cells, and medical preparations containing the erythrocytes.

In some embodiments, the present invention provides a method for increasing the fetal hemoglobin (HbF) expression in human hematopoietic stem cells, comprising: disrupting a BCL11A genomic region from positions 60495319-60495338, particularly position 60495335 in the chromosome 2 in the hematopoietic stem cells by gene editing technology. The gene editing technology is a zinc finger nuclease-based gene editing technology, a TALEN gene editing technology or a CRISPR/Cas gene editing technology, preferably a CRISPR/Cas9 gene editing technology, preferably the target nucleotide sequence in the BCL11A genome is complementary to a guide sequence of the sgRNA comprising SEQ ID NO: 25. The method of the present invention involves introducing an sgRNA comprising the sequence of SEQ ID NO: 25 into hematopoietic stem cells to effectively edit said BCL11A genome, preferably co-introducing the sgRNA with Cas9-encoding nucleotides (e.g., mRNA) into the hematopoietic stem cells, preferably co-introducing the sgRNA with Cas9-encoding nucleotides into the hematopoietic stem cells by electroporation under the electroporation conditions of 200-600 V, 0.5-2 ms. In some embodiments, the sgRNA is 2'-O-methyl modified and/or internucleotide 3'-thio modified, e.g., the chemical modification is 2'-O-methyl modification of the first one, two and/or three bases at the 5' end and/or the last base at the 3' end of the sgRNA. In some embodiments, the invention also relates to a hematopoietic stem cell, wherein one or more sites in the target sequence of the BCL11A genome from positions 60495319-60495338, particularly position 60495335 in the chromosome 2 in the hematopoietic stem cell are disrupted by gene editing technology. Further, the invention relates to erythrocytes obtained from *in vitro* differentiation culture of the hematopoietic stem cells, and medical preparations containing the erythrocytes.

In some embodiments, the present invention provides a method for increasing the fetal hemoglobin (HbF) expression in human hematopoietic stem cells, comprising: disrupting a BCL11A genomic region from positions 60495320-60495339, particularly position 60495336 in the chromosome 2 in the hematopoietic stem cells by gene editing technology. The gene editing technology is a zinc finger nuclease-based gene editing technology, a TALEN gene editing technology or a CRISPR/Cas gene editing technology, preferably a CRISPR/Cas9 gene editing technology, preferably the target nucleotide sequence in the BCL11A genome is complementary to a guide sequence of the sgRNA comprising SEQ ID NO: 23. The method of the present invention involves introducing an sgRNA comprising the sequence of SEQ ID NO: 23 into hematopoietic stem cells to effectively edit said BCL11A genome, preferably co-introducing the sgRNA with Cas9-encoding nucleotides (e.g., mRNA) into the hematopoietic stem cells, preferably co-introducing the sgRNA with Cas9-encoding nucleotides into the hematopoietic stem cells by electroporation under the electroporation conditions of 200-600 V, 0.5-2 ms. In some embodiments, the sgRNA is 2'-O-methyl modified and/or internucleotide 3'-thio modified, e.g., the chemical modification is 2'-O-methyl modification of the first one, two and/or three bases at the 5' end and/or the last base at the 3' end of the sgRNA. In some embodiments, the invention also relates to a hematopoietic stem cell , wherein one or more sites in the target sequence of the BCL11A genome from positions 60495320-60495339, particularly position 60495336 in the chromosome 2 in the hematopoietic stem cell are disrupted by gene editing technology. Further, the invention relates to erythrocytes obtained from *in vitro* differentiation culture of the hematopoietic stem cells, and medical preparations containing the erythrocytes.

Further, in the present invention, Indels are efficiently produced, i.e., gene editing is efficiently performed, by using any of the 23 sgRNAs designed by the inventors. The preferable sgRNA of the present invention comprises a sequence selected from any one of SEQ ID NOs: 3-25, and the sequence of the cleavage site in the target sequence which corresponds to the sgRNA is located in the region of chr2: 60495219-chr2: 60495336 (i.e., positions 60495219-60495336 in the chromosome 2).

In particular, the following table lists the positions of the genomic sequences on human chromosome 2 which are targeted by the 23 sgRNAs of the present invention, as well as the Cas9 cleavage sites triggered by each sgRNA,.

| **Names** | **Positions on the human genomic sequence** | **Cleavage sites** |
|---|---|---|
| BCL11A enhancer-7 | chr2:60495203-60495222 | chr2:60495219 |
| BCL11A enhancer-8 | chr2:60495208-60495227 | chr2:60495224 |
| BCL11A enhancer-9 | chr2:60495217-60495236 | chr2:60495233 |
| BCL11A enhancer-10 | chr2:60495218-60495237 | chr2:60495234 |
| BCL11A enhancer-11 | chr2:60495219-60495238 | chr2:60495235 |
| BCL11A enhancer-14 | chr2:60495221-60495240 | chr2:60495223 |
| BCL11A enhancer-12 | chr2:60495222-60495241 | chr2:60495238 |
| BCL11A enhancer-13 | chr2:60495223-60495242 | chr2:60495239 |
| BCL11A enhancer-15 | chr2:60495228-60495247 | chr2:60495244 |
| BCL11A enhancer-16 | chr2:60495229-60495248 | chr2:60495245 |
| BCL11A enhancer-17 | chr2:60495230-60495249 | chr2:60495246 |
| BCL11A enhancer-18 | chr2:60495231-60495250 | chr2:60495247 |
| BCL11A enhancer-19 | chr2:60495234-60495253 | chr2:60495250 |
| BCL11A enhancer-20 | chr2:60495235-60495254 | chr2:60495251 |
| BCL11A enhancer-2 | chr2:60495236-60495255 | chr2:60495238 |
| BCL11A enhancer-1 | chr2:60495247-60495266 | chr2:60495263 |
| BCL11A enhancer-6 | chr2:60495252-60495271 | chr2:60495268 |
| BCL11A enhancer-5 | chr2:60495253-60495272 | chr2:60495269 |
| BCL11A enhancer-4 | chr2:60495257-60495276 | chr2:60495273 |
| BCL11A enhancer-3 | chr2:60495264-60495283 | chr2:60495280 |
| BCL11A enhancer-22 | chr2:60495299-60495318 | chr2:60495301 |
| BCL11A enhancer-23 | chr2:60495319-60495338 | chr2:60495335 |
| BCL11A enhancer-21 | chr2:60495320-60495339 | chr2:60495336 |

Analysis of cleavage sites of the 23 sgRNAs reveals that, the Cas9 cleavage sites triggered by these sgRNAs are concentrated in the genomic region from positions 60495219 to 60495336 in the BCL11A gene.

A particular embodiment of the present invention further includes a composition containing said sgRNA, comprising any of the above 23 sgRNAs according to the present invention or a vector thereof.

In general, the guide sequence in an sgRNA is any polynucleotide sequence sufficiently complementary to a target polynucleotide sequence and capable of hybridizing to the target sequence, thereby directing the sequence-specific binding of CRISPR complex to the target sequence. In some embodiments, when optimal alignment is performed by using a suitable alignment algorithm, the degree of the complementarity between the guide sequence and its corresponding target sequence is about or higher than about 80%, 85%, 90%, 95%, 97.5%, 99% or more. Optimal alignment may be determined by using any suitable algorithm for aligning sequences, non-limiting examples of the algorithms include: the Smith-Waterman algorithm, the Needleman-Wimsch algorithm, the Burrows-Wheeler Transform based algorithms (e.g., Burrows Wheeler Aligner), ClustalW, Clustai X, BLAT, Novoalign (Novocaft Technologies, ELAND (Illumina, San Diego, CA), SOAP (available at soap. genomics. org. cn), and Maq (available at maq. sourceforge. net). In some embodiments, the length of the guide sequence may be about or greater than about 10, 11, 12, 13, 14, 15, 16, 17, 18, 19, 20, 21, 22, 23, 24, 25, 26, 27, 28, 29, 30, 35, 40, 45, 50, 55, 60, 65, 70, 75 or more nucleotides. In some embodiments, the length of the guide sequence is less than about 75, 70, 65, 60, 55, 50, 45, 40, 35, 30, 25, 20, 15, 12 or fewer nucleotides. The ability of the guide sequence to direct sequence-specific binding of the CRISPR complex to the target sequence may be assessed by any suitable assays. For example, a host cell having a corresponding target sequence may be provided with the components of the CRISPR system capable of forming a CRISPR complex (including the guide sequences to be tested), for instance, it may be performed by transfection with a vector encoding the components of the CRISPR sequences, followed by evaluation of preferential cleavages within the target sequence (e.g., determined by Surveyor as described herein). Likewise, the cleavage of a target polynucleotide sequence can also be assessed in a test tube by providing a target sequence and the components of a CRISPR complex (comprising a guide sequence to be tested, and a control guide sequence different from the guide sequence), and then comparing the binding or cleaving rate of the tested guide sequences and the control guide sequence on the target sequence. Other assays known to those skilled in the art can also be used for the above determination and assessment.

In the present invention, the sgRNAs used in performing gene editing are preferably chemically modified. "Chemically modified sgRNA" refers to an sgRNA modified with a specific chemical group, for example, 2'-O-methyl modification and/or an internucleotide 3'-thio modification at the 5' end and 3' end three bases.

The chemically modified sgRNA employed by the present inventors is believed to have the following two advantages. Firstly, as sgRNA is an single-stranded RNA, its half-life is very short, and it degrades rapidly after entering the cell (not more than 12 hours), while the binding of Cas9 protein to sgRNA to perform gene editing requires at least 48 hrsgene editing. Therefore, a chemically modified sgRNA is used for stable expression after entering a cell, and after binding to the Cas9 protein it can generate Indels by efficiently edit the genome. Secondly, an unmodified sgRNA has poor ability to penetrate cell membranes and cannot effectively enter cells or tissues to perform corresponding functions, while the ability of a chemically modified sgRNA to penetrate cell membranes is generally enhanced. Chemical modification methods commonly used in the art may used in the present invention, so long as the stability of the sgRNA may be enhanced (the half-life is prolonged), and the ability to enter the cell membrane may be improved. In addition to the specific chemical modifications used in the examples, other modification methods are also included, such as those chemical modification methods reported in the following literatures: Deleevey GF1, Damha MJ.; Designing chemically modified oligonucleotides for targeted gene silencing. Chem Biol. 2012 Aug 24; 19(8): 937-54, and Hendel et al.; Chemically modified guide RNAs enhance CRISPR-Cas gene editing in human primary cells. NatBiotechnol. 2015Sep; 33(9): 985-989.

In some embodiments, the sgRNA and/or Cas9-encoding nucleotides (e.g., mRNA) are introduced into hematopoietic stem cells by electroporation under the electroporation conditions such as 250-360 V, 0.5-1 ms; 250-300V, 0.5-1ms; 250V 1ms; 250V, 2ms; 300V, 0.5ms; 300V, 1ms; 360V, 0.5ms; or 360 V, 1ms. In some embodiments, the sgRNA and the Cas9-encoding nucleotides are co-introduced into the hematopoietic stem cells by electroporation. In some embodiments, the sgRNA is introduced into hematopoietic stem cells expressing Cas9 by electroporation.

In some embodiments, the Cas9-encoding nucleotide is an mRNA, such as an mRNA containing an ARCA cap. In some embodiments, the Cas9-encoding nucleotides are included in a viral vector, such as a lentiviral vector. In some embodiments, the Cas9-encoding nucleotides comprise the sequence of SEQ ID NO: 26. In some embodiments, the sgRNA and the Cas9-encoding nucleotides are included in the same vector.

The invention relates to hematopoietic stem cells obtained by the genetic modification method according to the present invention, the precursor cells at different differentiation stages before mature erythrocytes and being obtained by differentiation culture of the genetically modified hematopoietic stem cells, and the mature erythrocytes obtained by differentiation culture of the genetically modified hematopoietic stem cells.

The present invention relates to a pharmaceutical composition obtained by the method according to the present invention, the composition comprises the hematopoietic stem cells obtained by the genetic modification method of the present invention, or the precursor cells at different differentiation stages before mature erythrocytes and being obtained by differentiation culture of the genetically modified hematopoietic stem cells, or the mature erythrocytes obtained by differentiation culture of the genetically modified hematopoietic stem cells.

The pharmaceutical composition according to the present invention may be administered to a subject in need thereof by a route conventionally used for administering a medical preparation containing cellular components, e.g., by an intravenous infusion route. The dosage of administration may be specifically determined depending on the condition of the subject and the general health conditions.

In some aspects, the invention provides methods of delivering an sgRNA of the invention and/or Cas9-encoding nucleotides to hematopoietic stem cells. In some embodiments, in this the delivery the construct may be introduced into hematopoietic stem cells or other host cells by using conventional virus based and non-virus based gene transferring methods. Non-viral delivery systems include DNA plasmids, RNA (e.g., the transcripts of the vector described herein), naked nucleic acids, and liposomes. Viral vector delivery systems include DNA and RNA viruses having a free or integrated genome for the delivery to a cell. In some embodiments, the inventors introduce genes encoding Cas9 and the sgRNA into the hematopoietic stem cells by electroporation to perform gene editing of a specific sequence of the BCL11A gene in the hematopoietic stem cell. After repeated experiments, the inventors found that the gene editing efficiency for co-introducing Cas9-encoding nucleotides and the sgRNA into the hematopoietic stem cells by electroporation under the electroporation conditions of 200-600v, 0.5ms-2ms is significantly higher than that under other electroporation conditions.

In some embodiments, the chemically modified sgRNA and the Cas9-encoding gene are co-introduced into the CD34+ hematopoietic stem cells by electroporation, achieving a high efficiency of gene editing (indicated as Indels%). The data in the examples shows that, if the Cas9 mRNA is co-introduced together with a chemically unmodified sgRNA by electroporation, the Indels efficiency is only 2.7%, which is much lower than that obtained by introducing a chemically modified sgRNA by electroporation (the efficiency is at least 10% or more).

As used herein, the full name of "Indel" is insertion/deletion, which is referred to an insertion and deletion mutation.

As used herein, "hematopoietic stem cells (hematopoietic stem and progenitor cells, HSPCs)" are the most primitive hematopoietic cells for generating various blood cells. Their main characteristics are strong proliferation potential, multi-directional differentiation ability and self-renewal ability. Thus, they not only differentiates and supplements various blood cells, but also maintains the characteristics and quantity of stem cells through self-renewal. Hematopoietic stem cells have different differentiation degrees proliferation abilities, and are heterogeneous. Pluripotent hematopoietic stem cells are the most primitive, they firstly differentiate into committed pluripotent hematopoietic stem cells, such as myeloid hematopoietic stem cells capable of producing granulocytic, erythroid, mononuclear cells and megakaryo-platelet lineages, and lymphoid stem cells capable of producing B and T lymphocytes. These two types of stem cells maintain the basic characteristics of hematopoietic stem cells, but they are slightly differentiated and respectively responsible for the generation of "bone marrow components" and lymphocytes, hence they are called as "committed pluripotent hematopoietic stem cells". They further differentiate into hematopoietic progenitor cells, which are also primitive blood cells, but have lost many of the basic characteristics of hematopoietic stem cells, for example, losing the ability of multi-directional differentiation, and they can only differentiate into one cell line or two closely related cell lines; the repeated self-renewal capacity is lost, and their quantity is supplemented by the proliferation and differentiation of hematopoietic stem cells; and the proliferation potential is limited and they only divide several times. Depending on the number of blood cell lines that may be differentiated from the hematopoietic progenitor cells, they may be divided into unipotent hematopoietic progenitor cells (differentiating into only one blood cell line) and oligopotent hematopoietic progenitor cells (differentiating into 2-3 blood cell lines). The term "hematopoietic stem cell" in the present invention encompasses pluripotent hematopoietic stem cells, committed pluripotent hematopoietic stem cells, and hematopoietic progenitor cells, and it is a generic term for all hematopoietic stem cells with different heterogeneities.

In a particular embodiment of the present invention, the hematopoietic stem cells (HSPCs) uses in the present invention for gene editing may be derived from bone marrow, umbilical cord blood, or peripheral blood mononuclear cells (PBMCs).

As used herein, "CRISPR RGEN" is the name of a website developed by the research team of Korean scientist Jin-Soo Kim specifically for designing sgRNA at www.rgenome.net/about/.

As used herein, "TIDE" refers to the name of a tool website for analyzing Indels efficiency at tide-calculator. nki. nl.

As used herein, "CD34, CD45RA, CD3, CD4, CD8, CD33, CD19, CD56, CD71, and CD235a" are membrane protein markers of hematologic cells.

As used herein, "BCL11A" is a transcription factors first found in mice as a retroviral binding site, it was named Evi9 and was later found in a human genome locating at locus 2p13 of the short arm chromosome 2, and it is expressed primarily in the germinal center of B lymphocytes.

As used herein, "HBB/HBG" are different subtypes of hemoglobin. Hemoglobin is a protein responsible for oxygen transport in higher organisms. Hemoglobin consists of four chains, two α chains and two β chains, each having a cyclic heme which contains an iron atom. Oxygen is bound to the iron atom and transported by erythrocytes for using by the organism.

Further, the present invention relates to hematopoietic stem cells obtained by genetically editing a specific sequence of BCL11A in the hematopoietic stem cells by CRISPR/Cas9 gene editing technology through the method of the present invention as described above. In addition, preparations comprising the hematopoietic stem cells are also comprised in the scope of the present invention.

The hematopoietic stem cells or the preparations thereof according to the present invention may be used to treat a disease selected from an anemia disease, a hemorrhagic disease, a tumor, or other disease requiring massive blood transfusion for treatment. In particular it may be used for the treatment of β thalassemia or sickle cell anemia.

Further, the present invention relates to an erythrocyte obtained by in vitro differentiation culture (as described below) of the genetically modified hematopoietic stem cells according to the present invention.

Further, the present invention relates to precursor cells at different stages of differentiation from hematopoietic stem cells to mature erythrocytes, which are obtained through treating the genetically modified hematopoietic stem cells according to the present invention by the following erythroid expansion and differentiation steps for the hematopoietic stem cells:
wherein the above *in vitro* differentiation culture comprises: a hematopoietic stem cell erythroid expansion and differentiation step; and a hematopoietic stem cell erythroid differentiation and enucleation step; and
in the erythroid expansion and differentiation step, the hematopoietic stem cells are cultured by using a HSPCs erythroid expansion and differentiation medium; and
in the erythroid differentiation and enucleation step, an erythroid differentiation and enucleation medium is used.

In some embodiments, the HSPCs erythroid expansion and differentiation medium comprises: a basal culture medium, and a composition of growth factors, wherein the composition of growth factors comprises a stem cell growth factor (SCF); interleukin 3 (IL-3); and erythropoietin (EPO).

In some embodiments, the erythroid differentiation and enucleation medium comprises basal culture medium, growth factors, and antagonists and/or inhibitors of a progesterone receptor and a glucocorticoid receptor.

When the genetically modified hematopoietic stem cells are cultured by the above method, the hematopoietic stem cells may be differentiated into mature erythrocytes through two steps, compared with the prior art, only 14 days are needed for the *in vitro* differentiation culture according to the present invention, and the time period is shorter and greatly shortened as compared with a time period of more than 21 days needed in the prior art.

In some embodiments, the growth factors comprise erythropoietin (EPO), and the antagonists and/or inhibitors of a progesterone receptor and a glucocorticoid receptor are selected from any one or two or more of the following compounds (I)-(IV):

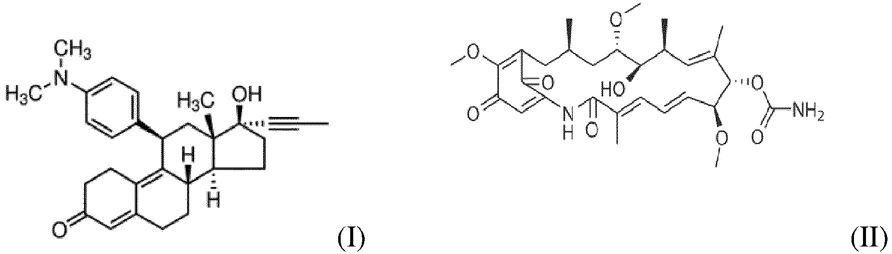

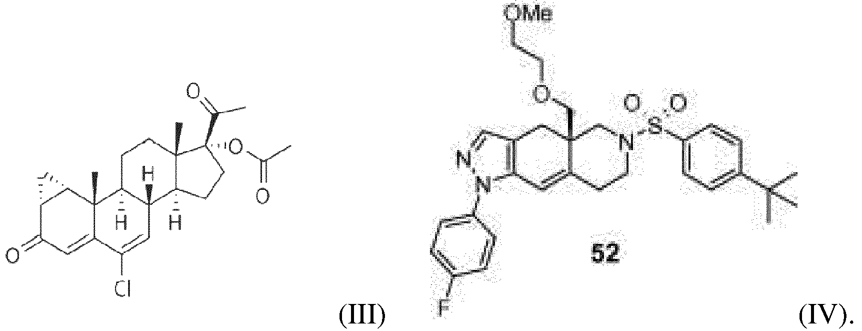

In some embodiments, the HSPCs erythroid expansion and differentiation medium comprises a basal culture medium such as STEMSSPAN™ SFEM II (STEM CELLS TECHNOLOGY Inc.), IMDM (Iscove's Modified Dulbecco's Medium), X-VIVO 15, alpha-MEM, RPMI 1640, DF12, and the like. As for the growth factors, for example if STEMSPAN™ SFEM II is used as basal culture medium, additional growth factors including 50-200 ng/ml SCF, 10-100 ng/ml IL-3, 1-10 U/ml EPO are needed to add into the medium, wherein U is defined as: the amount of protein which convert 1 µmol of substrate in 1 min under specific conditions, i.e. 1 IU = 1 µmol/min, and now, the letter "I" of "IU (international unit)" is often omitted in most clinical laboratories at home and abroad, and it is abbreviated as U. Erythropoietin (EPO) is a glycoprotein secreted primarily by kidney in response to hypoxia or anemia, in this embodiment, it promotes the differentiation of hematopoietic stem cells, and typically the duration of its effect is around 7 days.

When a basal culture medium other than STEMSPAN™ SFEM II is adopted as the basal medium, the following ingredients are needed: 100X ITS (in-transferrin-selenium) (wherein the final concentrations of each substance in the ITS of the medium are as follows: the concentration of insulin is 0.1 mg/ml; human transferrin, 0.0055 mg/ml; selenium, 6.7 × 10⁻⁶ mg/ml)(i.e., consisting essentially of insulin, human transferrin, and selenium); 10-50 µg/ml vitamin C; 0.5-5% BSA (Bovine serum albumin); growth factors, such as 50-200 ng/ml SCF, 10-100 ng/ml IL-3, and 1-10 U/ml EPO.

In addition, those skilled in the art will appreciate that any one of commonly used basal culture medium may be used. For example, the basal media commonly used in the art listed as follows: STEMSPAN™ SFEM II (available from STEM CELL TECHONOLOGIES); and IMDM; DF12; Knockout DMEM; RPMI 1640, Alpha MEM, DMEM, etc. available from Thermo Fisher.

In addition, some other ingredients also may be added to the above medium as desired, such as ITS (i.e., consisting essentially of insulin, human transferrin, and elemental selenium), L-glutamine, vitamin C, and bovine serum albumin. For example, the IMDM medium may be supplemented with ITS, 2 mM L-glutamine, 10-50 µg/ml vitamin C, and 0.5-5 wt% BSA (bovine serum albumin). In addition, the DF12 described above may be supplemented with the same concentrations of ITS, L-glutamine, vitamin C, and bovine serum albumin; Knockout DMEM may be supplemented with the same concentrations of ITS, L-glutamine, vitamin C and bovine serum albumin; RPMI 1640 may be supplemented with the same concentrations of ITS, L-glutamine, vitamin C and bovine serum albumin; Alpha MEM may be supplemented with the same concentrations of ITS, L-glutamine, vitamin C and bovine serum albumin; DMEM may be supplemented with the same concentrations of ITS, L-glutamine, vitamin C and bovine serum albumin. Herein, the concentrations of each substance in the added ITS in the various basal media are: insulin concentration is 0.1 mg/ml; human transferrin, 0.0055 mg/ml; and selenium, 6.7 x 10⁻⁶ mg/ml. In addition, the concentrations of each component in the added ITS may also be adjusted according to practical requirements. ITS may be purchased from Thermofisher, and adjusted to the appropriate final concentration for use as needed.

In an embodiment, the erythroid differentiation and enucleation medium comprises a basal culture medium, growth factors and antagonists of a progesterone receptor and a glucocorticoid receptor.

In an embodiment, the hematopoietic stem cell erythroid differentiation and enucleation medium comprises a basal culture medium such as STEMSSPAN™ SFEM II (STEM CELLS TECHNOLOGY Inc.), IMDM (Iscove's Modified Dulbecco's Medium), X-VIVO 15, alpha-MEM, RPMI 1640, DF12, and the like. Growth factors are also comprised, e.g. if STEMSPAN SFEM II is used as the basal culture medium, additional growth factors needed comprise: 1-10 U/ml EPO, 100-1000 µ g/ml human transferrin, and chemical small molecules of 0.5-10 µmol/ml mifepristone.

If a basal culture medium other than STEMSPAN™ SFEM II basal culture medium is used, it is necessary to add, for example, ITS (insulin-transferrin-selenium) (wherein the final concentrations of each substance in the ITS in the medium are: insulin concentration is 0.1 mg/ml; human transferrin, 0.0055 mg/ml; selenium, 6.7×10⁻⁶ mg/ml (i.e., consisting essentially of insulin, human transferrin, and selenium); vitamin C, 10-50 ug/ml; BSA (Bovine serum albumin), 0.5-5%; growth factors such as EPO, 1-10 U/ml; human transferrin, 100-1000 ug/ml; small chemical molecules such as mifepristone, 0.5-10 µmol/ml.

In addition, those skilled in the art will appreciate that any one of commonly used basal media may be used. For example, the basal media commonly used in the art listed as follows: STEMSPAN™ SFEM II (available from STEM CELL TECHONOLOGIES); IMDM; DF12; Knockout DMEM; RPMI 1640, Alpha MEM, DMEM, etc. available from Thermo Fisher.

In addition, some other ingredients also may be added to the above medium as desired, such as ITS (i.e., consisting essentially of insulin, human transferrin, and elemental selenium), L-glutamine, vitamin C, and bovine serum albumin. For example, the IMDM medium may be supplemented with ITS, 2 mM L-glutamine, 10-50 µg/ml vitamin C, and 0.5-5wt% BSA (bovine serum albumin). In addition, the DF12 described above may be supplemented with the same concentrations of ITS, L-glutamine, vitamin C, and bovine serum albumin; Knockout DMEM may be supplemented with the same concentrations of ITS, L-glutamine, vitamin C and bovine serum albumin; RPMI 1640 may be supplemented with the same concentrations of ITS, L-glutamine, vitamin C and bovine serum albumin; Alpha MEM may be supplemented with the same concentrations of ITS, L-glutamine, vitamin C and bovine serum albumin; DMEM may be supplemented with the same concentrations of ITS, L-glutamine, vitamin C and bovine serum albumin. Herein, the concentrations of each substance in the the added ITS in the various basal mediaare: insulin concentration is 0.1 mg/ml; human transferrin, 0.0055 mg/ml, and selenium, 6.7×10⁻⁶ mg/ml. In addition, the concentrations of each component in the added ITS may also be adjusted according to practical requirements. ITS may be purchased from Thermofisher and adjusted to the appropriate final concentration for use as needed.

Mifepristone used herein is a chemically synthesized small molecule as an antagonist of the progesterone receptor and the glucocorticoid receptor. The structural formula of the molecule is as follows:

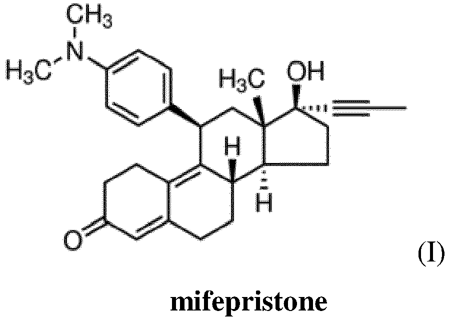

Other antagonists and inhibitors of a progesterone receptor and a glucocorticoid receptor may also be used in the present invention, including cyproterone acetate, geldanamycin, CORT 108297, and the like. The chemical structures are as follows:

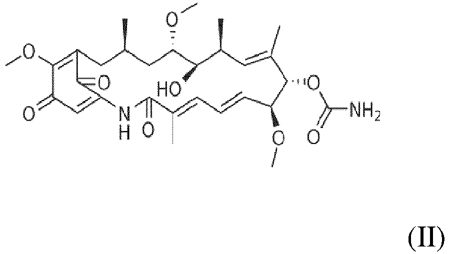

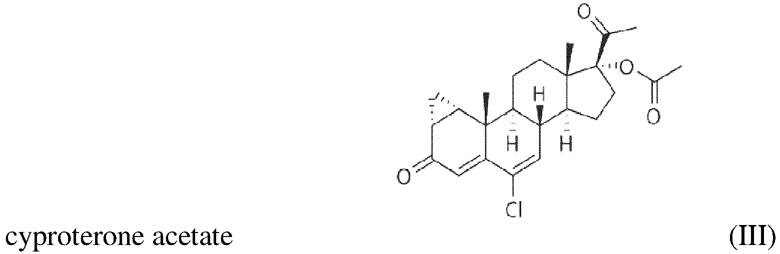

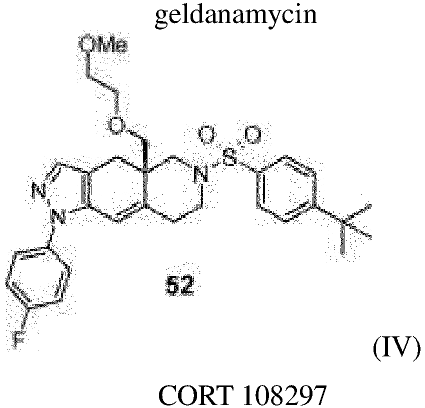

In some embodiments, the mature erythrocytes of the present invention may be produced by a method comprising the steps from a) to c): a) isolating CD34-positive HSPCs from human umbilical cord blood to perform gene modification by any one of the methods disclosed by the invention; b) amplifying and differentiating the genetically modified HSPCs for 5-10 days, e.g., 5 days, 6 days, 7 days, 8 days, 9 days, 10 days, to obtain erythrocyte precursor cells, e.g., erythroblasts, nucleated erythrocytes, young erythroblasts, and reticulocytes; c) further differentiating the erythrocyte precursor cells for 7 days to obtain mature erythrocytes.

In some embodiments, mature erythrocytes of the present invention may be produced by a method comprising the steps from a) to d):
a) isolating CD34-positive HSPCs from human umbilical cord blood by magnetic bead sorting;
b) genetically modifying the CD34-positive HSPCs by any one of the methods of the invention;
c) adding additional growth factors into the serum-free medium (SFME), and after 5-10 days, e.g., 5 days, 6 days, 7 days, 8 days, 9 days, 10 days of expansion and differentiation, the HSPCs are differentiated into erythrocyte precursor cells, and the medium at this stage is named as HSPCs erythroid expansion and differentiation medium (abbreviated as HEEDM);
d) adding additional growth factors into a serum-free medium (SFME), and after 7 days of differentiation, the mature erythrocytes may be obtained, the medium at this stage is named as HSPCs erythroid differentiation and enucleation medium (abbreviated as HEDEM).

That is, for genetically edited CD34-positive hematopoietic stem cells, the mature erythrocytes may be obtained in two steps by the method of the present invention described above, and the time period of the whole process may be 10-18 days, 11-17 days, 12-16 days, 13-15 days, 10-17 days, 10-16 days, 10-15 days, or 10-14 days, such a time period is greatly shorter than the period of at least 21 days in prior art by using three or four steps.

As described above, the present invention relates to a method for preparing mature erythrocytes or precursor cells thereof being genetically modified to increase the expression of fetal hemoglobin (HbF), the method comprises: (a) obtaining the hematopoietic stem cells by the genetic modification method disclosed by the invention; (b) performing hematopoietic stem cell erythroid expansion and differentiation of the genetically modified hematopoietic stem cells by using the above HSPCs erythroid expansion and differentiation medium.

Further, the present invention relates to a method for preparing mature erythrocytes or precursor cells thereof being genetically modified to increase the fetal hemoglobin (HbF) expression, the method comprises: (a) obtaining the hematopoietic stem cells by the genetic modification method disclosed by the invention; (b) performing hematopoietic stem cell erythroid expansion and differentiation of the genetically modified hematopoietic stem cells by using the above HSPCs erythroid expansion and differentiation medium; and (c) performing HSPCs erythroid differentiation and enucleation by using the erythroid differentiation and enucleation medium. The invention also relates to use of the HSPCs erythroid expansion and differentiation medium and/or the erythroid differentiation and enucleation medium used in the above method for expanding and/or differentiating hematopoietic stem cells or precursor cells of mature erythrocytes.

As used herein, "differentiation" refers to a phenomenon in which the structure or function of a cell is specialized during the process of division, proliferation, and growth thereof, i.e., the characteristics and function of cells or tissues of an organism are altered to perform a function imparted to the cells or tissues. In general, it refers to the phenomenon in which a relatively simple system is divided into two or more partial systems having different properties.

As used herein, "Ficoll liquid density gradient centrifugation" is based on the principle that the specific gravity of different components in the blood are different, and different cells may be separated in layers through low speed density gradient centrifugation. The density of erythrocytes and granulocytes is greater than the stratified liquid, and when erythrocytes encounter ficoll liquid they will rapidly aggregate into a string arrangement and accumulate at the bottom of the tube. Only the mononuclear cells with a density equivalent to that of the stratified liquid are enriched between the plasma layer and the stratified liquid, i.e., the white membrane layer; and the hematopoietic stem cells exist in this layer and they may be obtained through subsequent magnetic bead sorting. In the present invention, mononuclear cells are obtained by using commercially available lymphocyte separation tubes.

As used herein, "mature erythrocytes" refers to the most abundant type of cells in the blood, and they have the function of carrying nutrients such as oxygen, amino acids, carbon dioxide, and the like. Mature erythrocytes have no mitochondria and nuclei.

As used herein, "interleukin 3 (IL-3)" refers to a cytokine produced by activated CD4- and CD8-positive T lymphocytes, and its major biological function of which is involved in regulating the proliferation and differentiation of the hematopoietic stem cells in bone marrow.

As used herein, "erythropoietin (EPO)" refers to a growth factor secreted by erythroblasts, i.e., erythrocyte precursors. In adults, there are glycoproteins secreted by the interstitial cells surrounding the renal tubules in renal cortex and the liver. EPO stimulates the hematopoietic stem cells to differentiate into erythrocytes.

In some embodiments, the stem cell factors (SCFs) include mast cell growth factor (MGF), kit ligand (KL), and steel factor (SLF).

As used herein, "HSPCs erythroid expansion and differentiation medium" refers to a culturing system for facilitating the massive expansion of HSPCs and their differentiation into erythroid progenitor cells. In the present invention, the medium comprises two main components, namely a basal culture medium and a growth factor additive. The basal culture medium is a serum-free system, either STEMSPAN™ SFEM II (STEM CELLS TECHNOLOGY Inc.), or IMDM (Iscove's Modified Dulbecco's Medium), plus ITS (Thermofisher), L-glutamine (Thermofisher), vitamin C, and bovine serum albumin. The growth factor additive is a combination of different concentrations of IL-3, SCF and EPO.

As used herein, a "HSPCs erythroid differentiation and enucleation medium" refers to a medium that aids in further expansion and differentiation of erythroid progenitor cells into enucleated erythrocytes. In the present invention, the culture medium comprises two main components, namely a basal medium, and additives of growth factors and chemical small molecules. The basal culture medium is a serum-free system, either STEMSPAN™ SFEM II (STEM CELLS TECHNOLOGY Inc.), or IMDM (Iscove's Modified Dulbecco's Medium), plus ITS (Thermofisher), L-glutamine (Thermofisher) vitamin C, and bovine serum albumin. Additives of growth factors and chemical small molecules include EPO, human transferrin, and the chemical small molecule of mifepristone.

In some embodiments, hematopoietic stem cells are sorted by magnetic beads. For example, the cells are specifically labeled with superparamagnetic MACS microbeads, and after magnetic labeling, making the cells to pass through a sorting column placed in a strong and stable magnetic field. The matrix in the sorting column creates a high gradient magnetic field.

The magnetically labeled cells are retained in the column while the unlabeled cells are discharged. After the sorting column is removed from the magnetic field, magnetically labeled cells retained in the column may be eluted. Thus, both labeled and unlabeled cell components may be completely obtained.

In some specific embodiments of the present invention, through utilizing the sgRNA of the present invention, the hematopoietic stem cells derived from three different umbilical cord blood are genetically modified by the genetic modification methods described herein, and the efficient gene editing may be achieved with an efficiency of at least 40% or more, e.g., 50% or more, 60% or more, 70% or more, 80% or more, or 90% or more. For example, as for the preferred sgRNA, the average gene editing efficiency even reaches 80%.

In some embodiments of the present invention, the hematopoietic stem cells obtained by the gene editing method of the present invention are transplanted into a mouse. The proportion of human hCD45 expression in the mouse continuously increases after the transplantation of the genetically modified hematopoietic stem cells, as compared with that of the transplantation of the hematopoietic stem cells not being genetically modified. In peripheral blood samples, the proportion of hCD45 expression increases from 20% at week 6 to 60% at week 16; the proportion even reaches 90% in bone marrow and 70% in spleen at week 16; indicating that the genetically modified hematopoietic stem cells may be rapidly and efficiently implanted into the hematopoietic system of the mouse model, and the *in vivo* differentiation function of the cells is normal.

In some embodiments of the present invention, the umbilical cord blood-derived hematopoietic stem cells are modified by the gene editing method of the present invention, and then they are differentiated by using the "two-step" differentiation method of the present invention, i.e., the HSPCs erythroid expansion and differentiation medium is used for expansion and differentiation, and then HSPCs erythroid differentiation and enucleation medium is used for further differentiation. The detection results show that, the hemoglobin (HBG) expression in differentiated erythrocytes is increased by 20%-90%, even by 100% as compared with that of the original erythrocytes, i.e., increased by about 1 fold (2 folds of the expression of the original erythrocytes); the fetal hemoglobin expression is also increased by 20%-90%, even up to 100% as compared with that of the original erythrocytes, i.e. about 1 fold higher (2 times of the expression of the original erythrocytes); even up to 200%, i.e., about 2 folds higher (3 times of the original); even up to 300%, i.e., about 3 folds higher (4 times of the original); even up to 400%, i.e., about 4 folds higher (5 times of the original); even up to 500% or more, i.e., about 5 folds higher or more (6 or more times of the original), etc.

### Examples

Hereinafter, the present invention will be further described with reference to the following examples. It will be apparent to those skilled in the art that these examples are for illustrative purposes only and are not to be construed as limiting the scope of the invention. Accordingly, the essential scope of the invention is to be defined by the appended claims and their equivalents.

### Example 1: Efficient gene editing of umbilical cord blood-derived CD34-positive hematopoietic stem cells

### 1-1: Testing electroporation conditions by using K562 cells

Due to the limited source of hematopoietic stem cells and the high cost of each single isolation, the cancer cell line K562 (purchased from ATCC organization, website: https: //www.atcc.org) is selected as a model cell line for testing electroporation conditions in this example.

Particularly, the specific steps for implementation are described below.

The first experiments: transfecting 5×10⁵ K562 cells with 5µg of GFP mRNA (the sequence of SEQ ID NO: 1) by BTX830 electroporator under the conditions of 250 V, 1 ms; 360 V, 1 ms; 400 V, 1 ms; and 500 V, 1 ms respectively. 4 days after the electroporation, GFP expression and 7-AAD expression are determined by flow cytometry, wherein GFP represents electroporation efficiency, and 7-AAD represents the growth state, i.e. viability of the cells after electroporation.
SEQ ID NO:1: sequence information of GFP mRNA:

atgagtaaaggagaagaacttttcactggagttgtcccaattcttgttgaattagatggtgatgttaatgggcacaaattt
ctgtcagtggagagggtgaaggtgatgcaacatacggaaaacttacccttaaatttatttgcactactggaaaactacct
gttccatggccaacacttgtcactactttctcttatggtgttcaatgcttttcaagatacccagatcatatgaaacggcatga
ctttttcaagagtgccatgcccgaaggttatgtacaggaaagaactatatttttcaaagatgacgggaactacaagacac
gtgctgaagtcaagtttgaaggtgatacccttgttaatagaatcgagttaaaaggtattgattttaaagaagatggaaaca
ttcttggacacaaattggaatacaactataactcacacaatgtatacatcatggcagacaaacaaaagaatggaatcaa
agttaacttcaaaattagacacaacattgaagatggaagcgttcaactagcagaccattatcaacaaaatactccaattg
gcgatggccctgtccttttaccagacaaccattacctgtccacacaatctgccctttcgaaagatcccaacgaaaagag
agaccacatggtccttcttgagtttgtaacagctgctgggattacacatggcatggatgaactatacaaatag

The second experiments: transfecting 5×10⁵ K562 cells with 5µg of the above GFP mRNA by BTX830 electroporator under the conditions of 250 V, 1 ms; 250 V, 2 ms; 300 V, 0.5 ms; 300 V, 1 ms; 360 V, 0.5 ms; and 360 V; 1 ms respectively. 4 days after the electroporation, GFP expression and 7-AAD expression are determined by flow cytometry, wherein GFP represents electroporation efficiency, and 7-AAD represents the growth state, i.e. viability of the cells after electroporation.

The third experiments: transfecting 5×10⁵ K562 cells with 5µg of the above GFP mRNA by BTX830 electroporator under the conditions of 250 V, 1 ms; 250 V, 1 ms; 300 V, 1 ms; and 300 V, 1 ms respectively. 4 days after the electroporation, GFP expression and 7-AAD expression are determined by flow cytometry, wherein GFP represents electroporation efficiency, and 7-AAD represents the growth state, i.e. viability of the cells after electroporation. The results are shown in FIGs. 2 and 3.

Among them, FIG. 2 shows the fluorescence photomicrographs obtained 4 days after transfecting with GFP under the optimal electroporation conditions of the multi-batch experiments on cancer cell line K562. "V" refers to the pulse voltage, and "ms" refers to the pulse time.

FIG. 3 is a graph showing flow cytometry analysis of 7-AAD and statistical analysis of GFP expression obtained 4 days after transfecting with GFP under the optimal electroporation conditions of the multi-batch experiments on cancer cell line K562. 7-AAD negative in flow cytometry analysis indicates cell viability. 7-AAD (7-amino-actinomycin D) is a nucleic acid dye that does not pass through the normal plasma membrane, while the permeability of plasma membrane to 7-AAD increases gradually during the progresses of cell apoptosis and death. 7-AAD emits bright red fluorescence when it is excited by appropriate wavelength. 7-AAD negative cells possess normal viability; GFP efficiency indicates electroporation efficiency. Herein, taking "250-1" as an example, it represents a voltage of 250 V, and a pulse time of 1 ms.

The data of these experiments shows that, under the electroporation conditions of "300V, 1ms", the comprehensive index of electroporation efficiency and cell viability is the highest for cancer cell line K562, and they will be used as the electroporation conditions for subsequent experiments.

### 1-2. Transfecting the hematopoietic stem cells with GFP mRNA under the electroporation conditions of 300 v, 1 ms

5x10⁵ hematopoietic stem cells (purchased from ALLCELLs Biotechnology (Shanghai) Co., Ltd., www.allcells.com) are transfected with the above GFP mRNA by electroporation under the electroporation conditions of "300V, 1 ms" which produce the highest efficiency and viability in the previous step. GFP expression and 7-AAD expression are detected 4 days later. The results are shown in FIGs. 4 and 5.

FIG. 4 shows fluorescence photomicrographs obtained 4 days after transfecting the hematopoietic stem cells with the above GFP mRNA by electroporation under the electroporation conditions of 300V, 1 ms, and the four fields included are the bright field, green channel, red channel, and the overlay of the bright field and the green channel.

FIG. 5 is a flow cytometric analysis of GPF and CD34 protein expression, 4 days after transfecting the hematopoietic stem cells with GFP mRNA by electroporation under the electroporation conditions of 300 V, 1 ms. In FIG. 5, the control group is the hematopoietic stem cells not being transfected with GFP mRNA, and in the flow cytometric analysis CD34 antibodies are not stained.

It can be seen from the results of FIGs. 4 and 5 that, the data of these experiments shows the proportion of GFP expression in the umbilical cord blood-derived hematopoietic stem cells is high under the electroporation conditions of "300 V, 1 ms".

### 1-3: Gene editing of BCL11A locus in the cord blood-derived hematopoietic stem cells by electroporation with CRISPR/Cas9

A. Multiple sgRNAs for locus BCL11A (+58) are designed by using the "CRISPR RGEN TOOLS" software, and the chemically modified sgRNAs (the information shown in FIGs. 6, 7 and 8) are synthesized for the locus 58K of BCL11A enhancer (the sequence of the targeted locus 58K is shown in SEQ ID NO: 2).
SEQ ID NO: 2: the 150bp sequence at the locus 58K of BCL11A enhancer: 
ctgccagtcctcttctaccccacccacgcccccaccctaatcagaggccaaacccttcctggagcctgtgataaaa
gcaactgttagcttgcactagactagcttcaaagttgtattgaccctggtgtgttatgtctaagagtagatgcc
SEQ ID NO: 3: referred to as BCL11A Enhancer-1 sgRNA (sometimes abbreviated as Enhancer-1):
   cacaggctccaggaagggtt
SEQ ID NO: 4: referred to as BCL11A Enhancer-2 sgRNA (sometimes abbreviated as Enhancer-2):
   atcagaggccaaacccttcc
SEQ ID NO: 5: referred to as BCL11A Enhancer-3 sgRNA (sometimes abbreviated as Enhancer-3):
   ctaacagttgcttttatcac
SEQ ID NO: 6: referred to as BCL11A Enhancer-4 sgRNA (sometimes abbreviated as Enhancer-4):
   ttgcttttatcacaggctcc
SEQ ID NO: 7: referred to as BCL11A Enhancer-5 sgRNA (sometimes abbreviated as Enhancer-5):
   ttttatcacaggctccagga
SEQ ID NO: 8: referred to as BCL11A Enhancer-6 sgRNA (sometimes abbreviated as Enhancer-6):
   tttatcacaggctccaggaa
SEQ ID NO: 9: referred to as BCL11A Enhancer-7 sgRNA (sometimes abbreviated as Enhancer-7):
   tgggtggggtagaagaggac
SEQ ID NO: 10: referred to as BCL11A Enhancer-8 sgRNA (sometimes abbreviated as Enhancer-8):
   gggcgtgggtggggtagaag
SEQ ID NO: 11: referred to as BCL11A Enhancer-9 sgRNA (sometimes abbreviated as Enhancer-9):
   ttagggtgggggcgtgggtg
SEQ ID NO: 12: referred to as BCL11A Enhancer-10 sgRNA (sometimes abbreviated as Enhancer-10):
   attagggtgggggcgtgggt
SEQ ID NO: 13: referred to as BCL11A Enhancer-11 sgRNA (sometimes abbreviated as Enhancer-11):
   gattagggtgggggcgtggg
SEQ ID NO: 14: referred to as BCL11A Enhancer-12 sgRNA (sometimes abbreviated as Enhancer-12):
   tctgattagggtgggggcgt
SEQ ID NO: 15: referred to as BCL11A Enhancer-13 sgRNA (sometimes abbreviated as Enhancer-13):
   ctctgattagggtgggggcg
SEQ ID NO: 16: referred to as BCL11A Enhancer-14 sgRNA (sometimes abbreviated as Enhancer-14):
   cacgcccccaccctaatcag
SEQ ID NO: 17: referred to as BCL11A Enhancer-15 sgRNA (sometimes abbreviated as Enhancer-15):
   ttggcctctgattagggtgg
SEQ ID NO: 18: referred to as BCL11A Enhancer-16 sgRNA (sometimes abbreviated as Enhancer-16):
   tttggcctctgattagggtg
SEQ ID NO: 19: referred to as BCL11A Enhancer-17 sgRNA (sometimes abbreviated as Enhancer-17):
   gtttggcctctgattagggt
SEQ ID NO: 20: referred to as BCL11A Enhancer-18 sgRNA (sometimes abbreviated as Enhancer-18):
   ggtttggcctctgattaggg
SEQ ID NO: 21: referred to as BCL11A Enhancer-19 sgRNA (sometimes abbreviated as Enhancer-19):
   aagggtttggcctctgatta
SEQ ID NO: 22: referred to as BCL11A Enhancer-20 sgRNA (sometimes abbreviated as Enhancer-20):
   gaagggtttggcctctgatt
SEQ ID NO: 23: referred to as BCL11A Enhancer-21 sgRNA (sometimes abbreviated as Enhancer-21):
   actcttagacataacacacc
SEQ ID NO: 24: referred to as BCL11A Enhancer-22 sgRNA (sometimes abbreviated as Enhancer-22):
   cttcaaagttgtattgaccc
SEQ ID NO: 25: referred to as BCL11A Enhancer-23 sgRNA (sometimes abbreviated as Enhancer-23):
   ctcttagacataacacacca

As described above, the above 23 sgRNAs are designed for the 150 bp sequence at the locus 58K of BCL11A enhancer.

B. The electroporation conditions of "300 V, 1 ms" are used, and the inventors synthesized Cas9 mRNA (the sequence of SEQ ID NO: 26) and the 23 chemically modified sgRNAs which are designed as described above, wherein the chemical modification of the 23 sgRNAs is 2'-O-methyl modified and internucleotide 3'-thio modification of the first three bases at the 5' end and the last three bases at the 3' end of the sgRNA. As shown in the formulas below, the chemically modified sgRNA is shown on the left, and unmodified sgRNA is shown on the right.

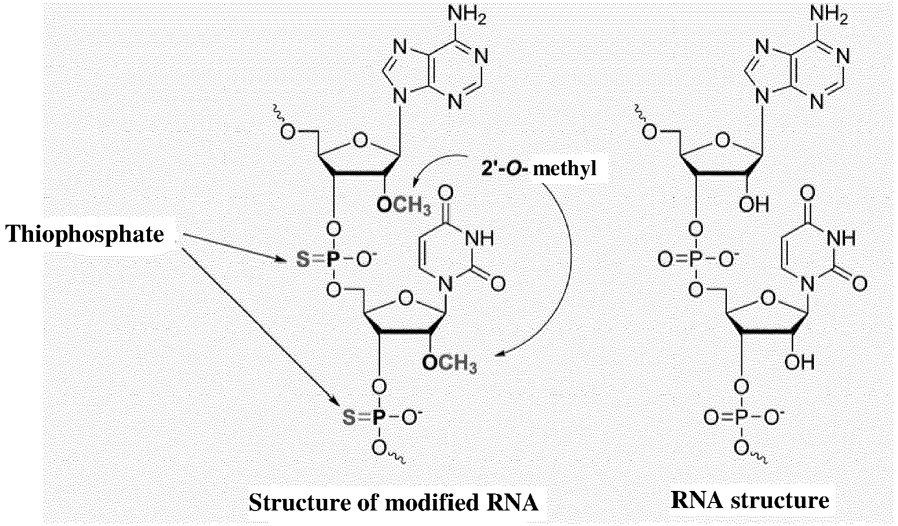

The cord blood-derived CD34-positive hematopoietic stem cells (purchased from ALLCELLS Biotechnology (Shanghai) Co., Ltd., www.allcells.com) are transfected with the above 23 chemically modified sgRNAs by electroporation under the above-identified electroporation conditions, analyzing the Indels efficiency by TIDE software 4 says later. The results are shown in FIG. 9. Also, in this example, as a comparison, the unmodified sgRNA is also used for transfection by electroporation in the same method, but the unmodified sgRNA produces an Indels efficiency of only 2.7%. The chemically modified sgRNAs are used in the following examples.

FIG. 9 shows that the CD34-positive hematopoietic stem cells are transfected with Cas9 mRNA and the 23 sgRNAs by electroporation. The genome of the CD34-positive hematopoietic stem cells is extracted after 4 days, and a fragment covering about 450 bp at the left and right side of the cleavage site of the sgRNA (with a total of 903 bp in length) is selected for amplification. The primer sequences for amplification are as follows:
Forward primer: cacctcagcagaaacaaagttatc (SEQ ID NO: 29)
Reverse primer: gggaagctccaaactctcaa (SEQ ID NO: 30)
Cleavage site selection: in the case of Enhancer-3, 5'-ctaaacagttg cttttatcac-3 (SEQ ID NO: 5), the cleavage site is at the right site of the 3' end (Cong L, et al. Science. 2013).

Sanger sequencing is performed to amplify the above fragment by using the upstream and downstream primer sequences as shown in SEQ ID NO: 29 and SEQ ID NO: 30. Based on the sequencing results, statistic analysis of the resulting Indels efficiency is performed by using TIDE software, wherein the TIDE software is an on-line software for Indels efficiency analysis. The efficiency of Indels for producing double-peak mutation may be analyzed according to the first-generation sequencing results by referring to Tide. deskgen. com.

The results show that all the 23 sgRNAs synthesized in this example may be used to successfully perform gene editing of the hematopoietic stem cells, and Indels may be effectively produced with an efficiency of at least 10%. The most efficient one of them is Enhancer-2.

SEQ ID NO: 26: the sequence of Cas9 mRNA
gacaagaagtacagcatcggcctggacatcggcaccaactctgtgggctgggccgtgatcaccgacgagtacaag
gtgcccagcaagaaattcaaggtgctgggcaacaccgaccggcacagcatcaagaagaacctgatcggagccctg
ctgttcgacagcggcgaaacagccgaggccacccggctgaagagaaccgccagaagaagatacaccagacgga
agaaccggatctgctatctgcaagagatcttcagcaacgagatggccaaggtggacgacagcttcttccacagactg

gaagagtccttcctggtggaagaggataagaagcacgagcggcaccccatcttcggcaacatcgtggacgaggtgg
cctaccacgagaagtaccccaccatctaccacctgagaaagaaactggtggacagcaccgacaaggccgacctgc
ggctgatctatctggccctggcccacatgatcaagttccggggccacttcctgatcgagggcgacctgaaccccgac
aacagcgacgtggacaagctgttcatccagctggtgcagacctacaaccagctgttcgaggaaaaccccatcaacgc
cagcggcgtggacgccaaggccatcctgtctgccagactgagcaagagcagacggctggaaaatctgatcgccca
gctgcccggcgagaagaagaatggcctgttcggcaacctgattgccctgagcctgggcctgacccccaacttcaag
agcaacttcgacctggccgaggatgccaaactgcagctgagcaaggacacctacgacgacgacctggacaacctg
ctggcccagatcggcgaccagtacgccgacctgtttctggccgccaagaacctgtccgacgccatcctgctgagcga
catcctgagagtgaacaccgagatcaccaaggcccccctgagcgcctctatgatcaagagatacgacgagcaccac
caggacctgaccctgctgaaagctctcgtgcggcagcagctgcctgagaagtacaaagagattttcttcgaccagag
caagaacggctacgccggctacattgacggcggagccagccaggaagagttctacaagttcatcaagcccatcctg
gaaaagatggacggcaccgaggaactgctcgtgaagctgaacagagaggacctgctgcggaagcagcggacctt
cgacaacggcagcatcccccaccagatccacctgggagagctgcacgccattctgcggcggcaggaagatttttac
ccattcctgaaggacaaccgggaaaagatcgagaagatcctgaccttccgcatcccctactacgtgggccctctggc
caggggaaacagcagattcgcctggatgaccagaaagagcgaggaaaccatcaccccctggaacttcgaggaagt
ggtggacaagggcgcttccgcccagagcttcatcgagcggatgaccaacttcgataagaacctgcccaacgagaag
gtgctgcccaagcacagcctgctgtacgagtacttcaccgtgtataacgagctgaccaaagtgaaatacgtgaccga
gggaatgagaaagcccgccttcctgagcggcgagcagaaaaaggccatcgtggacctgctgttcaagaccaaccg
gaaagtgaccgtgaagcagctgaaagaggactacttcaagaaaatcgagtgcttcgactccgtggaaatctccggcg
tggaagatcggttcaacgcctccctgggcacataccacgatctgctgaaaattatcaaggacaaggacttcctggaca
atgaggaaaacgaggacattctggaagatatcgtgctgaccctgacactgtttgaggacagagagatgatcgaggaa
cggctgaaaacctatgcccacctgttcgacgacaaagtgatgaagcagctgaagcggcggagatacaccggctgg
ggcaggctgagccggaagctgatcaacggcatccgggacaagcagtccggcaagacaatcctggatttcctgaagt
ccgacggcttcgccaacagaaacttcatgcagctgatccacgacgacagcctgacctttaaagaggacatccagaaa
gcccaggtgtccggccagggcgatagcctgcacgagcacattgccaatctggccggcagccccgccattaagaag
ggcatcctgcagacagtgaaggtggtggacgagctcgtgaaagtgatgggccggcacaagcccgagaacatcgtg
atcgaaatggccagagagaaccagaccacccagaagggacagaagaacagccgcgagagaatgaagcggatcg
aagagggcatcaaagagctgggcagccagatcctgaaagaacaccccgtggaaaacacccagctgcagaacgag
aagctgtacctgtactacctgcagaatgggcgggatatgtacgtggaccaggaactggacatcaaccggctgtccga
ctacgatgtggaccatatcgtgcctcagagctttctgaaggacgactccatcgacaacaaggtgctgaccagaagcg

acaagaaccggggcaagagcgacaacgtgccctccgaagaggtcgtgaagaagatgaagaactactggcggcag
ctgctgaacgccaagctgattacccagagaaagttcgacaatctgaccaaggccgagagaggcggcctgagcgaa
ctggataaggccggcttcatcaagagacagctggtggaaacccggcagatcacaaagcacgtggcacagatcctgg
actcccggatgaacactaagtacgacgagaatgacaagctgatccgggaagtgaaagtgatcaccctgaagtccaa
gctggtgtccgatttccggaaggatttccagttttacaaagtgcgcgagatcaacaactaccaccacgcccacgacgc
ctacctgaacgccgtcgtgggaaccgccctgatcaaaaagtaccctaagctggaaagcgagttcgtgtacggcgact
acaaggtgtacgacgtgcggaagatgatcgccaagagcgagcaggaaatcggcaaggctaccgccaagtacttctt
ctacagcaacatcatgaactttttcaagaccgagattaccctggccaacggcgagatccggaagcggcctctgatcga
gacaaacggcgaaaccggggagatcgtgtgggataagggccgggattttgccaccgtgcggaaagtgctgagcat
gccccaagtgaatatcgtgaaaaagaccgaggtgcagacaggcggcttcagcaaagagtctatcctgcccaagagg
aacagcgataagctgatcgccagaaagaaggactgggaccctaagaagtacggcggcttcgacagccccaccgtg
gcctattctgtgctggtggtggccaaagtggaaaagggcaagtccaagaaactgaagagtgtgaaagagctgctgg
ggatcaccatcatggaaagaagcagcttcgagaagaatcccatcgactttctggaagccaagggctacaaagaagtg
aaaaaggacctgatcatcaagctgcctaagtactccctgttcgagctggaaaacggccggaagagaatgctggcctc
tgccggcgaactgcagaagggaaacgaactggccctgccctccaaatatgtgaacttcctgtacctggccagccact
atgagaagctgaagggctcccccgaggataatgagcagaaacagctgtttgtggaacagcacaagcactacctgga
cgagatcatcgagcagatcagcgagttctccaagagagtgatcctggccgacgctaatctggacaaagtgctgtccg
cctacaacaagcaccgggataagcccatcagagagcaggccgagaatatcatccacctgtttaccctgaccaatctg
ggagcccctgccgccttcaagtactttgacaccaccatcgaccggaagaggtacaccagcaccaaagaggtgctgg
acgccaccctgatccaccagagcatcaccggcctgtacgagacacggatcgacctgtctcagctgggaggcgac

SEQ ID NO: 27, sequencing primer
cacctcagcagaaacaaagttatc

SEQ ID NO: 28, sequencing primer
gggaagctccaaactctcaa

C. According to the above experimental results, Enhancer-2, Enhancer-3, Enhancer-4, Enhancer-5 and Enhancer-6 with relatively high gene editing efficiency are selected. The umbilical cord blood-derived hematopoietic stem cells are transfected with Cas9 mRNA and Enhancer-2, Enhancer-3, Enhancer-4, Enhancer-5 or Enhancer-6 by electroporation under the electroporation conditions of 300V, 1ms; and the Indels efficiency is determined after 4 days, and the results are shown in FIG. 10. FIG. 10 shows the statistical analysis of the Indels efficiency by using the same method as above and the TIDE software, after transfecting the CD34-positive hematopoietic stem cells derived from 3 different cord blood sources with Cas9 mRNA and BCL11A Enhancer-2 sgRNA, Enhancer-3 sgRNA, Enhancer-4 sgRNA, Enhancer-5 sgRNA, and Enhancer-6 sgRNA respectively by electroporation 4 days later.

The results in FIG. 10 shows that, 5 sgRNAs achieve efficient gene editing in hematopoietic stem cells derived from 3 different cord blood sources with the efficiencies of at least 40% or more. Enhancer-2 sgRNA has the highest efficiency, the average gene editing efficiency of Enhancer-2 sgRNA is 80%, and it is significantly higher than that of Enhancer-3, Enhancer-4, Enhancer-5, and Enhancer-6 sgRNA. Meanwhile, the gene editing efficiency in hematopoietic stem cells is higher than that reported in the existing literatures (Xu, et al. Molecular Therapy. 2017; DeWitt, et al. Sci Transl Med. 2016).

Based on this, Enhancer-2 sgRNA is used as a target in the subsequent experiments.

### Example 2: In vitro colony-formation of the genetically modified hematopoietic stem cells derived from cord blood

This experiment involves the detection of colony forming unit (CFU) of the genetically modified hematopoietic stem cells derived from cord blood.

The hematopoietic stem cells are transfected with Cas9 mRNA and Enhancer-2 under the electroporation conditions of 300 V, 1 ms. 800-1000 cells are resuspended in 1 ml of the mixture of H4434 (available from STEM CELLS TECHNOLOGY, Canada), IMDM (available from Thermo Fisher) and FBS (available from Thermo Fisher). The number of the formed colonies with different morphologies such as CFU-M, BFU-E, CFU-E, CFU-G, CFU-GM are observed under microscope after 14 days, and the results are shown in FIG. 11. Among them, FIG. 11 shows the number of colonies for different blood systems, which is obtained by transfecting CD34-positive hematopoietic stem cells derived from cord blood with Cas9 mRNA and BCL11A enhancer-2 sgRNA by electroporation, performing *in vitro* colony-forming units assay (CFU assay) 2 days later, and then counting the number of colonies for different blood systems 14 days later; wherein BFU-E, CFU-M, CFU-GM, CFU-E, CFU-G, and CFU-MM represent the cell colonies of different lineages, such as erythroid, myeloid, and lymphoid lineage, etc. in blood system, and wherein Mock represents cells not being genetically modified.

According to the data shown in example 2, compared with that of the hematopoietic stem cells not being genetically modified, the *in vitro* differentiation function of the genetically modified cells is normal, and the cells are differentiated into colonies of different lineages of blood system.

### Example 3: The reconstruction of hematopoietic system in mouse model with genetically modified hematopoietic stem cells derived from cord blood

The cord blood-derived hematopoietic stem cells are transfected with Cas9 mRNA and Enhancer-2 by electroporation under the electroporation conditions of 300 V, 1 ms; transplanting into an irradiated NPG immunodeficient mouse model (purchased from Beijing Vitalstar Biotechnology, Inc.). The expression of human CD45 and mouse CD45 in peripheral blood is detected 6 weeks, 8 weeks, 10 weeks, 12 weeks and 16 weeks after the transplantation, while the expression of human CD45 and mouse CD45 in bone marrow and spleen is detected 16 weeks after transplantation; and the results are shown in FIGs. 12 and 14. The method for transplantation into the mice comprises the following steps: removing bone marrow from the mouse model by 1.0 Gy irradiation twenty-four hours prior to the cell transplantation; then resuspending 1.0×10⁶ cells in 20 µL of 0.9% saline and injecting through the tail vein of the mice which are subsequently raised in a clean room.

The results of FIGs. 12 and 14 show that, after transplantation into a mouse model, as compared with the genetically unmodified hematopoietic stem cells, the proportion of human hCD45 expression in the genetically modified hematopoietic stem cells continues to increase over time, and it increases from 20% at week 6 to 60% at week 16 in the peripheral blood samples, and even reaches 90% in bone marrow and 70% in spleen at week 16, indicating that the genetically modified hematopoietic stem cells may be transplanted quickly and efficiently into the hematopoietic system of a mouse model, and the *in vivo* differentiation function of the cells is normal. The reports of the existing literatures show that, the proportion of CD45 expression in peripheral blood is 1-10% after 6 weeks, the proportion of CD45 expression in peripheral blood is 20-40% after 16 weeks, and the proportion of CD45 expression in bone marrow is about 50%. Thus, the results of the animal experiments in the invention are obviously superior to those in the transplantation experiments reported in the prior art (Xu, et al., Molecular Therapy. 2017; DeWitt, et al., Sci Transl Med. 2016; Mettananda, et al., Nature Communications. 2017).

Meanwhile, in mice transplanted with genetically modified cells, the expression of cell membrane proteins such as human CD3, CD4, CD8, CD33, CD19, CD56 is detected after 16 weeks, as shown in FIGs. 13, 14 and 15. The results show that the genetically modified cells express these proteins normally, indicating that the cells may differentiate into T cells, B cells, macrophages and other cells of the blood system, and enable to efficiently repopulate the hematopoietic system of a mouse model. Compared with the results reported in the literatures, firstly, we test six cell membrane surface proteins including CD3, CD4, CD8, CD33, CD19 and CD56, to determine the expression of T cells, myeloid cells, B cells and NK cells in the blood of mice, and evaluate the capability of the transplanted human hematopoietic stem cells for repopulating the hematopoietic system of mice more accurately, while in the existing literatures, only proteins CD3, CD19, CD56, CD33 are tested; secondly, we test the expression profiles of the above six proteins in three important blood system related tissues including peripheral blood, bone marrow, and spleen, to evaluate the capacity of repopulating the hematopoietic system of the mouse more comprehensively; while in the existing literatures, only the bone marrow is taken as the test tissue (Chang, et al. Methods & Clinical Development. 2017; deWitt, et al. Sci Transl Med. 2016; Mettananda, et al. Nature Communications. 2017).

In addition, the genetically modified cells are capable of rapidly and efficiently repopulating the hematopoietic system of a mouse model. The result shown in FIG. 16 is used for judging whether the cells for repopulating a mouse model are genetically modified. Genomes of the cells before transplantation, and genomes of the peripheral blood, bone marrow and spleen 16 weeks after transplantation are extracted, amplifying the target fragments and performing Sanger sequencing. The Indels efficiency is determined by TIDE analysis. The results show that all the human cells in the peripheral blood, bone marrow and spleen are genetically modified 16 weeks after transplantation, and the Indels efficiency is from 50% to 70%, and similar to that of the cells before transplantation.

### Example 4: The erythroid differentiation of the genetically modified hematopoietic stem cells derived from cord blood and the Detection of γ globin and fetal hemoglobin expression

### 4-1. Differentiation of erythrocytes

The cord blood-derived hematopoietic stem cells are transfected with Cas9 mRNA and Enhancer-2 by electroporation under the electroporation conditions of 300 v, 1 ms, differentiating the cells by using the "two-step" differentiation protocol described below.

In the two-step method, firstly the HSPCs erythroid expansion and differentiation medium is used for differentiation, then the HSPCs erythroid differentiation and enucleation medium is used for differentiation.

The basal medium of the HSPCs erythroid expansion and differentiation culture medium is StemSpan™ SFEM II, including the growth factors of 50-200ng/ml SCF, 10-100ng/ml IL-3, and 1-10U EPO/ml. The culture conditions: the hematopoietic stem cells are cultured with a density of 1.0×10⁵ cells/ml in the erythroid expansion and differentiation medium for 7 days.

The basal medium of HSPCs erythroid differentiation and enucleation medium is StemSpan™ SFEM II including the growth factors of 1-10 U EPO, 100-1000 µg/ml human transferrin, 0.5-10 µm the small molecule of mifepristone. The cells cultured in the previous step with a density of 1.0×10⁶ cells/ml are differentiated in the HSPCs erythroid differentiation and enucleation medium for 5 days.

Then, the expression of CD71 and CD235a is detected, as shown in FIG. 17. In the experimental group and the control group, the cells are differentiated into erythrocytes efficiently, and the proportion of CD71 and CD235a expression is 90% or more.

### 4-2. Detection of the expression of γ globin and fetal hemoglobin

mRNA of the cells differentiated from the above erythrocytes is extracted and reverse-transcribed into cDNA. The expression of BCL11A, HBB, HBG and other genes is detected by quantitative fluorescence PCR, as shown in FIG. 18. The results show that in cells with knockout of the BCL11A enhancer locus, the expression of BCL11A gene is down-regulated by 1-fold, and the expression of HBG is up-regulated by 1-fold.

Fetal hemoglobin expression in cells after erythroid differentiation is detected, as shown in FIG. 19. Flow cytometry analysis shows that in cells with knockout of BCL11A enhancer locus, fetal hemoglobin expression is increased approximately 1-fold.

### Example 5: Gene editing of the BCL11A enhancer locus in the hematopoietic stem cells derived from β thalassemia patients

### 5-1. Isolation of the hematopoietic stem cells from peripheral blood of patients with β thalassemia

CD34-positive hematopoietic stem cells are obtained by conventional magnetic bead sorting. The results are shown in FIG. 20.

### 5-2. Transfecting the hematopoietic stem cells derived from the peripheral blood of β thalassemia patients by electroporation

The peripheral blood-derived hematopoietic stem cells from patients with β thalassemia are transfected with Cas9 mRNA and Enhancer-2, Enhancer-3, Enhancer-4, Enhancer-5, or Enhancer-6 by electroporation under the electroporation conditions of 300 V, 1 ms. Indels efficiency is detected 4 days later, as shown in FIG. 21. The results show that efficient gene editing is achieved in peripheral blood-derived hematopoietic stem cells from 3 different patients by using each of the 5 sgRNAs, wherein Enhancer-2 has the highest efficiency of at least 70% or more.

### Example 6: The erythroid differentiation of the genetically modified hematopoietic stem cells derived from peripheral blood of patients with anemia and the Detection of γ globin and fetal hemoglobin expression

The genetically modified hematopoietic stem cells of Example 5 are subjected to erythroid differentiation *in vitro* as described in Example 4-1. Differentiation results are shown in FIG.23. The results of the experiments show that, the erythroid differentiation efficiencies of the control group, Enhancer-2, Enhancer-3, Enhancer-4, Enhancer-5 and Enhancer-6 are similar, and the cell membrane proteins CD71 and CD235a are highly expressed.

12 days later, mRNA of cells after erythroid differentiation is extracted and reverse-transcribed into cDNA. The expressions of BCL11A, HBB, HBG, HBA and other genes are determined by quantitative fluorescence PCR, as shown in FIG. 24. The results show that Enhancer-2, Enhancer-3, Enhancer-4 and Enhancer-5, but not Enhancer-6, down-regulate the expression of BCL11A gene, wherein Enhancer-2 has the most significant effect that the expression is down-regulated by about 1-fold. In addition, each of Enhancer-2, Enhancer-3, Enhancer-4, Enhancer-5 and Enhancer-6 increases the expression of γ globin, wherein Enhancer-2 has the most significant effect that the expression of γ globin is enhanced by 9-fold, meeting the standard of clinical treatment. Compared with the results in the existing literatures, firstly, the hematopoietic stem cells derived from severe thalassemia patients are used as the original cells for the accurate evaluation of the method according to the invention, and the method is verified to meet the requirements of clinical treatment, while in the existing literatures, only normal human bone marrow or peripheral blood samples are used; secondly, the increased folds of fetal hemoglobin expression reported in the prior literatures are only 4-7 folds, which is less than the increased proportion of fetal hemoglobin expression in the present invention. Thus, it shows that the experimental results of the present invention are significantly superior to that in the existing literatures reports (Chang et al. Methods & Clinical Development. 2017; mattew C et al. Nature.2015).

In addition, in order to more accurately evaluate the effects on the protein expression levels of fetal hemoglobin (HbF) and normal hemoglobin (HbA) caused by the gene editing of BCL11A enhancer locus with Enhancer-2, Enhancer-3, Enhancer-4, Enhancer-5 and Enhancer-6, in the present invention, we extract the proteins of the erythrocytes obtained 12 days after differentiation of the hematopoietic stem cells, performing HPLC assay (High Performance Liquid Chromatography), as shown in FIGs. 27, 28, and 29. The results show that, 1) the chromatographic peaks with the retention times of about 5.4 min and 10 min represent HbF and HbA, respectively; 2) as compared with cells not being genetically modified, cells genetically modified with Enhancer-2, Enhancer-3, Enhancer-4, Enhancer-5, and Enhancer-6 genes have higher HbF expression (higher peak and larger peak area) and lower HbA expression (lower peak and smaller peak area), indicating that the gene editing pf BCL11A Enhancer locus increases HbF expression and down regulates HbA expression; 3) among them, the ratio of HbF/HbA in cells of Mock group is about 0.79, while the ratio of HbF/HbA in cells of Enhancer-2 group is about 5.28, which is increased by 6.68 times and significantly higher than the ratio of HbF/HbA in the cells of the Enhancer-3, Enhancer-4, Enhancer-5, and Enhancer-6 group. Clinically, as for patients with severe β thalassemia as described in the context of the present invention, the hemoglobin in the blood consists of a small amount of HbA and a majority of HbF, due to the absence of β globin. Typically, there's about 20 g/L hemoglobin in patients with severe β thalassemia, wherein the proportion of HbF is up to 90% or more. Accordingly, taking the proportion of 90% as an example, the expression of HbF in genetically modified cells increases 6.68 times, the final hemoglobin content is 20×0.9×6.68+20×0.1 = 122.24 g/L, which absolutely meets the standard of clinical treatment, as the hemoglobin expression in normal subjects is 115-150 g/L (Antonio Cao, et al. Genes in Medicine. 2010; guidelines for Diagnosis and Treatment of Severe B thalassemia in 2010). Compared with the prior literatures, the invention is the only research in which the effect of gene editing of BCL11A enhancer in the hematopoietic stem cells of β thalassemia patients on improving the fetal hemoglobin expression is accurately evaluated by HPLC experiment, and the result shows that the expression of the fetal hemoglobin is significantly enhanced and meets the requirements for the clinical treatment of severe β thalassemia patients (Chang et al. Methods & Clinical Development. 2017; mattew C et al. Nature.2015; lin Ye, et al. PNAS. 2016; matthew H. Porteus, Advances in Experimental Medicine and Biology. 2013).

### Example 7: In vitro Colony-formation of genetically modified hematopoietic stem cells derived from peripheral blood of anemia patients

The hematopoietic stem cells derived from peripheral blood of anemia patients are transfected with Cas9 mRNA and Enhancer-2 by electroporation under the electroporation conditions of 300 V, 1 ms. 500-800 cells are resuspended in 1 ml of the mixture of H4434 (available from STEM CELLS TECHNOLOGY, Canada), IMDM (available from Thermo Fisher) and FBS (available from Thermo Fisher). The number of the formed colonies with different morphologies such as CFU-M, BFU-E, CFU-E, CFU-G, CFU-GM, and GEMM are observed under microscope 14 days later. The results are shown in FIG. 25.

The experimental data show that, compared with the hematopoietic stem cells not being genetically modified, the *in vitro* differentiation function of the genetically modified cells is normal, and the cells are differentiated into colonies of different lineages in blood system.

### Example 8: Off-target effect of the genetically modified hematopoietic stem cells derived from peripheral blood of anemia patients

The experiment relates to a gene sequencing method, in particular to an off-target effect analysis of the genetically modified hematopoietic stem cells derived from peripheral blood of an anemia patient by the next generation sequencing (NGS) technology.

The hematopoietic stem cells from peripheral blood of anemia patients are transfected with Cas9 mRNA and Enhancer-2 by electroporation under the electroporation conditions of 300 V, 1 ms. After 4 days of expansion, the genome of the cells is extracted and analyzed by next generation sequencing. 14 potential off-target sites are predicted by software, and the results are shown in FIG. 22.

The experimental data shows that, the on-target efficiency of gene editing is 76%, and the proportion of the 14 potential off-target sites is less than 0.3%, which is equivalent to the error of the next generation sequencing per se. Therefore, in the range of the sequencing error, off-target phenomenon caused by gene editing is not detected. Thus, this gene editing scheme is safe.

As shown in the data of the above examples, as for cells derived from different sources, Enhancer-2 shows the best effect.

### INDUSTRIAL APPLICABILITY

According to the invention, the method provided herein has the following advantages: firstly, the method may be used for gene editing of the hematopoietic stem cells derived from thalassemia patients, and it completely meets the requirements for the clinical treatment of thalassemia and sickle cell anemia; secondly, by using the chemically modified sgRNA, the gene editing efficiency of the method is high, the expression of fetal hemoglobin is remarkably improved, and the cells can be used for repopulating the hematopoietic system of a mouse model; and finally, the off-target analysis shows a high degree of safety. Accordingly, the method developed in the present invention has the potential to replace the conventional hematopoietic stem cell transplantation therapy technique for curing patients with severe thalassemia and sickle cell anemia.

Although the invention has been described in detail with reference to specific features, it is obvious to those skilled in the art that this description is intended for the illustration of the preferred embodiments only, and is not intended to limit the scope of the invention. Therefore, the essential scope of the invention is defined by the appended claims and their equivalents.

## Claims

1. A method for increasing fetal hemoglobin (HbF) expression in human hematopoietic stem cells, comprising:
disrupting a BCL11A genomic region from positions 60495219-60495336 in the chromosome 2 of the hematopoietic stem cells by gene editing technology.

2. The method of claim 1, wherein the gene editing technology is a zinc finger nuclease-based gene editing technology, a TALEN gene editing technology, or a CRISPR/Cas gene editing technology.

3. The method of claim 2, wherein the gene editing technology is CRISPR/Cas9 gene editing technology.

4. The method of any one of claims 1-3, wherein the target nucleotide sequence of BCL11A genome is complementary to a sequence selected from any one of SEQ ID NOs: 3-25.

5. The method of claim 3 or 4, an sgRNA comprising a sequence selected from any one of SEQ ID NOs: 3-25 is introduced into the hematopoietic stem cell for the gene editing of the BCL11A genome.

6. The method of claim 5, wherein the sgRNA is 2'-O-methyl modified and/or internucleotide 3'-thio modified.

7. The method of claim 6, wherein the chemical modification is 2'-O-methyl modification of the first one, two and/or three bases at the 5' end and/or the last base at the 3' end of the sgRNA.

8. The method of any one of claims 3-7, wherein the sgRNA and the Cas9-encoding nucleotides are co-introduced into the hematopoietic stem cells.

9. The method of claim 8, wherein the sgRNA and the Cas9-encoding nucleotides are co-introduced into the hematopoietic stem cells by electroporation.

10. The method of claim 9, wherein the electroporation conditions are 200-600 V, 0.5-2 ms.

11. A method for efficiently editing hematopoietic stem cells in vitro by the CRISPR/Cas9 system, comprising: introducing an sgRNA comprising a sequence selected from any one of SEQ ID NOs: 3-25 into the hematopoietic stem cells, wherein the sgRNA is 2'-O-methyl modified and/or internucleotide 3'-thio modified.

12. The method of claim 11, wherein the sgRNA and the Cas9-encoding nucleotides are co-introduced into the hematopoietic stem cells.

13. The method of claim 12, wherein the sgRNA and the Cas9-encoding nucleotides are co-introduced into the hematopoietic stem cells by electroporation.

14. The method of claim 13, wherein the electroporation conditions are 200-600 V, 0.5-2 ms.

15. A hematopoietic stem cell obtained by the method of any one of claims 1-14.

16. A human hematopoietic stem cell increasing the fetal hemoglobin (HbF) expression by genetic modification, wherein a BCL11A genomic region from positions 60495219-60495336 in the chromosome 2 of the hematopoietic stem cell is disrupted by gene editing technology.

17. Precursor cells at different stages of differentiation before mature erythrocytes are obtained by differentiation culture of the hematopoietic stem cells of claim15 or 16.

18. Mature erythrocytes obtained by differentiation culture of the hematopoietic stem cells of claim 15 or 16.

19. A method for preparing mature erythrocytes or precursor cells thereof being genetically modified to increase the fetal hemoglobin (HbF) expression, comprising:
(a) obtaining genetically modified hematopoietic stem cells by the method of any one of claims 1-14;
(b) performing hematopoietic stem cell erythroid expansion and differentiation of the genetically modified hematopoietic stem cells by using a HSPCs erythroid expansion and differentiation medium;
wherein the HSPCs erythroid expansion and differentiation medium comprises a basal medium and a composition of growth factors, and wherein the composition of growth factors comprises a stem cell growth factor (SCF); interleukin 3, (IL-3) and erythropoietin (EPO).

20. The method of claim 19, further comprising:
performing erythroid differentiation and enucleation on hematopoietic stem cells by using an erythroid differentiation and enucleation medium;
wherein the erythroid differentiation and enucleation medium comprises a basal medium, growth factors, and antagonists and/or inhibitors of a progesterone receptor and a glucocorticoid receptor.

21. The method of claim 20, wherein the growth factors in the erythroid differentiation and enucleation medium comprise erythropoietin (EPO), wherein the antagonists and/or inhibitors of a progesterone receptor and a glucocorticoid receptor are any one, or two or more selected from the following compounds (I)-(IV):

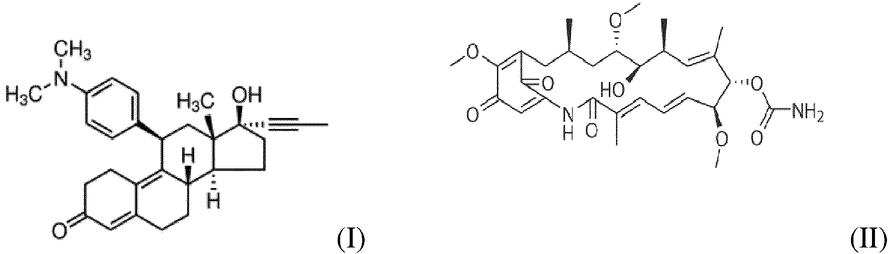

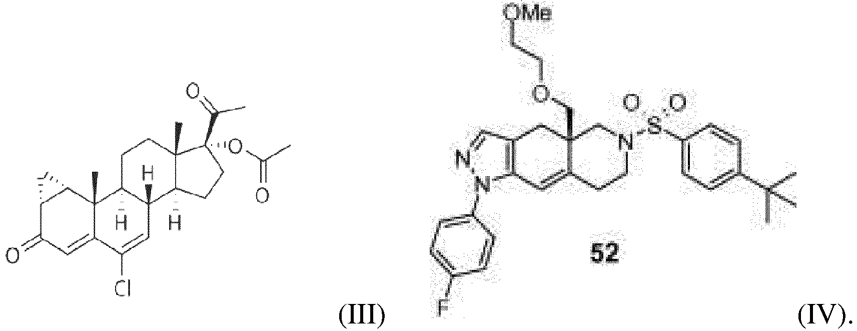

22. A composition comprising the hematopoietic stem cells of claim 15 or 16, or the precursor cells of claim 17, or the mature erythrocytes of claim 18.

23. A medical preparation comprising the hematopoietic stem cells of claim 15 or 16, or the precursor cells of claim 17, or the mature erythrocytes of claim 18.

24. Use of the hematopoietic stem cells of claim 15 or 16, or the precursor cells of claim 17, or the mature erythrocytes of claim 18 in the prevention or treatment of a disease in a subject in need thereof.

25. The use of claim 24, wherein the disease is an anemia disease, a hemorrhagic disease, a tumor or other diseases requiring massive blood transfusion for prevention or treatment.

26. The use of claim 25, wherein the disease is β thalassemia or sickle cell anemia.

27. The use of any one of claims 24-26, wherein the subject is a human.

28. Use of the hematopoietic stem cells of claim 15 or 16, or the precursor cells of claim 17, or the mature erythrocytes of claim 18 in the manufacture of a medicament or medical preparation for preventing or treating a disease in a subject.

29. The use of claim 28, wherein the disease is an anemia disease, a hemorrhagic disease, a tumor or other disease requiring massive blood transfusion for prevention or treatment.

30. The use of claim 29, wherein the disease is β thalassemia or sickle cell anemia.

31. The use of any one of claims 28-30, wherein the subject is a human.

32. A sgRNA construct comprising a nucleotide sequence selected from any one of SEQ ID NOs: 3-25

33. The construct of claim 32, comprising a 2'-O-methyl nucleotide modification and/or an internucleotide 3'-thio modification.

34. The construct of claim 33, wherein the chemical modification is a 2'-O-methyl modification of the first one, two and/or three bases at the 5' end and/or the last base at the 3' end of a nucleotide sequence selected from any one of SEQ ID NOs: 3-25.

35. A vector, a host cell or a formulation comprising the construct of any one of claims 32-34.

36. Use of the construct of any one of claims 32-34 in performing gene editing of hematopoietic stem cells.

37. A method for treating or preventing an anemia disease, a hemorrhagic disease, a tumor, or other diseases requiring massive blood transfusion in a subject, comprising administering the hematopoietic stem cells of claim 15 or 16, the precursor cells of claim 17, or the mature erythrocytes of claim 18 to the subject.

38. The method of claim 37, wherein the disease is β thalassemia or sickle cell anemia.

39. The method of claim 38, wherein the subject is a human.


SEQUENCE LISTING

<110>  EdiGene Inc.

<120>  METHOD FOR INCREASING FETAL HEMOGLOBIN EXPRESSION LEVEL

<130>  BET 20M1676

<140>  EP 18870256.7
<141>  2018-10-26

<150>  CN 201711027708.6
<151>  2017-10-27

<160>  30

<170>  PatentIn version 3.5

<210>  1
<211>  717
<212>  DNA
<213>  Artificial sequence

<220>
<223>  GFP mRNA sequence information

<400>  1
atgagtaaag gagaagaact tttcactgga gttgtcccaa ttcttgttga attagatggt      60

gatgttaatg ggcacaaatt ttctgtcagt ggagagggtg aaggtgatgc aacatacgga     120

aaacttaccc ttaaatttat ttgcactact ggaaaactac ctgttccatg gccaacactt     180

gtcactactt tctcttatgg tgttcaatgc ttttcaagat acccagatca tatgaaacgg     240

catgactttt tcaagagtgc catgcccgaa ggttatgtac aggaaagaac tatatttttc     300

aaagatgacg ggaactacaa gacacgtgct gaagtcaagt ttgaaggtga tacccttgtt     360

aatagaatcg agttaaaagg tattgatttt aaagaagatg gaaacattct tggacacaaa     420

ttggaataca actataactc acacaatgta tacatcatgg cagacaaaca aaagaatgga     480

atcaaagtta acttcaaaat tagacacaac attgaagatg gaagcgttca actagcagac     540

cattatcaac aaaatactcc aattggcgat ggccctgtcc ttttaccaga caaccattac     600

ctgtccacac aatctgccct ttcgaaagat cccaacgaaa agagagacca catggtcctt     660

cttgagtttg taacagctgc tgggattaca catggcatgg atgaactata caaatag        717


<210>  2
<211>  150
<212>  DNA
<213>  Artificial sequence

<220>
<223>  150 bp sequence of locus 58K of BCL11A enhancer

<400>  2
ctgccagtcc tcttctaccc cacccacgcc cccaccctaa tcagaggcca aacccttcct      60

ggagcctgtg ataaaagcaa ctgttagctt gcactagact agcttcaaag ttgtattgac 120

cctggtgtgt tatgtctaag agtagatgcc 150


<210> 3
<211> 20
<212> DNA
<213> Artificial sequence

<220>
<223> An sgRNA named as BCL11A Enhancer-1 (sometimes abbreviated as
 Enhancer-1)

<400> 3
cacaggctcc aggaagggtt 20


<210> 4
<211> 20
<212> DNA
<213> Artificial sequence

<220>
<223> An sgRNA named as BCL11A Enhancer-2 (sometimes abbreviated as
 Enhancer-2)

<400> 4
atcagaggcc aaacccttcc 20


<210> 5
<211> 20
<212> DNA
<213> Artificial sequence

<220>
<223> An sgRNA named as BCL11A Enhancer-3 (sometimes abbreviated as
 Enhancer-3)

<400> 5
ctaacagttg cttttatcac 20


<210> 6
<211> 20
<212> DNA
<213> Artificial sequence

<220>
<223> An sgRNA named as BCL11A Enhancer-4 (sometimes abbreviated as
 Enhancer-4)

<400> 6
ttgcttttat cacaggctcc 20


<210> 7
<211> 20
<212> DNA
<213> Artificial sequence

<220>

<223>  An sgRNA named as BCL11A Enhancer-5 (sometimes abbreviated as
       Enhancer-5)

<400>  7
ttttatcaca ggctccagga                                                   20


<210>  8
<211>  20
<212>  DNA
<213>  Artificial sequence

<220>
<223>  An sgRNA named as BCL11A Enhancer-6 (sometimes abbreviated as
       Enhancer-6)

<400>  8
tttatcacag gctccaggaa                                                   20


<210>  9
<211>  20
<212>  DNA
<213>  Artificial sequence

<220>
<223>  An sgRNA named as BCL11A Enhancer-7 (sometimes abbreviated as
       Enhancer-7)

<400>  9
tgggtggggt agaagaggac                                                   20


<210>  10
<211>  20
<212>  DNA
<213>  Artificial sequence

<220>
<223>  An sgRNA named as BCL11A Enhancer-8 (sometimes abbreviated as
       Enhancer-8)

<400>  10
gggcgtgggt ggggtagaag                                                   20


<210>  11
<211>  20
<212>  DNA
<213>  Artificial sequence

<220>
<223>  An sgRNA named as BCL11A Enhancer-9 (sometimes abbreviated as
       Enhancer-9)

<400>  11
ttagggtggg ggcgtgggtg                                                   20


<210>  12
<211>  20
<212>  DNA
<213>  Artificial sequence

<220>
<223>  An sgRNA named as BCL11A Enhancer-10 (sometimes abbreviated as
      Enhancer-10)

<400>  12
attagggtgg gggcgtgggt                                                   20


<210>  13
<211>  20
<212>  DNA
<213>  Artificial sequence

<220>
<223>  An sgRNA named as BCL11A Enhancer-11 (sometimes abbreviated as
      Enhancer-11)

<400>  13
gattagggtg ggggcgtggg                                                   20


<210>  14
<211>  20
<212>  DNA
<213>  Artificial sequence

<220>
<223>  An sgRNA named as BCL11A Enhancer-12 (sometimes abbreviated as
      Enhancer-12)

<400>  14
tctgattagg gtgggggcgt                                                   20


<210>  15
<211>  20
<212>  DNA
<213>  Artificial sequence

<220>
<223>  An sgRNA named as BCL11A Enhancer-13 (sometimes abbreviated as
      Enhancer-13)

<400>  15
ctctgattag ggtgggggcg                                                   20


<210>  16
<211>  20
<212>  DNA
<213>  Artificial sequence

<220>
<223>  An sgRNA named as BCL11A Enhancer-14 (sometimes abbreviated as
      Enhancer-14)

<400>  16
cacgccccca ccctaatcag                                                   20


<210>  17
<211>  20

<212>  DNA
<213>  Artificial sequence

<220>
<223>  An sgRNA named as BCL11A Enhancer-15 (sometimes abbreviated as
       Enhancer-15)

<400>  17
ttggcctctg attagggtgg                                                    20


<210>  18
<211>  20
<212>  DNA
<213>  Artificial sequence

<220>
<223>  An sgRNA named as BCL11A Enhancer-16 (sometimes abbreviated as
       Enhancer-16)

<400>  18
tttggcctct gattagggtg                                                    20


<210>  19
<211>  20
<212>  DNA
<213>  Artificial sequence

<220>
<223>  An sgRNA named as BCL11A Enhancer-17 (sometimes abbreviated as
       Enhancer-17)

<400>  19
gtttggcctc tgattagggt                                                    20


<210>  20
<211>  20
<212>  DNA
<213>  Artificial sequence

<220>
<223>  An sgRNA named as BCL11A Enhancer-18 (sometimes abbreviated as
       Enhancer-18)

<400>  20
ggtttggcct ctgattaggg                                                    20


<210>  21
<211>  20
<212>  DNA
<213>  Artificial sequence

<220>
<223>  An sgRNA named as BCL11A Enhancer-19 (sometimes abbreviated as
       Enhancer-19)

<400>  21
aagggtttgg cctctgatta                                                    20

<210>  22
<211>  20
<212>  DNA
<213>  Artificial sequence

<220>
<223>  An sgRNA named as BCL11A Enhancer-20 (sometimes abbreviated as
       Enhancer-20)

<400>  22
gaagggtttg gcctctgatt                                                   20


<210>  23
<211>  20
<212>  DNA
<213>  Artificial sequence

<220>
<223>  An sgRNA named as BCL11A Enhancer-21 (sometimes abbreviated as
       Enhancer-21)

<400>  23
actcttagac ataacacacc                                                   20


<210>  24
<211>  20
<212>  DNA
<213>  Artificial sequence

<220>
<223>  An sgRNA named as BCL11A Enhancer-22 (sometimes abbreviated as
       Enhancer-22)

<400>  24
cttcaaagtt gtattgaccc                                                   20


<210>  25
<211>  20
<212>  DNA
<213>  Artificial sequence

<220>
<223>  An sgRNA named as BCL11A Enhancer-23 (sometimes abbreviated as
       Enhancer-23)

<400>  25
ctcttagaca taacacacca                                                   20


<210>  26
<211>  4101
<212>  DNA
<213>  Artificial sequence

<220>
<223>  Cas9 mRNA sequence

<400>  26
gacaagaagt acagcatcgg cctggacatc ggcaccaact ctgtgggctg ggccgtgatc       60

accgacgagt acaaggtgcc cagcaagaaa ttcaaggtgc tgggcaacac cgaccggcac 120

agcatcaaga agaacctgat cggagccctg ctgttcgaca gcggcgaaac agccgaggcc 180

acccggctga agagaaccgc cagaagaaga tacaccagac ggaagaaccg gatctgctat 240

ctgcaagaga tcttcagcaa cgagatggcc aaggtggacg acagcttctt ccacagactg 300

gaagagtcct tcctggtgga agaggataag aagcacgagc ggcaccccat cttcggcaac 360

atcgtggacg aggtggccta ccacgagaag taccccacca tctaccacct gagaaagaaa 420

ctggtggaca gcaccgacaa ggccgacctg cggctgatct atctggccct ggcccacatg 480

atcaagttcc ggggccactt cctgatcgag ggcgacctga accccgacaa cagcgacgtg 540

gacaagctgt tcatccagct ggtgcagacc tacaaccagc tgttcgagga aaaccccatc 600

aacgccagcg gcgtggacgc caaggccatc ctgtctgcca gactgagcaa gagcagacgg 660

ctggaaaatc tgatcgccca gctgcccggc gagaagaaga atggcctgtt cggcaacctg 720

attgccctga gcctgggcct gacccccaac ttcaagagca acttcgacct ggccgaggat 780

gccaaactgc agctgagcaa ggacacctac gacgacgacc tggacaacct gctggcccag 840

atcggcgacc agtacgccga cctgtttctg gccgccaaga acctgtccga cgccatcctg 900

ctgagcgaca tcctgagagt gaacaccgag atcaccaagg cccccctgag cgcctctatg 960

atcaagagat acgacgagca ccaccaggac ctgaccctgc tgaaagctct cgtgcggcag 1020

cagctgcctg agaagtacaa agagattttc ttcgaccaga gcaagaacgg ctacgccggc 1080

tacattgacg gcggagccag ccaggaagag ttctacaagt tcatcaagcc catcctggaa 1140

aagatggacg gcaccgagga actgctcgtg aagctgaaca gagaggacct gctgcggaag 1200

cagcggacct tcgacaacgg cagcatcccc caccagatcc acctgggaga gctgcacgcc 1260

attctgcggc ggcaggaaga tttttaccca ttcctgaagg acaaccggga aaagatcgag 1320

aagatcctga ccttccgcat cccctactac gtgggccctc tggccagggg aaacagcaga 1380

ttcgcctgga tgaccagaaa gagcgaggaa accatcaccc cctggaactt cgaggaagtg 1440

gtggacaagg gcgcttccgc ccagagcttc atcgagcgga tgaccaactt cgataagaac 1500

ctgcccaacg agaaggtgct gcccaagcac agcctgctgt acgagtactt caccgtgtat 1560

aacgagctga ccaaagtgaa atacgtgacc gagggaatga gaaagcccgc cttcctgagc 1620

ggcgagcaga aaaaggccat cgtggacctg ctgttcaaga ccaaccggaa agtgaccgtg 1680

aagcagctga aagaggacta cttcaagaaa atcgagtgct tcgactccgt ggaaatctcc 1740

ggcgtggaag atcggttcaa cgcctccctg ggcacatacc acgatctgct gaaaattatc 1800

aaggacaagg acttcctgga caatgaggaa aacgaggaca ttctggaaga tatcgtgctg 1860

accctgacac tgtttgagga cagagagatg atcgaggaac ggctgaaaac ctatgcccac 1920

ctgttcgacg acaaagtgat gaagcagctg aagcggcgga gatacaccgg ctggggcagg 1980

ctgagccgga agctgatcaa cggcatccgg gacaagcagt ccggcaagac aatcctggat 2040

ttcctgaagt ccgacggctt cgccaacaga aacttcatgc agctgatcca cgacgacagc 2100

ctgaccttta aagaggacat ccagaaagcc caggtgtccg gccagggcga tagcctgcac 2160

gagcacattg ccaatctggc cggcagcccc gccattaaga agggcatcct gcagacagtg 2220

aaggtggtgg acgagctcgt gaaagtgatg ggccggcaca agcccgagaa catcgtgatc 2280

gaaatggcca gagagaacca gaccacccag aagggacaga agaacagccg cgagagaatg 2340

aagcggatcg aagagggcat caaagagctg ggcagccaga tcctgaaaga acaccccgtg 2400

gaaaacaccc agctgcagaa cgagaagctg tacctgtact acctgcagaa tgggcgggat 2460

atgtacgtgg accaggaact ggacatcaac cggctgtccg actacgatgt ggaccatatc 2520

gtgcctcaga gctttctgaa ggacgactcc atcgacaaca aggtgctgac cagaagcgac 2580

aagaaccggg gcaagagcga caacgtgccc tccgaagagg tcgtgaagaa gatgaagaac 2640

tactggcggc agctgctgaa cgccaagctg attacccaga gaaagttcga caatctgacc 2700

aaggccgaga gaggcggcct gagcgaactg gataaggccg gcttcatcaa gagacagctg 2760

gtggaaaccc ggcagatcac aaagcacgtg gcacagatcc tggactcccg gatgaacact 2820

aagtacgacg agaatgacaa gctgatccgg gaagtgaaag tgatcaccct gaagtccaag 2880

ctggtgtccg atttccggaa ggatttccag ttttacaaag tgcgcgagat caacaactac 2940

caccacgccc acgacgccta cctgaacgcc gtcgtgggaa ccgccctgat caaaaagtac 3000

cctaagctgg aaagcgagtt cgtgtacggc gactacaagg tgtacgacgt gcggaagatg 3060

atcgccaaga gcgagcagga aatcggcaag gctaccgcca agtacttctt ctacagcaac 3120

atcatgaact ttttcaagac cgagattacc ctggccaacg gcgagatccg gaagcggcct 3180

ctgatcgaga caaacggcga aaccggggag atcgtgtggg ataagggccg ggattttgcc 3240

accgtgcgga aagtgctgag catgccccaa gtgaatatcg tgaaaaagac cgaggtgcag 3300

acaggcggct tcagcaaaga gtctatcctg cccaagagga acagcgataa gctgatcgcc 3360

agaaagaagg actgggaccc taagaagtac ggcggcttcg acagccccac cgtggcctat 3420

tctgtgctgg tggtggccaa agtggaaaag ggcaagtcca agaaactgaa gagtgtgaaa 3480

gagctgctgg ggatcaccat catggaaaga agcagcttcg agaagaatcc catcgacttt 3540

ctggaagcca agggctacaa agaagtgaaa aaggacctga tcatcaagct gcctaagtac 3600

tccctgttcg agctggaaaa cggccggaag agaatgctgg cctctgccgg cgaactgcag 3660

aagggaaacg aactggccct gccctccaaa tatgtgaact tcctgtacct ggccagccac 3720

tatgagaagc tgaagggctc ccccgaggat aatgagcaga aacagctgtt tgtggaacag 3780

cacaagcact acctggacga gatcatcgag cagatcagcg agttctccaa gagagtgatc 3840

ctggccgacg ctaatctgga caaagtgctg tccgcctaca acaagcaccg ggataagccc 3900

atcagagagc aggccgagaa tatcatccac ctgtttaccc tgaccaatct gggagcccct 3960

gccgccttca agtactttga caccaccatc gaccggaaga ggtacaccag caccaaagag 4020

gtgctggacg ccaccctgat ccaccagagc atcaccggcc tgtacgagac acggatcgac 4080

ctgtctcagc tgggaggcga c 4101

<210> 27
<211> 24
<212> DNA
<213> Artificial sequence

<220>
<223> Sequencing primer

<400> 27
cacctcagca gaaacaaagt tatc 24

<210> 28
<211> 20
<212> DNA
<213> Artificial sequence

<220>
<223> Sequencing primer

<400> 28
gggaagctcc aaactctcaa 20

<210> 29
<211> 24
<212> DNA
<213> Artificial sequence

<220>
<223> Primer sequence for amplificationforward primer

<400> 29
cacctcagca gaaacaaagt tatc 24

<210> 30
<211> 20
<212> DNA
<213> Artificial sequence

<220>
<223> Primer sequence for amplificationreverse primer

<400> 30
gggaagctcc aaactctcaa 20